# EUROPEAN PATENT APPLICATION

(11) **EP 3 978 530 A1**
(43) Date of publication of application: **06.04.2022**
(21) Application number: 21184652.2
(22) Date of filing: 25.02.2016
(51) Int. Cl.: C07K 16/28, A61K 39/395, A61K 31/573, A61K 31/58, A61P 1/00, A61P 1/04, A61P 37/00, A61P 37/06

(54) **INTEGRIN BETA7 ANTAGONISTS AND METHODS OF TREATING CROHN'S DISEASE**

(30) Priority: 26.02.2015 US 201562121290 P
(62) Divisional of application: 16708894.7
(71) Applicant: F. Hoffmann-La Roche AG, 4070 Basel (CH)
(72) Inventor: HASSANALI, Azra, South San Francisco, 94080 (US); MACIUCA, Romeo, South San Francisco, 94080 (US); TANG, Meina Tao, South San Francisco, 94080 (US); TOLE, Swati, South San Francisco, 94080 (US); WEI, Xiaohui, Rockville, 20850 (US)
(74) Representative: Bernard, Guillaume

(57) **Abstract**

Methods of treating gastrointestinal inflammatory disorders such as inflammatory bowel diseases including Crohn's disease are provided. Also provided are methods of administering and dosing integrin beta7 antagonists, such as anti-integrin beta7 antibodies. In addition, methods of administrating and dosing such integrin beta7 antagonists to induce remission or to induce and maintain remission of Crohn's disease are provided.

SEQUENCE LISTING

<110> GENENTECH, INC. ET AL.

<120> INTEGRIN BETA7 ANTAGONISTS AND METHODS OF TREATING CROHN'S DISEASE

<130> P32628-WO

<140>
<141>

<150> 62/121,290
<151> 2015-02-26

<160> 32

<170> PatentIn version 3.5

<210> 1
<211> 11
<212> PRT
<213> Artificial Sequence

<220>
<221> source
<223> /note="Description of Artificial Sequence: Synthetic
      peptide"

<400> 1
Arg Ala Ser Glu Ser Val Asp Thr Tyr Leu His
1               5                   10


<210> 2
<211> 8
<212> PRT
<213> Artificial Sequence

<220>
<221> source
<223> /note="Description of Artificial Sequence: Synthetic
      peptide"

<400> 2
Lys Tyr Ala Ser Gln Ser Ile Ser
1               5


<210> 3
<211> 9
<212> PRT
<213> Artificial Sequence

<220>
<221> source
<223> /note="Description of Artificial Sequence: Synthetic
      peptide"

<400> 3
Gln Gln Gly Asn Ser Leu Pro Asn Thr
1               5

<210> 4
<211> 10
<212> PRT
<213> Artificial Sequence

<220>
<221> source
<223> /note="Description of Artificial Sequence: Synthetic
      peptide"

<400> 4
Gly Phe Phe Ile Thr Asn Asn Tyr Trp Gly
1               5                   10


<210> 5
<211> 17
<212> PRT
<213> Artificial Sequence

<220>
<221> source
<223> /note="Description of Artificial Sequence: Synthetic
      peptide"

<400> 5
Gly Tyr Ile Ser Tyr Ser Gly Ser Thr Ser Tyr Asn Pro Ser Leu Lys
1               5                   10                  15


Ser




<210> 6
<211> 10
<212> PRT
<213> Artificial Sequence

<220>
<221> source
<223> /note="Description of Artificial Sequence: Synthetic
      peptide"

<400> 6
Met Thr Gly Ser Ser Gly Tyr Phe Asp Phe
1               5                   10


<210> 7
<211> 11
<212> PRT
<213> Artificial Sequence

<220>
<221> source
<223> /note="Description of Artificial Sequence: Synthetic
      peptide"

<400> 7
Arg Ala Ser Glu Ser Val Asp Ser Leu Leu His
1               5                   10

<210> 8
<211> 11
<212> PRT
<213> Artificial Sequence

<220>
<221> source
<223> /note="Description of Artificial Sequence: Synthetic
      peptide"

<400> 8
Arg Ala Ser Glu Ser Val Asp Thr Leu Leu His
1               5                   10


<210> 9
<211> 11
<212> PRT
<213> Artificial Sequence

<220>
<221> source
<223> /note="Description of Artificial Sequence: Synthetic
      peptide"

<400> 9
Arg Ala Ser Glu Ser Val Asp Asp Leu Leu His
1               5                   10


<210> 10
<211> 108
<212> PRT
<213> Rattus sp.

<400> 10
Asp Val Val Met Thr Gln Ser Pro Ala Thr Leu Ser Val Thr Pro Gly
1               5                   10                  15

Glu Arg Ile Ser Leu Ser Cys Arg Ala Ser Glu Ser Val Asp Thr Tyr
            20                  25                  30

Leu His Trp Tyr Gln Gln Lys Pro Asn Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Thr Asp Phe Thr Leu Ser Ile Asn Gly Val Glu Leu
65                  70                  75                  80

Glu Asp Leu Ser Ile Tyr Tyr Cys Gln Gln Gly Asn Ser Leu Pro Asn
                85                  90                  95

Thr Phe Gly Ala Gly Thr Lys Leu Glu Leu Lys Arg
            100                 105

<210> 11
<211> 117
<212> PRT
<213> Rattus sp.

<400> 11
Glu Val Gln Leu Gln Glu Ser Gly Pro Gly Leu Val Lys Pro Ser Gln
1               5                   10                  15

Ser Leu Ser Leu Thr Cys Ser Val Thr Gly Phe Phe Ile Thr Asn Asn
            20                  25                  30

Tyr Trp Gly Trp Ile Arg Lys Phe Pro Gly Asn Lys Met Glu Trp Met
        35                  40                  45

Gly Tyr Ile Ser Tyr Ser Gly Ser Thr Ser Tyr Asn Pro Ser Leu Lys
    50                  55                  60

Ser Arg Ile Ser Ile Thr Arg Asp Thr Ser Lys Asn Gln Phe Phe Leu
65                  70                  75                  80

Gln Leu Asn Ser Val Thr Thr Glu Asp Thr Ala Thr Tyr Tyr Cys Ala
                85                  90                  95

Met Thr Gly Ser Ser Gly Tyr Phe Asp Phe Trp Gly Pro Gly Thr Met
            100                 105                 110

Val Thr Val Ser Ser
        115


<210> 12
<211> 108
<212> PRT
<213> Homo sapiens

<400> 12
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Thr Tyr Tyr Cys Gln Gln Tyr Asn Ser Leu Pro Trp
                85                  90                  95

Thr Phe Gly Gln Gly Thr Lys Val Glu Ile Lys Arg
            100                 105

<210> 13
<211> 113
<212> PRT
<213> Homo sapiens

<400> 13
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Val Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Asp Tyr Trp Gly Gln Gly Thr Leu Val Thr Val Ser
            100                 105                 110

Ser

<210> 14
<211> 108
<212> PRT
<213> Artificial Sequence

<220>
<221> source
<223> /note="Description of Artificial Sequence: Synthetic
      polypeptide"

<400> 14
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Thr Tyr
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 15
<211> 117
<212> PRT
<213> Artificial Sequence

<220>
<221> source
<223> /note="Description of Artificial Sequence: Synthetic
      polypeptide"

<400> 15
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Phe Ile Thr Asn Asn
            20                  25                  30

Tyr Trp Gly Trp Val Arg Gln Ala Pro Gly Lys Gly Leu Glu Trp Val
        35                  40                  45

Gly Tyr Ile Ser Tyr Ser Gly Ser Thr Ser Tyr Asn Pro Ser Leu Lys
    50                  55                  60

Ser Arg Phe Thr Ile Ser Ala Asp Thr Ser Lys Asn Thr Ala Tyr Leu
65                  70                  75                  80

Gln Met Asn Ser Leu Arg Ala Glu Asp Thr Ala Val Tyr Tyr Cys Ala
                85                  90                  95

Met Thr Gly Ser Ser Gly Tyr Phe Asp Phe Trp Gly Gln Gly Thr Leu
            100                 105                 110

Val Thr Val Ser Ser
        115



<210> 16
<211> 11
<212> PRT
<213> Artificial Sequence

<220>
<221> source
<223> /note="Description of Artificial Sequence: Synthetic
      peptide"

<400> 16
Ala Met Thr Gly Ser Ser Gly Tyr Phe Asp Phe
1               5                   10



<210> 17
<211> 11
<212> PRT
<213> Artificial Sequence

<220>
<221> source
<223> /note="Description of Artificial Sequence: Synthetic
      peptide"

<400> 17
Ala Arg Thr Gly Ser Ser Gly Tyr Phe Asp Phe
1               5                   10



<210> 18
<211> 11
<212> PRT
<213> Artificial Sequence

<220>
<221> source
<223> /note="Description of Artificial Sequence: Synthetic
      peptide"

<400> 18
Ala Gln Thr Gly Ser Ser Gly Tyr Phe Asp Phe
1               5                   10



<210> 19
<211> 10
<212> PRT
<213> Artificial Sequence

<220>
<221> source
<223> /note="Description of Artificial Sequence: Synthetic
      peptide"

<400> 19
Arg Thr Gly Ser Ser Gly Tyr Phe Asp Phe
1               5                   10

<210> 20
<211> 8
<212> PRT
<213> Artificial Sequence

<220>
<221> source
<223> /note="Description of Artificial Sequence: Synthetic
      peptide"

<400> 20
Arg Tyr Ala Ser Gln Ser Ile Ser
1               5


<210> 21
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> Any amino acid

<400> 21
Xaa Tyr Ala Ser Gln Ser Ile Ser
1               5


<210> 22
<211> 108
<212> PRT
<213> Artificial Sequence

<220>
<221> source
<223> /note="Description of Artificial Sequence: Synthetic
      polypeptide"

<400> 22
Asp Ile Gln Met Thr Gln Ser Pro Ser Ser Leu Ser Ala Ser Val Gly
1               5                   10                  15


Asp Arg Val Thr Ile Thr Cys Arg Ala Ser Glu Ser Val Asp Ser Leu
            20                  25                  30


Leu His Trp Tyr Gln Gln Lys Pro Gly Lys Ala Pro Lys Leu Leu Ile
        35                  40                  45


Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 23
<211> 108
<212> PRT
<213> Artificial Sequence

<220>
<221> source
<223> /note="Description of Artificial Sequence: Synthetic
      polypeptide"

<400> 23
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Thr Leu
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 24
<211> 108
<212> PRT
<213> Artificial Sequence

<220>
<221> source
<223> /note="Description of Artificial Sequence: Synthetic
      polypeptide"

<400> 24
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Asp Leu
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105


<210> 25
<211> 117
<212> PRT
<213> Artificial Sequence

<220>
<221> source
<223> /note="Description of Artificial Sequence: Synthetic
      polypeptide"

<400> 25
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Phe Ile Thr Asn Asn
            20                  25                  30

Tyr Trp Gly Trp Val Arg Gln Ala Pro Gly Lys Gly Leu Glu Trp Val
        35                  40                  45

Gly Tyr Ile Ser Tyr Ser Gly Ser Thr Ser Tyr Asn Pro Ser Leu Lys
    50                  55                  60

Ser Arg Phe Thr Ile Ser Arg Asp Thr Ser Lys Asn Thr Phe Tyr Leu
65                  70                  75                  80

Gln Met Asn Ser Leu Arg Ala Glu Asp Thr Ala Val Tyr Tyr Cys Ala
                85                  90                  95

Met Thr Gly Ser Ser Gly Tyr Phe Asp Phe Trp Gly Gln Gly Thr Leu
            100                 105                 110

Val Thr Val Ser Ser
        115

<210> 26
<211> 11
<212> PRT
<213> Artificial Sequence

<220>
<221> source
<223> /note="Description of Artificial Sequence: Synthetic
      peptide"

<220>
<221> VARIANT
<222> (2)..(2)
<223> /replace="Gly" or "Ser" or "Thr" or "Val"

<220>
<221> VARIANT
<222> (3)..(3)
<223> /replace="Gly" or "Ile" or "Lys" or "Asn" or "Pro" or "Gln"
      or "Arg" or "Thr"

<220>
<221> VARIANT
<222> (4)..(4)
<223> /replace="Val" or "Gln" or "Ala" or "Asp" or "Gly" or "His"
      or "Ile" or "Lys" or "Leu" or "Asn" or "Arg"

<220>
<221> VARIANT
<222> (5)..(5)
<223> /replace="Tyr" or "Ala" or "Asp" or "Gly" or "His" or "Ile"
      or "Lys" or "Asn" or "Pro" or "Arg" or "Thr" or "Val"

<220>
<221> VARIANT
<222> (6)..(6)
<223> /replace="Arg" or "Ile" or "Ala" or "Gly" or "Lys" or "Leu"
      or "Met" or "Gln"

<220>
<221> VARIANT
<222> (7)..(7)
<223> /replace="Val" or "Ser" or "Ala" or "Glu" or "Gly" or "His"
      or "Ile" or "Lys" or "Leu" or "Asn" or "Pro" or "Ser" or "Thr"

<220>
<221> VARIANT
<222> (8)..(8)
<223> /replace="Gly" or "Asn" or "Glu" or "Thr" or "Pro" or "Ser"

<220>
<221> VARIANT
<222> (9)..(9)

<223> /replace="Tyr" or "Ile" or "Met"

<220>
<221> VARIANT
<222> (10)..(10)
<223> /replace="Ala" or "Ile" or "Met" or "Val"

<220>
<221> VARIANT
<222> (11)..(11)
<223> /replace="Tyr" or "Phe" or "Ser"

<220>
<221> MISC_FEATURE
<222> (1)..(11)
<223> /note="Variant residues given in the sequence have no
      preference with respect to those in the annotations
      for variant positions"

<400> 26
Arg Ala Ser Glu Ser Val Asp Asp Leu Leu His
1               5                   10


<210> 27
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> Any amino acid

<220>
<221> VARIANT
<222> (4)..(4)
<223> /replace="Asp"

<220>
<221> VARIANT
<222> (5)..(5)
<223> /replace="Ser"

<220>
<221> VARIANT
<222> (6)..(6)
<223> /replace="Asp" or "Leu" or "Arg"

<220>
<221> VARIANT
<222> (7)..(7)
<223> /replace="Val" or "Glu" or "Lys"

<220>
<221> MISC_FEATURE
<222> (1)..(8)

<223> /note="Variant residues given in the sequence have no
      preference with respect to those in the annotations
      for variant positions"

<220>
<223> See specification as filed for detailed description of
      substitutions and certain embodiments

<400> 27
Xaa Tyr Ala Ser Gln Ser Ile Ser
1               5


<210> 28
<211> 9
<212> PRT
<213> Artificial Sequence

<220>
<221> source
<223> /note="Description of Artificial Sequence: Synthetic
      peptide"


<220>
<221> VARIANT
<222> (8)..(8)
<223> /replace="Val" or "Trp" or "Tyr" or "Arg" or "Ser" or "Thr"
      or "Ala" or "Phe" or "His" or "Ile" or "Leu" or "Met"

<220>
<221> MISC_FEATURE
<222> (1)..(9)
<223> /note="Variant residues given in the sequence have no
      preference with respect to those in the annotations
      for variant positions"

<400> 28
Gln Gln Gly Asn Ser Leu Pro Asn Thr
1               5


<210> 29
<211> 17
<212> PRT
<213> Artificial Sequence

<220>
<221> source
<223> /note="Description of Artificial Sequence: Synthetic
      peptide"


<220>
<221> VARIANT
<222> (2)..(2)
<223> /replace="Phe" or "Val" or "Asp"

<220>
<221> VARIANT
<222> (6)..(6)
<223> /replace="Gly"

<220>
<221> VARIANT
<222> (10)..(10)
<223> /replace="Tyr"

<220>
<221> VARIANT
<222> (12)..(12)
<223> /replace="Thr" or "Ala" or "Asp"

<220>
<221> VARIANT
<222> (13)..(13)
<223> /replace="His" or "Asp" or "Ala"

<220>
<221> VARIANT
<222> (15)..(15)
<223> /replace="Val"

<220>
<221> VARIANT
<222> (17)..(17)
<223> /replace="Gly"

<220>
<221> MISC_FEATURE
<222> (1)..(17)
<223> /note="Variant residues given in the sequence have no
      preference with respect to those in the annotations
      for variant positions"

<400> 29
Gly Tyr Ile Ser Tyr Ser Gly Ser Thr Ser Tyr Asn Pro Ser Leu Lys
1               5                   10                  15

Ser

<210> 30
<211> 11
<212> PRT
<213> Artificial Sequence

<220>
<221> source
<223> /note="Description of Artificial Sequence: Synthetic
      peptide"

<220>
<221> VARIANT
<222> (1)..(1)
<223> /replace=" "

<220>
<221> VARIANT
<222> (2)..(2)
<223> /replace="Met" or "Ala" or "Glu" or "Gly" or "Gln" or "Ser"

<220>
<221> VARIANT

<222> (11)..(11)
<223> /replace="Tyr"

<220>
<221> MISC_FEATURE
<222> (1)..(11)
<223> /note="Variant residues given in the sequence have no
      preference with respect to those in the annotations
      for variant positions"

<400> 30
Ala Arg Thr Gly Ser Ser Gly Tyr Phe Asp Phe
1               5                   10


<210> 31
<211> 108
<212> PRT
<213> Artificial Sequence

<220>
<221> source
<223> /note="Description of Artificial Sequence: Synthetic
      polypeptide"

<400> 31
Asp Ile Gln Met Thr Gln Ser Pro Ser Ser Leu Ser Ala Ser Val Gly
1               5                   10                  15


Asp Arg Val Thr Ile Thr Cys Arg Ala Ser Glu Ser Val Asp Asp Leu
            20                  25                  30


Leu His Trp Tyr Gln Gln Lys Pro Gly Lys Ala Pro Lys Leu Leu Ile
        35                  40                  45


Lys Tyr Ala Ser Gln Ser Ile Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Gly Asn Ser Leu Pro Asn
                85                  90                  95


Thr Phe Gly Gln Gly Thr Lys Val Glu Ile Lys Arg
            100                 105


<210> 32
<211> 117
<212> PRT
<213> Artificial Sequence

<220>
<221> source
<223> /note="Description of Artificial Sequence: Synthetic
      polypeptide"

<400> 32

## Description

### CROSS REFERENCE TO RELATED APPLICATIONS

This application claims the benefit of priority of provisional U.S. Application No. 62/121,290 filed February 26, 2015, which is hereby incorporated by reference in its entirety.

### SEQUENCE LISTING

The instant application contains a Sequence Listing which has been submitted via EFS-Web and is hereby incorporated by reference in its entirety. Said ASCII copy, created on February 24, 2016, is named P32628WO_PCTSL.txt and is 22,201 bytes in size.

### FIELD

Methods of treating gastrointestinal inflammatory disorders such as inflammatory bowel diseases including Crohn's disease are provided. Also provided are methods of administering and dosing integrin beta7 antagonists, such as anti-integrin beta7 antibodies. In addition, methods of administrating and dosing such integrin beta7 antagonists to induce remission or to induce and maintain remission of Crohn's disease are provided.

### BACKGROUND

Inflammatory bowel disease (IBD) is a chronic inflammatory autoimmune condition of the gastrointestinal (GI) tract, which presents clinically as either ulcerative colitis (UC) or Crohn's disease (CD). CD is a chronic transmural inflammatory disease with the potential to affect any part of the entire GI tract, and UC is a mucosal inflammation of the colon. Both conditions are characterized clinically by frequent bowel motions, malnutrition, and dehydration, with disruption in the activities of daily living. The etiology of IBD is complex, and many aspects of the pathogenesis remain unclear.

CD is a chronic, relapsing form of IBD that can affect any portion of the gastrointestinal tract, with 40%-50% of cases affecting the small bowel. CD is characterized by patchy, transmural inflammation, ulcers, and granulomatous lesions that are interspersed with healthy sections of bowel (skip lesions). The disease is progressive; uncontrolled inflammation develops into stricturing or penetrating complications such as prestenotic dilatation, obstruction (stricturing), and intra-abdominal or perianal fistulae and abscesses (penetrating). Clinical signs and symptoms include chronic diarrhea, abdominal pain, cachexia, abdominal mass, or tenderness as well as the overt signs of fistulae. The disease course is variable; patients can experience a severe initial flare followed by few symptoms over the next 10 years (43%) or symptoms that are chronic and persistent (19%) or relapsing-remitting (32%) (Baumgart DC, et al., Lancet 380:1590-605, 2012).

The annual incidences of CD reported in Europe, Asia and the Middle East, and North America were 12.7, 5.0, and 20.2 per 100,000 person-years, respectively (Molodecky NA, et al., Gastroenterology 142:46-54, 2012). Current prevalence rates in North America are reported to be 319 per 100,000 persons (*Id*.). Disease-related mortality in CD accounts for approximately 30% of deaths in this population, resulting from clinical and/or surgical complications that occur early in the disease course or intestinal cancer occurring later. The global incidence of CD is expected to continue increasing substantially, affecting individuals in the most formative and productive years of life, with long-term costs to patients, healthcare systems, and society (Duricova D. et al., Inflamm Bowel Dis 16:347-53, 2010).

To date, there is no cure for CD. Accordingly, the current treatment goals for CD are to induce and maintain symptom improvement, induce mucosal healing, avoid surgery, and improve quality of life (Lichtenstein GR, et al., Am J Gastroenterol 104:465-83, 2009; Van Assche G, et al., J Crohns Colitis. 4:63-101, 2010).

Systemic corticosteroids (CSs) have been the mainstay treatment for inducing remission and are effective in approximately 80% of patients (Summers RW, et al., Gastroenterology 77:847-69, 1979; Malchow H, et al., Gastroenterology 86:249-66, 1984). However, they are less effective as a maintenance therapy, with only 28% of patients achieving a prolonged response after 1 year of treatment and 32% of patients becoming steroid dependent (Faubion WA, et al., Gastroenterology 121:255-60, 2001; Peyrin-Biroulet L, et al., Am J Gastroenterol 105:289-97, 2010). Even if patients' symptoms improve, fewer than 30% are expected to achieve endoscopic improvement with steroid treatment (Modigliani R, et al., Gastroenterology 98:811-8, 1990). The adverse effects of steroids are well documented and 50% of patients will stop their treatment because of this; long-term safety outcomes include osteoporosis, cataracts, and diabetes.

Immunosuppressants (ISs) (e.g., azathioprine [AZA], 6-mercaptopurine [6-MP], or methotrexate [MTX]), are typically administered to induce remission in patients who are intolerant of or refractory to steroids and to maintain remission in patients who achieve quiescent CD. Immunosuppressants are given with or without a steroid bridge, depending on a patient's symptoms during the 2-4-month onset of IS efficacy. In patients with ileal or ascending colonic disease, budesonide presents a less toxic, more tolerable bridge because of its low systemic bioavailability resulting from a rapid first-pass metabolism. An earlier initiation of IS to alter the inflammatory disease course has been advocated over the past 20 years. However, a decrease in the rate of intestinal resections and complications has not been observed during this time (Cosnes J, et al., Gut 54:237-41, 2005). This may reflect poor adoption of this top-down treatment over concern for systemic toxicities including leukopenia, thromobocytopenia, and increased risk for lymphoma with AZA and 6-MP (Prefontaine E, et al., Cochrane Database Syst Rev (4):CD000545, 2009) and hepatotoxicity and hair loss with MTX (Hausmann J, et al., Inflamm Bowel Dis 16:1195-202, 2010).

The development of monoclonal antibodies (mAbs) against tumor necrosis factor (TNF)-α has provided an additional treatment option. Although anti-TNFs are effective in a significant proportion of patients, efficacy is suboptimal; remission rates after 4 weeks of induction therapy are fewer than 35% and among patients who respond to induction therapy fewer than 50% achieve remission when assessed in maintenance at 20-30 weeks (Peyrin-Biroulet L, et al., Aliment Pharmacol Ther 33:870-9, 2011). Furthermore, 30% of patients are reported to be primary non-responders to anti-TNF therapy when assessed after 4 weeks of induction therapy (Targan SR, et al., N Engl J Med 337:1029-35, 1997; Sandborn WJ, et al., Ann Intern Med 2007:19;146:829-38. Epub 2007 Apr 30), possibly because of an underlying pathobiology that is not TNF-α driven and as such may benefit from a different mechanistic class of drug. It is estimated that 30%-40% of patients will be secondary non-responders (i.e., initially responsive) but lose response or become intolerant in their first year of treatment (Colombel JF, et al., Gastroenterology 132:52-65, 2007). Secondary non-response has been attributed to the development of neutralizing antibodies, resulting in low drug serum levels, to accelerated drug clearance, or a biological escape mechanism that may benefit from a therapy with a different pharmacological target. Anti-TNFs are also associated with significant side effects, including serious infection, opportunistic infection, lupus-like reactions, and an increased risk of lymphoma (Siegal CA, et al., Therap Adv Gastroenterol 2:245-51, 2009). Tolerability concerns include infusion reactions (occurring in 9%-17% of patients treated with infliximab, *see* de Vries HS, et al., Br J Clin Pharmacol 71:7-19, 2011) and injection site reactions (occurring in 10% of patients receiving adalimumab, *see* van der Heijde D, et al., Arthritis Rheum. 54:2136-46, 2006). Overall, the benefits versus risks are considered acceptable for this drug class, but there continues to be a need for treatments with better benefit-risk profiles that attenuate inflammation and the clinical sequelae and improve the long-term prognosis of patients with CD.

The integrins are alpha/beta heterodimeric cell surface glycoprotein receptors that play a role in numerous cellular processes including leukocyte adhesion, signaling, proliferation, and migration, as well as in gene regulation (Hynes, R. O., Cell, 1992, 69:11-25; and Hemler, M. E., Annu. Rev. Immunol., 1990, 8:365-368). They are composed of two heterodimeric, non-covalently interacting α and β transmembrane subunits that bind specifically to distinct cell adhesion molecules (CAMs) on endothelia, epithelia, and extracellular matrix proteins. In this manner, integrins can function as tissue-specific cell adhesion receptors aiding in the recruitment of leukocytes from blood into nearly all tissue sites in a highly regulated manner, playing a role in the homing of leukocytes to normal tissue and to sites of inflammation (von Andrian et al., N Engl J Med 343:1020-34 (2000)). In the immune system, integrins are involved in leukocyte trafficking, adhesion and infiltration during inflammatory processes (Nakajima, H. et al., J. Exp. Med., 1994, 179:1145-1154). Differential expression of integrins regulates the adhesive properties of cells and different integrins are involved in different inflammatory responses. (Butcher, E. C. et a/., Science, 1996, 272:60-66). The beta7 containing integrins (*i.e.,* alpha4beta7 and alphaEbeta7) are expressed primarily on monocytes, lymphocytes, eosinophils, basophils, and macrophages but not on neutrophils (Elices, M. J. et al., Cell, 1990, 60:577-584)

The anti-integrins are another class of biologics approved for the treatment of CD. Natalizumab is an anti-integrin approved in the U.S. only for the treatment of moderate to severely active CD. The use of natalizumab, which blocks both α4β1 and α4β7 has been limited due to concerns that inhibition of α4β1/VCAM-1 binding increases the risk of progressive multifocal leukoencephalopathy (PML) a rare but serious infection of the CNS. Vedolizumab is the most recently approved gut-selective anti-integrin for CD, but this targets only the α4β7 integrin receptor, inhibiting T-lymphocyte binding to the adhesion molecule MAdCAM-1, and is administered as an intravenous (IV) infusion. In the pivotal trials for vedolizumab 31% of patients had a clinical response with 6 weeks of induction treatment, defined as ≥ 100-point decrease in the Crohn's Disease Activity Index [CDAI] score from baseline; up to 39% of the vedolizumab responders achieved remission with 46 weeks of maintenance treatment (defined as CDAI score of ≤ 150), compared with 22% of patients given placebo (Sandborn WJ, et al., N Engl J Med 369(8):711-721, 2013; Sandborn WJ, et al., Aliment Pharmacol Ther 37:204-13, 2013). While vedolizumab shows promise as a new treatment for CD, there remains a need for a more convenient therapy which is gut selective and achieves better response and remission rates.

The α4β7 integrin (the target of vedolizumab) is a leukocyte-homing receptor that is important in the migration of cells to the intestinal mucosa and associated lymphoid tissues, such as Peyer's patches in the small intestine, lymphoid follicles in the large intestine, and mesenteric lymph nodes. In the gut, leukocyte rolling and firm adhesion to the mucosal endothelium is initiated by signals from chemokines and is mediated via mucosal addressin cell adhesion molecule (MAdCAM)-1-associated sialyl Lewis X. Chemokine signaling induces the α4β7 integrin to undergo a change from low to high MAdCAM-1 binding affinity. The leukocyte then arrests and begins the process of extravasation through the vascular endothelium to underlying tissue. This extravasation process is believed to occur in both the normal immune cell recirculation state and in inflammatory conditions (von Andrian et al., *supra*). The numbers of α4β7⁺ cells in infiltrates and the expression of the ligand MAdCAM-1 are higher at sites of chronic inflammation such as in the intestinal tract of patients with UC or CD (Briskin et al., Am J Pathol 151:97-110 (1997); Souza et al., Gut 45:856-63 (1999)). α4β7 binds preferentially to high endothelial venules expressing MAdCAM-1 and vascular cell adhesion molecule (VCAM)-1, as well as to the extracellular matrix molecule fibronectin fragment CS-1 (Chan et al., J Biol Chem 267:8366-70 (1992); Ruegg et al., J Cell Biol 17:179-89 (1992); Berlin et al., Cell 74:185-95 (1993)). Together with constitutively expressed MAdCAM-1 in gut mucosal vessels, the α4β7 integrin plays a selective role in leukocyte gut tropism but does not seem to contribute to homing of leukocytes to the peripheral tissue or the CNS. Instead, peripheral lymphoid trafficking has been associated with α4β1 interaction with VCAM-1 (Yednock et al., Nature 356:63-6 (1992); Rice et al., Neurology 64:1336-42 (2005)).

Another member of the β7 integrin family, expressed exclusively on T lymphocytes and associated with mucosal tissues, is the αEβ7 integrin, otherwise known as CD103. The αEβ7 integrin binds selectively to E-cadherin on epithelial cells and has been proposed to play a role in the retention of T cells in the mucosal tissue in the intraepithelial lymphocyte compartment (Cepek et al., J Immunol 150:3459-70 (1993); Karecla et al. Eur J Immunol 25:852-6 (1995)). The αEβ7^{÷} cells in the lamina propria have been reported to exhibit cytotoxicity against stressed or infected epithelial cells (Hadley et al., J Immunol 159:3748-56 (1997); Buri et al., J Pathol 206:178-85 (2005)). The expression of αEβ7 is increased in CD (Elewaut et al., Acta Gastroenterol Belg 61:288-94 (1998); Oshitani et al., Int J Mol Med 12:715-9 (2003)), and anti-αEβ7 antibody treatment has been reported to attenuate experimental colitis in mice, implicating a role for αEβ7⁺ lymphocytes in experimental models of IBD (Ludviksson et al., J Immunol 162:4975-82 (1999)).

Humanized monoclonal antibodies targeted against the β7 integrin subunit have been described previously. See, e.g., Intn'1 Patent Pub. No. WO2006/026759. One such antibody, etrolizumab (rhuMAb Beta7) is derived from the rat anti-mouse/human monoclonal antibody FIB504 (Andrew DP, et al., J Immunol 153:3847-61, 1994). It was engineered to include human IgG1-heavy chain and κ1-light chain frameworks. Intn'l Patent Pub. No. WO2006/026759.

Etrolizumab, a subcutaneously administered mAb, is a novel anti-integrin which unlike vedolizumab, targets both the α4β7 and αEβ7 receptors that regulate trafficking, and retention of T-cell subsets in the intestinal mucosa, respectively. Thus, etrolizumab offers the potential of an additive therapeutic effect in CD via a dual mechanism of action (MOA), without generalized immunosuppression. Etrolizumab binds with high affinity to α4β7 (Holzmann B, et al., Cell 56:37-46, 1989; Hu M, et al., Proc Natl Acad Sci USA 89:8254-8, 1992) and αEβ7 (Cepek KL, et al., J Immunol 150:3459-70, 1993). By this mechanism, it blocks the homing and retention of leukocyte subpopulations in the intestinal mucosa, which occur via binding with the cell adhesion molecules (MAdCAM-1) and E-cadherin, respectively. As such, it represents a novel gut mucosal-selective anti-trafficking agent whose selectivity may eliminate generalized immunosuppression by preferentially targeting trafficking to the gut rather than to other organs and tissues. Data from multiple, non-clinical, general toxicity studies of up to 6 months duration demonstrated that etrolizumab had no adverse effects in any organ system. See, e.g., Stefanich et al., British Journal of Pharmacology 162:1855-1870, 2011; Intn'1 Patent Pub. No. WO 2009/140684.

It is important to note that unlike natalizumab, etrolizumab does not bind to α4β1 or inhibit the interaction of α4β1 and VCAM-1 and the distribution and homing of lymphocytes to the CNS and peripheral lymphoid tissue. As such, etrolizumab is not expected to increase the risk of progressive multifocal leukoencephalopathy (PML). Safety assessments for etrolizumab have been completed in adult Phase 1 and Phase 2 studies, in which patients with moderate to severely active UC received either single or multiple doses of IV or subcutaneous (SC) etrolizumab. A total of 158 patients have been exposed to etrolizumab with no significant adverse safety signals, including any evidence of increased rates of serious or opportunistic infections, being associated with etrolizumab treatment. Acknowledging that the clinical experience with etrolizumab is limited, no events of PML have been reported in patients treated with etrolizumab.

To date, the primary outcome measure in Crohn's Disease clinical trials is the Crohn's Disease Activity Index (CDAI), which has served as the basis for approval of multiple drug treatments, including for example, vedolizumab and natalizumab. The CDAI was developed by regressing clinician global assessment of disease activity on eighteen potential items representing patient reported outcomes (PROs) (i.e. abdominal pain, pain awakening patient from sleep, appetite), physical signs (i.e. average daily temperature, abdominal mass), medication use (i.e. loperamide or opiate use for diarrhea) and a laboratory test (i.e. hematocrit). Backward stepwise regression analysis identified eight independent predictors which are the number of liquid or soft stools, severity of abdominal pain, general well-being, occurrence of extra-intestinal symptoms, need for antidiarrheal drugs, presence of an abdominal mass, hematocrit, and body weight. The final score is a composite of these eight items, adjusted using regression coefficients and standardization to construct an overall CDAI score, ranging from 0 to 600 with higher score indicating greater disease activity. Widely used benchmarks are: CDAI <150 is defined as clinical remission, 150 to 219 is defined as mildly active disease, 220 to 450 is defined as moderately active disease, and above 450 is defined as very severe disease (Best WR, et al., Gastroenterology 77:843-6, 1979). Vedolizumab and natalizumab have been approved on the basis of demonstrated clinical remission, i.e. CDAI ≤ 150.

Although the CDAI has been in use for over 40 years, and has served as the basis for drug approval, it has several limitations as an outcome measure for clinical trials. For example, most of the overall score comes from the patient diary card items (pain, number of liquid bowel movements, and general well-being), which are vaguely defined and not standardized terms (Sandler et al., J. Clin. Epidemiol 41:451-8, 1988; Thia et al., Inflamm Bowel Dis 17:105-11, 2011). In addition, measurement of pain is based on a four-point scale rather than an updated seven-point scale. ENREF 4The remaining 5 index items contribute very little to identifying an efficacy signal and may be a source of measurement noise. Furthermore, concerns have been raised about poor criterion validity for the CDAI, a reported lack of correlation between the CDAI and endoscopic measures of inflammation (which may render the CDAI as a poor discriminator of active CD and irritable bowel syndrome) and high reported placebo rates (Korzenik et al., N Engl J Med. 352:2193-201, 2005; Sandborn WJ, et al., N Engl J Med 353:1912-25, 2005; Sandborn WJ, et al., Ann Intern 19;146:829-38, 2007, Epub 2007 Apr 30; Kim et al., Gastroenterology 146: (5 supplement 1) S-368, 2014).

It is, thus, generally recognized that additional or alternative measures of CD symptoms are needed, such as new PRO tools or adaptations of the CDAI to derive a new PRO. The PRO2 and PRO3 tools are such adaptations of the CDAI and have been recently described in Khanna et al., Aliment Pharmacol. Ther. 41:77-86, 2015. The PRO2 evaluates the frequency of loose/liquid stools and abdominal pain (*Id*.). These items are derived and weighted accordingly from the CDAI and are the CDAI diary card items, along with general well-being, that contribute most to the observed clinical benefit measured by CDAI (Sandler et al., J. Clin. Epidemiol 41:451-8, 1988; Thia et al., Inflamm Bowel Dis 17:105-11, 2011; Kim et al., Gastroenterology 146: (5 supplement 1) S-368, 2014). The remission score of ≤ 11 is the CDAI-weighted sum of the average stool frequency and pain scores in a 7-day period, which yielded optimum sensitivity and specificity for identification of CDAI remission (score of < 150) in a retrospective data analysis of ustekinumab induction treatment for moderate to severe CD in a Phase II clinical study (Gasink C, et al., [abstract] ACG Annual Meeting 2014). The PRO2 was shown to be sensitive and responsive when used as a continuous outcome measure in a retrospective data analysis of MTX treatment in active CD (Khanna R, et al., Inflamm Bowel Dis 20:1850-61, 2014) measured by CDAI.

An additional means of assessing the extent and severity of Crohn's Disease is endoscopy. Endoscopic lesions typical of Crohn's disease have been described in numerous studies and include, e.g., aphthoid ulcerations, "punched-out ulcers," cobblestoning and stenosis. Endoscopic evaluation of such lesions was used to develop the first validated endoscopic score, the Crohn's Disease Endoscopic Index of Severity (CDEIS) (Mary et al., Gut 39:983-9, 1989). More recently, because the CDEIS is time-consuming, complicated and impractical for routine use, a Simplied Endoscopic Activity Score for Crohn's Disease (SES-CD) was developed and validated (Daperno et al., Gastrointest. Endosc. 60(4):505-12, 2004). The SES-CD consists of four endoscopic variables (size of ulcers, proportion of surface covered by ulcers, proportion of surface with any other lesions (e.g., inflammation), and presence of narrowings [stenosis]) that are scored in five ileocolonic segments, with each variable, or assessment, rated from 0 to 3.

Notwithstanding these recent advances in the development and validation of new PRO measures and new endoscopy measures of disease extent and severity in CD, no published prospective clinical studies to date are available to determine how to employ such new measures in the design and conduct of a Phase III clinical study prospectively, i.e. a pivotal or registrational study, for the purpose of evaluating the efficacy of a particular dosing regimen of a therapeutic candidate. There is, thus, a need for improved uses of these measures to define optimal efficacy endpoints for various classes of therapeutic candidates, including anti-integrins. Such improvements are also needed with respect to defining particular dosing regimens, e.g., of anti-integrins, including anti-integrin β7 antibodies, that will meet optimized efficacy endpoints.

The invention described herein meets certain of the above-described needs and provides other benefits.

All references cited herein, including patent applications and publications, are incorporated by reference in their entirety for any purpose.

### SUMMARY

The methods of the invention are based, at least in part, on the discovery that therapeutically effective amounts of integrin beta7 antagonists, such as anti-beta7 antibodies, also referred to herein as anti-integrin beta7 antibodies, including etrolizumab (rhuMAb Beta7), can be administered subcutaneously according to certain dosing regimens that demonstrate efficacy as determined by measuring certain efficacy outcome measures, as described herein.

Accordingly, in one aspect, methods of treating Crohn's Disease (CD) in a mammalian subject comprising administering to the subject a therapeutically effective amount of an integrin beta7 antagonist are provided. In certain embodiments, the mammalian subject is a patient. In certain embodiments, the patient is human. In certain embodiments, the integrin beta7 antagonist is administered subcutaneously. In certain embodiments, the integrin beta7 antagonist is a monoclonal anti-integrin beta7 subunit antibody, also referred to as an anti-beta7 antibody. In certain such embodiments, the anti-beta7 antibody is selected from a chimeric antibody, a human antibody, and a humanized antibody. In certain embodiments, the anti-beta7 antibody is an antibody fragment. In certain embodiments, the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs: 1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29) wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

In certain embodiments, the anti-beta7 antibody comprises three heavy chain hypervariable region (HVR-H1-H3) sequences and three light chain hypervariable region (HVR-L1-L3) sequences, wherein:
(i) HVR-L1 comprises SEQ ID NO:7, SEQ ID NO:8 or SEQ ID NO:9;
(ii) HVR-L2 comprises SEQ ID NO:2;
(iii) HVR-L3 comprises SEQ ID NO:3;
(iv) HVR-H1 comprises SEQ ID NO:4;
(v) HVR-H2 comprises SEQ ID NO:5; and
(vi) HVR-H3 comprises SEQ ID NO:6 or SEQ ID NO:16 or SEQ ID NO:17 or SEQ ID NO:19. In certain embodiments, the anti-beta7 antibody comprises a variable light chain comprising the amino acid sequence of SEQ ID NO:31 and a variable heavy chain comprising the amino acid sequence of SEQ ID NO:32.

In certain embodiments the anti-beta7 antibody is etrolizumab, also referred to as rhuMAb Beta7.

In another aspect, methods of inducing remission in a patient with Crohn's disease are provided. In certain embodiments, the method comprises administering subcutaneously to the patient a therapeutically effective amount of an integrin beta7 antagonist, wherein the therapeutically effective amount induces remission 14 weeks after administration of a first dose. In some embodiments, the integrin beta7 antagonist is a monoclonal anti-integrin beta7 antibody. In some embodiments, the anti-integrin beta7 antibody is selected from a chimeric antibody, a human antibody, and a humanized antibody. In some embodiments, the anti-integrin beta7 antibody is an antibody fragment. In certain embodiments, the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

In certain embodiments, the anti-integrin beta7 antibody comprises three heavy chain hypervariable region (HVR-H1-H3) sequences and three light chain hypervariable region (HVR-L1-L3) sequences, wherein:
(i) HVR-L1 comprises SEQ ID NO:7, SEQ ID NO:8 or SEQ ID NO:9;
(ii) HVR-L2 comprises SEQ ID NO:2;
(iii) HVR-L3 comprises SEQ ID NO:3;
(iv) HVR-H1 comprises SEQ ID NO:4;
(v) HVR-H2 comprises SEQ ID NO:5; and
(vi) HVR-H3 comprises SEQ ID NO:6 or SEQ ID NO:16 or SEQ ID NO:17 or SEQ ID NO:19.

In certain embodiments, the anti-integrin beta7 antibody comprises a variable light chain comprising the amino acid sequence of SEQ ID NO:31 and a variable heavy chain comprising the amino acid sequence of SEQ ID NO:32. In certain embodiments, the anti-integrin beta7 antibody is etrolizumab.

In yet another aspect, the patient has moderately to severely active Crohn's disease prior to administration of the first dose of the integrin beta7 antagonist. In some embodiments, the patient is determined to have a Crohn's Disease Activity Index (CDAI) score of greater than or equal to 220 and less than or equal to 480 at any time in the seven days prior to administration of the first dose. In some embodiments, the patient is determined to have a Patient Reported Outcomes 2 (PRO2) score of greater than or equal to 14 at any time in the seven days prior to administration of the first dose. In some embodiments, the patient is determined to have active inflammation, wherein the active inflammation is determined as a Simplified Endoscopic Index for Crohn's Disease (SES-CD) score of greater than or equal to 7 as determined by ileocolonoscopy. In some embodiments, the patient has isolated ileitis or post-ileocecal resection and the patient is determined to have active inflammation, wherein the active inflammation is determined as a SES-CD score of greater than or equal to 4 as determined by ileocolonoscopy. In certain embodiments, the patient has CDAI score and PRO2 score according to the above. In certain embodiments, the patient has CDAI score, PRO2 score, and SES-CD score according to the above.

In yet still another aspect, the patient had an inadequate response, a loss of response, or intolerance to conventional therapy. In some embodiments, the conventional therapy is selected from one or more of immunosuppressant therapy, corticosteroid therapy, and anti-TNF therapy. In some embodiments, the immunosuppressant therapy is selected from 6-mercaptopurine, azathioprine, and methotrexate. In some embodiments, the corticosteroid therapy is selected from prednisone (or prednisone equivalent) and budesonide. In some embodiments, the anti-TNF therapy is selected from infliximab, adalimumab, and certolizumab pegol.

In yet still another further aspect, the anti-integrin beta7 antibody is administered at a flat dose of 105 mg every 4 weeks. In certain embodiments, the anti-integrin beta7 antibody is administered at a flat dose of 210 mg every 4 weeks. In some embodiments, the anti-integtrin beta7 antibody is administered as a flat dose of 210 mg at the first dose, 210 mg two weeks after the first dose, 210 mg four weeks after the first dose, 210 mg eight weeks after the first dose, and 210 mg 12 weeks after the first dose. In some embodiments, the anti-integtrin beta7 antibody is administered as a flat dose of 210 mg at the first dose, 210 mg two weeks after the first dose, 210 mg four weeks after the first dose, 210 mg eight weeks after the first dose, and 210 mg 12 weeks after the first dose. In some embodiments, remission is determined by Crohn's Disease Activity Index (CDAI) score, wherein the CDAI score is less than 150. In some embodiments, the therapeutically effective amount induces remission 10 weeks after administration of the first dose. In some embodiments, remission is determined by Patient Reported Outcomes 2 (PRO2) score, wherein the PRO2 score is less than or equal to 11. In some embodiments, the therapeutically effective amount induces endoscopic improvement as determined by Simplified Endoscopic Index for Crohn's Disease (SES-CD) score. In some embodiments, the SES-CD score determined 14 weeks after administration of the first dose is reduced by 50% compared to the SES-CD score determined at baseline. In some embodiments, the therapeutically effective amount induces a response, wherein the response is determined as a decrease of CDAI score of at least 70 points compared to CDAI score determined at baseline. In some embodiments, the response is determined as a decrease of CDAI score of at least 100 points compared to CDAI score determined at baseline.

In one aspect, methods of maintaining remission in a patient with Crohn's disease are provided. In certain embodiments, the method comprises administering subcutaneously to the patient a therapeutically effective amount of an integrin beta7 antagonist, wherein the therapeutically effective amount maintains remission for at least 52 weeks, or for at least 66 weeks, or for at least 70 weeks, or for at least 74 weeks, after administration of a first dose. In some embodiments, the therapeutically effective amount maintains remission for at least 74 weeks after administration of the first dose, the patient receives corticosteroid therapy for 14 weeks after administration of the first dose and the corticosteroid therapy is reduced over time beginning at 14 weeks after administration of the first dose until discontinuation. In some embodiments, the corticosteroid therapy is less than or equal to 20 mg of prednisone or prednisone equivalent per day and the corticosteroid therapy is reduced by 2.5 mg prednisone or prednisone equivalent per week until discontinuation. In some embodiments, the corticosteroid therapy is less than or equal to 6 mg oral budesonide per day and wherein the he corticosteroid therapy is reduced by 3 mg oral budesonide every 2 weeks until discontinuation. In certain embodiments, the therapeutically effective amount maintains durable remission, wherein durable remission is determined by CDAI score less than 150 at each of six or more timepoints selected from, 24 weeks after administration of the first dose, 28 weeks after administration of the first dose, 32 weeks after administration of the first dose, 44 weeks after administration of the first dose, 56 weeks after administration of the first dose, 66 weeks after administration of the first dose, 70 weeks after administration of the first dose, and 74 weeks after administration of the first dose. In certain embodiments, the integrin beta7 antagonist is a monoclonal anti-integrin beta7 antibody. In some embodiments, the anti-integrin beta7 antibody is selected from a chimeric antibody, a human antibody, and a humanized antibody. In some embodiments, the anti-integrin beta7 antibody is an antibody fragment. In some embodiments, the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y. In some embodiments, the anti-integrin beta7 antibody comprises three heavy chain hypervariable region (HVR-H1-H3) sequences and three light chain hypervariable region (HVR-L1-L3) sequences, wherein:
   (i) HVR-L1 comprises SEQ ID NO:7, SEQ ID NO:8 or SEQ ID NO:9;
   (ii) HVR-L2 comprises SEQ ID NO:2;
   (iii) HVR-L3 comprises SEQ ID NO:3;
   (iv) HVR-H1 comprises SEQ ID NO:4;
   (v) HVR-H2 comprises SEQ ID NO:5; and
   (vi) HVR-H3 comprises SEQ ID NO:6 or SEQ ID NO:16 or SEQ ID NO:17 or SEQ ID NO:19. In some embodiments, the anti-integrin beta7 antibody comprises a variable light chain comprising the amino acid sequence of SEQ ID NO:31 and a variable heavy chain comprising the amino acid sequence of SEQ ID NO:32. In some embodiments, the anti-integrin beta7 antibody is etrolizumab.

In another aspect of the methods of the preceding paragraph, the patient has moderately to severely active Crohn's disease prior to administration of the first dose of the integrin beta7 antagonist. In some embodiments, the patient is determined to have a CDAI score of greater than or equal to 220 and less than or equal to 480 at any time in the seven days prior to administration of the first dose. In some embodiments, the patient is determined to have a PRO2 score of greater than or equal to 14 at any time in the seven days prior to administration of the first dose. In some embodiments, the patient is determined to have active inflammation, wherein the active inflammation is determined as a SES-CD score of greater than or equal to 7 as determined by ileocolonoscopy. In some embodiments, the patient has isolated ileitis or post-ileocecal resection and the patient is determined to have active inflammation, wherein the active inflammation is determined as a SES-CD score of greater than or equal to 4 as determined by ileocolonoscopy. In certain embodiments, the patient has CDAI score and PRO2 score according to the above. In certain embodiments, the patient has CDAI score, PRO2 score, and SES-CD score according to the above.

In yet another aspect of the methods of the preceding paragraph, the patient had an inadequate response, a loss of response, or intolerance to conventional therapy. In some embodiments, the conventional therapy is selected from one or more of immunosuppressant therapy, corticosteroid therapy, and anti-TNF therapy. In some embodiments, the immunosuppressant therapy is selected from 6-mercaptopurine, azathioprine, and methotrexate. In some embodiments, the corticosteroid therapy is selected from prednisone, prednisone equivalent, and budesonide. In some embodiments, the anti-TNF therapy is selected from infliximab, adalimumab, and certolizumab pegol.

In yet still a further aspect of maintaining remission, the anti-integrin beta7 antibody is administered at a flat dose of 105 mg every 4 weeks. In some embodimetns, the anti-integrin beta7 antibody is administered at a flat dose of 210 mg every 4 weeks. In some embodiments, the anti-integtrin beta7 antibody is administered as a flat dose of 210 mg at the first dose, 210 mg two weeks after the first dose, 210 mg four weeks after the first dose, 210 mg eight weeks after the first dose, 210 mg 12 weeks after the first dose and 105 mg every 4 weeks thereafter. In some embodiments, remission is determined by Crohn's Disease Activity Index (CDAI) score, wherein the CDAI score is less than 150. In some embodiments, the patient is corticosteroid-free for at least 52 weeks. In some embodiments, remission is determined by Patient Reported Outcomes 2 (PRO2) score, wherein the PRO2 score is less than or equal to 11. In some embodiments, the therapeutically effective amount induces endoscopic improvement as determined by Simplified Endoscopic Index for Crohn's Disease (SES-CD) score. In some embodiments, the SES-CD score determined 66 weeks after administration of the first dose is reduced by 50% compared to the SES-CD score determined at baseline. In some embodiments, the endoscopic improvement 66 weeks after administration of the first dose is resolution of mucosal inflammation, wherein resolution of mucosal inflammation is SES-CD score determined as zero. In some embodiments, the therapeutically effective amount induces a response 14 weeks after administration of the first dose, wherein the response is determined as a decrease of CDAI score of at least 70 points compared to CDAI score determined at baseline. In some embodiments, the therapeutically effective amount induces a response 66 weeks after administration of the first dose, wherein the response is determined as a decrease of CDAI score of at least 100 points compared to CDAI score determined at baseline.

In a still further aspect of any of the above embodiments, the integrin beta7 antagonist is administered using a prefilled syringe or a prefilled syringe and autoinjector combination.

### BRIEF DESCRIPTION OF THE DRAWINGS

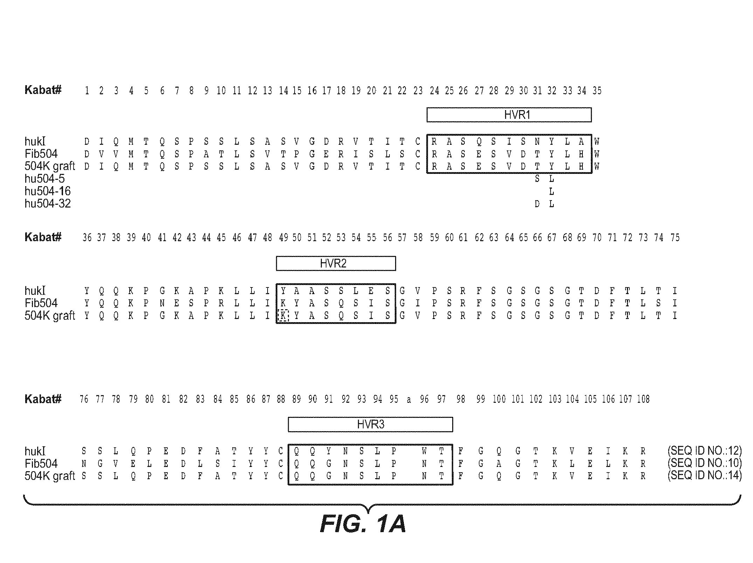

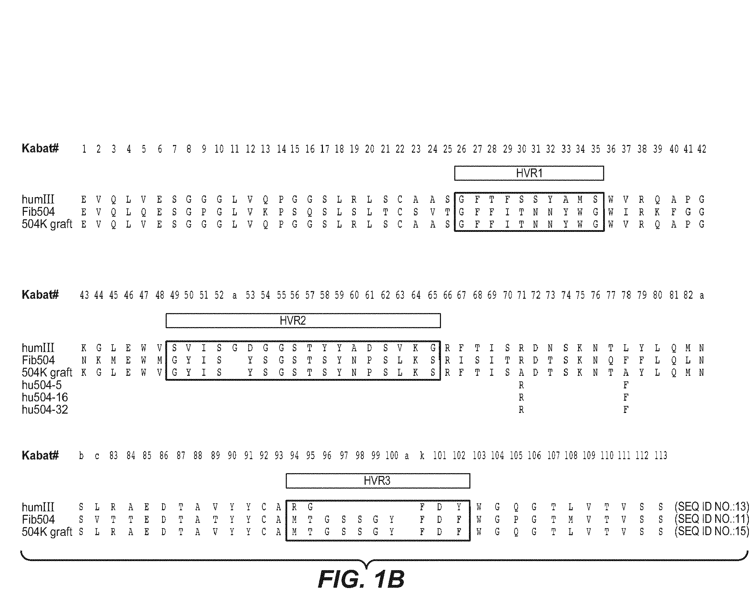

**Figures 1A** **and** **1B** show alignments of sequences of the variable light and heavy chains for the following consensus sequences and anti-beta7 subunit antibody sequences: light chain human subgroup kappa I consensus sequence (FIG. 1A, SEQ ID NO:12), heavy chain human subgroup III consensus sequence (FIG. 1B, SEQ ID NO:13), rat anti-mouse beta7 antibody (Fib504) variable light chain (FIG. 1A, SEQ ID NO:10), rat anti-mouse beta7 antibody (Fib504) variable heavy chain (FIG. 1B, SEQ ID NO:11), and humanized antibody variants: Humanized hu504Kgraft variable light chain (FIG. 1A, SEQ ID NO:14), humanized hu504K graft variable heavy chain (FIG. 1B, SEQ ID NO:15), variants hu504-5, hu504-16, and hu504-32 (amino acid variations from humanized hu504K graft are indicated in FIG. 1A) (light chain) (SEQ ID NOS:22-24, respectively, in order of appearance) and FIG. 1B (heavy chain) for variants hu504-5, hu504-16, and hu504-32 (SEQ ID NO:25).

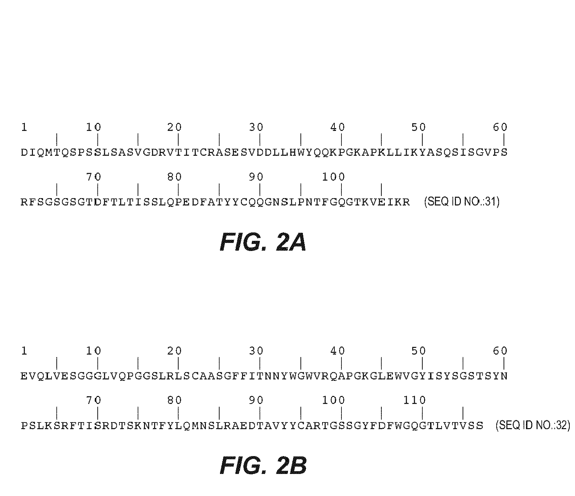

**Figure 2A** shows the variable light chain region (SEQ ID NO:31) and **Figure 2B** shows the variable heavy chain region (SEQ ID NO:32) of etrolizumab.
**Figure 3** shows the study schema for the Induction Phase of the Phase III clinical study as described in Example 1. Anti-TNF = anti-tumor necrosis factor; CD = Crohn's Disease; ETRO = etrolizumab; IS = immunosuppressants; SC = subcutaneous; Wk = week.
**Figure 4** shows the study schema for the Maintenance Phase of the Phase III clinical study as described in Example 1. CS = corticosteroids; ETRO = etrolizumab; OLE = open label extension phase; PBO = placebo; q4w = every 4 weeks; Re-Rx = re-randomized.
**Figure 5** shows the medical aid known as the Bristol Stool Scale as described in Example 1.

### DETAILED DESCRIPTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### CERTAIN DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

As used in this specification and the appended claims, the singular forms "a," "an" and "the" include plural referents unless the context clearly dictates otherwise. Thus, for example, reference to "a protein" includes a plurality of proteins; reference to "a cell" includes mixtures of cells, and the like.

Ranges provided in the specification and appended claims include both end points and all points between the end points. Thus, for example, a range of 2.0 to 3.0 includes 2.0, 3.0, and all points between 2.0 and 3.0.

"Treatment," "treating," and grammatical variations thereof refer to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis.

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Modifying a treatment" refers to changing the treatment regimen including, changing dosage, frequency of administration, or duration of treatment, and/or addition of a second medication.

"Patient response" or "patient responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (*i.e.,* reduction, slowing down or complete stopping) of disease cell infiltration into adjacent peripheral organs and/or tissues; (5) inhibition (*i.e.,* reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment. The term "responsiveness" refers to a measurable response, including complete response (CR) and partial response (PR).

As used herein, "complete response" or "CR" means the disappearance of all signs of inflammation or remission in response to treatment. This does not necessarily mean the disease has been cured.

"Partial response" or "PR" refers to a decrease of at least 50% in the severity of inflammation, in response to treatment.

A "beneficial response" of a patient to treatment with an integrin beta7 antagonist and similar wording refers to the clinical or therapeutic benefit imparted to a patient at risk for or suffering from a gastrointestinal inflammatory disorder from or as a result of the treatment with the antagonist, such as an anti-beta7 integrin antibody. Such benefit includes cellular or biological responses, a complete response, a partial response, a stable disease (without progression or relapse), or a response with a later relapse of the patient from or as a result of the treatment with the antagonist.

"A patient maintains responsiveness to a treatment" when the patient' responsiveness does not decrease with time during the course of a treatment.

As used herein, "baseline," means a clinical (e.g., signs or symptoms) or laboratory value describing a patient's condition determined prior to administration of a certain therapeutic agent, e.g., an integrin beta7 antagonist. Examples of clinical or laboratory values for which baseline values can be determined include, but are not limited to, hematology and clinical chemistry values, such as hemoglobin, hematocrit, platelet count, sodium, potassium, chloride and the like, CDAI score, SES-CD score, PRO2 score, or other patient-reported outcome measures such as IBDQ or CD-PRO/SS.

The term "sample," as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. The sample can be obtained from a tissue for the subject of interest or from peripheral blood of the subject.

"A beta7 integrin antagonist" or "beta7 antagonist" refers to any molecule that inhibits one or more biological activities or blocking binding of beta7 integrin with one or more of its associated molecules. Antagonists of the invention can be used to modulate one or more aspects of integrin beta7 associated effects, including but not limited to association with alpha4 integrin subunit, association with alphaE integrin subunit, binding of alpha4beta7 integrin to MAdCAM, VCAM-1 or fibronectin and binding of alphaEbeta7 integrin to E-cadherin. These effects can be modulated by any biologically relevant mechanism, including disruption of ligand binding to beta7 subunit or to the alpha4beta7 or alphaEbeta7 dimeric integrin, and/or by disrupting association between the alpha and beta integrin subunits such that formation of the dimeric integrin is inhibited. In one embodiment of the invention, the beta7 antagonist is an anti-beta7 integrin antibody (or anti-beta7 antibody). In one embodiment, the anti-beta7 integrin antibody is a humanized anti-beta7 integrin antibody and more particularly a recombinant humanized monoclonal anti-beta7 antibody (or rhuMAb beta7). In some embodiments, the anti-beta7 antibodies of the present invention are anti-integrin beta7 antagonistic antibodies that inhibit or block the binding of beta7 subunit with alpha4 integrin subunit, association with alphaE integrin subunit, binding of alpha4beta7 integrin to MAdCAM, VCAM-1 or fibronectin and binding of alphaEbeta7 integrin to E-cadherin.

By "beta7 subunit" or "β7 subunit" is meant the human β7 integrin subunit (Erle et al., (1991) J. Biol. Chem. 266:11009-11016). The beta7 subunit associates with alpha4 integrin subunit, such as the human .alpha.4 subunit (Kilger and Holzmann (1995) J. Mol. Biol. 73:347-354). The alpha4beta7 integrin is reportedly expressed on a majority of mature lymphocytes, as well as a small population of thymocytes, bone marrow cells and mast cells. (Kilshaw and Murant (1991) Eur. J. Immunol. 21:2591-2597; Gurish *et al*., (1992) 149: 1964-1972; and Shaw, S. K. and Brenner, M. B. (1995) Semin. Immunol. 7:335). The beta7 subunit also associates with the alphaE subunit, such as the human alphaE integrin subunit (Cepek, K. L, et al. (1993) J. Immunol. 150:3459). The alphaEbeta7 integrin is expressed on intra-intestinal epithelial lymphocytes (iIELs) (Cepek, K. L. (1993) *supra*)*.*

By "alphaE subunit" or "alphaE integrin subunit" or "αE subunit" or "αE integrin subunit" or "CD103" is meant an integrin subunit found to be associated with beta7 integrin on intra-epithelial lymphocytes, which alphaEbeta7 integrin mediates binding of the iELs to intestinal epithelium expressing E-cadherin (Cepek, K. L. et al. (1993) J. Immunol. 150:3459; Shaw, S. K. and Brenner, M. B. (1995) Semin. Immunol. 7:335).

"MAdCAM" or "MAdCAM-1" are used interchangeably in the context of the present invention and refer to the protein mucosal addressin cell adhesion molecule-1, which is a single chain polypeptide comprising a short cytoplasmic tail, a transmembrane region and an extracellular sequence composed of three immunoglobulin-like domains. The cDNAs for murine, human and macaque MAdCAM-1 have been cloned (Briskin, et al., (1993) Nature, 363:461-464; Shyjan et al., (1996) J. Immunol. 156:2851-2857).

"VCAM-1" or "vascular cell adhesion molecule-1" "CD106" refers to a ligand of alpha4beta7 and alpha4beta1, expressed on activated endothelium and important in endothelial-leukocyte interactions such as binding and transmigration of leukocytes during inflammation.

"CD45" refers to a protein of the protein tyrosine phosphatase (PTP) family. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. This gene is specifically expressed in hematopoietic cells. This PTP has been shown to be an essential regulator of T- and B-cell antigen receptor signaling. It functions through either direct interaction with components of the antigen receptor complexes, or by activating various Src family kinases required for the antigen receptor signaling. This PTP also suppresses JAK kinases, and thus functions as a regulator of cytokine receptor signaling. Four alternatively spliced transcripts variants of this gene, which encode distinct isoforms, have been reported. (Tchilian EZ, Beverley PC (2002). "CD45 in memory and disease." Arch. Immunol. Ther. Exp. (Warsz.) 50 (2): 85-93. Ishikawa H, Tsuyama N, Abroun S, et al. (2004). "Interleukin-6, CD45 and the src-kinases in myeloma cell proliferation." Leuk. Lymphoma 44 (9): 1477-81.

Various isoforms of CD45 exist: CD45RA, CD45RB, CD45RC, CD45RAB, CD45RAC, CD45RBC, CD45RO, CD45R (ABC). CD45 is also highly glycosylated. CD45R is the longest protein and migrates at 200 kDa when isolated from T cells. B cells also express CD45R with heavier glycosylation, bringing the molecular weight to 220 kDa, hence the name B220; B cell isoform of 220 kDa. B220 expression is not restricted to B cells and can also be expressed on activated T cells, on a subset of dendritic cells and other antigen presenting cells. Stanton T, Boxall S, Bennett A, et al. (2004). "CD45 variant alleles: possibly increased frequency of a novel exon 4 CD45 polymorphism in HIV seropositive Ugandans." Immunogenetics 56 (2): 107-10.

"Gut-homing lymphocytes" refer to a subgroup of lymphocytes having the characteristic of selectively homing to intestinal lymph nodes and tissues but not homing to peripheral lymph nodes and tissues. This subgroup of lymphocytes are characterized by an unique expression pattern of a combination of multiples cell surface molecules, including, but not limited to, the combination of CD4, CD45RA and Beta7. Typically, at least two subsets of peripheral blood CD4⁺ lymphocytes can be subdivided based on the markers of CD45RA and Beta7, CD45RA⁻ β7_{□}^{high} , and CD45RA⁻ β7_{□}^{low} CD4⁺ cells. CD45RA⁻ β7_{□}^{high} CD4⁺ cells home preferentially to intestinal lymph nodes and tissues, whereas CD45RA⁻ β7_{□}^{low} CD4⁺ cells home preferentially to peripheral lymph nodes and tissues (Rott *et al.* 1996; Rott *et al.* 1997; Williams *et al.* 1998; Rose *et al.* 1998; Williams and Butcher 1997; Butcher *et al.* 1999). Gut-homing lymphocytes are therefore a distinctive subgroup of lymphocytes identified as CD45RA⁻ β7 ^{high} CD4⁺ in a flow cytometry assay. The methods of identifying this group of lymphocytes are well-known in the art and also disclosed in detail in Examples of the present application.

As used herein with respect to a cell surface marker, the symbol "+" indicates a positive expression of a cell surface marker. For instance, CD4⁺ lymphocytes are a group of lymphocytes having CD4 expressed on their cell surfaces.

As used herein with respect to a cell surface marker, the symbol "-" indicates a negative expression of a cell surface marker. For instance, CD45RA⁻ lymphocytes are a group of lymphocytes having no CD45RA expressed on their cell surfaces.

As used herein with respect to the expression of a cell surface marker, the symbol "low" indicates a relatively low level of expression of a cell surface marker on lymphocytes, while "high" indicates a relatively high level of expression of a cell surface marker on lymphocytes. In a flow cytometry, the intensity of β7 ^{high} is at least about 10 or 100 fold higher than that of β7 ^{low}. Thus, as provided herein in exemplary embodiments, the CD45RA⁻ β7 ^{low} CD4⁺ and CD45RA⁻ β7 ^{high} CD4⁺ lymphocytes locate in distinct portions of a dot plot or histogram of a flow cytometry analysis where X-axis is the intensity of expression of CD45AR and Y-axis is the intensity of the expression of Beta7.

"Peripheral-homing lymphocytes" refer to a subgroup of lymphocytes having the characteristic of homing to peripheral lymph nodes and tissues and not homing to intestinal lymph nodes and tissues. In an exemplary embodiment, as explained above, Peripheral-homing lymphocytes are a distinctive group of lymphocytes identified as CD45RA⁻ β7 ^{low} CD4⁺ cells in a flow cytometry assay. The methods of identifying this group of lymphocytes are known in the art.

"Gastrointestinal inflammatory disorders" are a group of chronic disorders that cause inflammation and/or ulceration in the mucous membrane. These disorders include, for example, inflammatory bowel disease (e.g., Crohn's disease, ulcerative colitis, indeterminate colitis and infectious colitis), mucositis (e.g., oral mucositis, gastrointestinal mucositis, nasal mucositis and proctitis), necrotizing enterocolitis and esophagitis.

"Inflammatory Bowel Disease" or "IBD" is used interchangeably herein to refer to diseases of the bowel that cause inflammation and/or ulceration and includes without limitation Crohn's disease and ulcerative colitis.

"Crohn's disease (CD)" and "ulcerative colitis (UC)" are chronic inflammatory bowel diseases of unknown etiology. Crohn's disease, unlike ulcerative colitis, can affect any part of the bowel. The most prominent feature Crohn's disease is the granular, reddish-purple edematous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual.

Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

Anti-inflammatory drugs sulfasalazine and 5-aminosalisylic acid (5-ASA) are used for treating mildly active colonic Crohn's disease and are commonly prescribed in an attempt to maintain remission of the disease. Metroidazole and ciprofloxacin are similar in efficacy to sulfasalazine and are particularly prescribed for treating perianal disease. In more severe cases, corticosteroids are prescribed to treat active exacerbations and can sometimes maintain remission. Azathioprine and 6-mercaptopurine have also been used in patients who require chronic administration of corticosteroids. It has been suggested that these drugs may play a role in the long-term prophylaxis. Unfortunately, there can be a very long delay (up to six months) before onset of action in some patients. Antidiarrheal drugs can also provide symptomatic relief in some patients. Nutritional therapy or elemental diet can improve the nutritional status of patients and induce symptomatic improvement of acute disease, but it does not induce sustained clinical remissions. Antibiotics are used in treating secondary small bowel bacterial overgrowth and in treatment of pyogenic complications.

An "effective dosage" refers to an amount effective, at dosages and for periods of time necessary, to achieve the desired therapeutic or prophylactic result.

As used herein, the term "patient" refers to any single subject for which treatment is desired. In certain embodiments, the patient herein is a human.

A "subject" herein is typically a human. In certain embodiments, a subject is a non-human mammal. Exemplary non-human mammals include laboratory, domestic, pet, sport, and stock animals, e.g., mice, cats, dogs, horses, and cows. Typically, the subject is eligible for treatment, e.g., treatment of a gastrointestinal inflammatory disorder.

As used herein, "lifetime" of a subject refers to the remainder of the life of the subject after starting treatment.

The terms "inadequate response," "loss of response," and "refractory" are used interchangeably herein and refer to the persistence of or reappearance of signs or symptoms of active disease despite a history of treatment with one or more therapeutics, for example, corticosteroids, immunosuppressants and/or anti-TNFs.

The terms "antibody" and "immunoglobulin" are used interchangeably in the broadest sense and include monoclonal antibodies (for example, full length or intact monoclonal antibodies), polyclonal antibodies, multivalent antibodies, multispecific antibodies (e.g., bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, and typically most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, *e.g.,* Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, *e.g.,* Kozbor J. Immunol, 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (e.g., mice) that are capable of producing a full repertoire of human antibodies in the absence of endogenous immunoglobulin production (see, *e.g.,* Jakobovits et al., Proc. Natl. Acad. Sci USA, 90: 2551 (1993); Jakobovits et al., Nature, 362: 255 (1993); Bruggermann et al.,Year in Immunol., 7: 33 (1993)). This definition of a human antibody specifically excludes a humanized antibody comprising antigen-binding residues from a non-human animal.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In certain embodiments, the antibody will be purified (1) to greater than 95% by weight of antibody as determined by the Lowry method, and often more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or silver stain. Isolated antibody includes the antibody in situ within recombinant cells since at least one component of the antibody's natural environment will not be present. Ordinarily, however, isolated antibody will be prepared by at least one purification step.

The term "hypervariable region," "HVR," or "HV," when used herein refers to the regions of an antibody variable domain which are hypervariable in sequence and/or form structurally defined loops. Generally, antibodies comprise six hypervariable regions; three in the VH (HI, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

Hypervariable regions may comprise "extended hypervariable regions" as follows: 24-36 or 24-34 (L1), 46-56 or 49-56 or 50-56 or 52-56 (L2) and 89-97 (L3) in the VL and 26-35 (HI), 50-65 or 49-65 (H2) and 93-102, 94-102 or 95-102 (H3) in the VH. The variable domain residues are numbered according to Kabat *et al*., *supra* for each of these definitions.

"Framework" or "FR" residues are those variable domain residues other than the hypervariable region residues as herein defined.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat *et al.* In one embodiment, for the VL, the subgroup is subgroup kappa I as in Kabat *et al.* In one embodiment, for the VH, the subgroup is subgroup III as in Kabat *et al.*

An "affinity matured" antibody is one with one or more alterations in one or more CDRs thereof which result in an improvement in the affinity of the antibody for antigen, compared to a parent antibody which does not possess those alteration(s). In certain embodiments, affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1996); Yelton et al. J. Immunol. 155:1994-2004 (1995); Jackson et al., J. Immunol. 154(7):3310-9 (1995); and Hawkins et al. J. Mol. Biol. 226:889-896 (1992).

The phrase "substantially similar," or "substantially the same," as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristic measured by said values *(e.g.,* Kd values). The difference between said two values is less than about 50%, less than about 40%, less than about 30%, less than about 20%, less than about 10% as a function of the value for the reference/comparator antibody.

"Binding affinity" generally refers to the strength of the sum total of noncovalent interactions between a single binding site of a molecule *(e.g.,* an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CHI) of the heavy chain. Fab= fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG₁, IgG₂, IgG₃, IgG₄, IgA₁, and IgA₂. The heavy-chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W. B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors *(e.g.,* B cell receptor; BCR), etc. Such effector functions generally require the Fc region to be combined with a binding domain *(e.g.,* an antibody variable domain) and can be assessed using various assays as herein disclosed, for example.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification. In certain embodiments, the variant Fc region has at least one amino acid substitution compared to a native sequence Fc region or to the Fc region of a parent polypeptide, e.g., from about one to about ten amino acid substitutions, and in certain embodiments from about one to about five amino acid substitutions in a native sequence Fc region or in the Fc region of the parent polypeptide. In certain embodiments, the variant Fc region herein will possess at least about 80% homology with a native sequence Fc region and/or with an Fc region of a parent polypeptide, or at least about 90% homology therewith, or at least about 95% homology therewith.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g.,* IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcyRI, FcyRII and FcyRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95:652-656 (1998).

"Human effector cells" are leukocytes which express one or more FcRs and perform effector functions. In certain embodiments, the cells express at least FcyRIII and perform ADCC effector function. Examples of human leukocytes which mediate ADCC include peripheral blood mononuclear cells (PBMC), natural killer (NK) cells, monocytes, cytotoxic T cells and neutrophils. The effector cells may be isolated from a native source thereof, *e.g.,* from blood or PBMCs as described herein.

The terms "Fc receptor" or "FcR" are used to describe a receptor that binds to the Fc region of an antibody. In certain embodiments, FcR is a native sequence human FcR. Moreover, FcR is one which binds an IgG antibody (a gamma receptor) and includes receptors of the FcyRI, FcyRII, and FcyRIII subclasses, including allelic variants and alternatively spliced forms of these receptors. FcyRII receptors include FcyRIIA (an "activating receptor") and FcγRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)), and regulates homeostasis of immunoglobulins. Antibodies with improved binding to the neonatal Fc receptor (FcRn), and increased half-lives, are described in WO00/42072 (Presta, L.) and US2005/0014934A1 (Hinton et al.). These antibodies comprise an Fc region with one or more substitutions therein which improve binding of the Fc region to FcRn. For example, the Fc region may have substitutions at one or more of positions 238, 250, 256, 265, 272, 286, 303, 305, 307, 311, 312, 314, 317, 340, 356, 360, 362, 376, 378, 380, 382, 413, 424, 428 or 434 (Eu numbering of residues). In certain embodiments, the Fc region-comprising antibody variant with improved FcRn binding comprises amino acid substitutions at one, two or three of positions 307, 380 and 434 of the Fc region thereof (Eu numbering of residues).

"Single-chain Fv" or "scFv" antibody fragments comprise the V_{H} and V_{L} domains of antibody, wherein these domains are present in a single polypeptide chain. In certain embodiments, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994). HER2 antibody scFv fragments are described in WO93/16185; U.S. Patent No. 5,571,894; and U.S. Patent No. 5,587,458.

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "affinity matured" antibody is one with one or more alterations in one or more hypervariable regions thereof which result an improvement in the affinity of the antibody for antigen, compared to a parent antibody which does not possess those alteration(s). In certain embodiments, affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. In certain embodiments, amino acid sequence variants will possess at least about 70% homology with the main species antibody, or they will be at least about 80%, or at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (e.g., deamidated antibody variant), a basic variant, an antibody with an amino-terminal leader extension (e.g. VHS-) on one or two light chains thereof, an antibody with a C-terminal lysine residue on one or two heavy chains thereof, etc, and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieties attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc, and combinations of glycosylation alterations. Where the antibody has an Fc region, an oligosaccharide structure may be attached to one or two heavy chains of the antibody, e.g. at residue 299 (298, Eu numbering of residues).

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-a, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "immunosuppressive agent" as used herein for adjunct therapy refers to substances that act to suppress or mask the immune system of the subject being treated herein. This would include substances that suppress cytokine production, down-regulate or suppress self-antigen expression, or mask the MHC antigens. Examples of such agents include 2-amino-6-aryl-5-substituted pyrimidines (see U.S. Patent No. 4,665,077); non-steroidal anti-inflammatory drugs (NSAIDs); ganciclovir; tacrolimus; glucocorticoids such as cortisol or aldosterone; anti-inflammatory agents such as a cyclooxygenase inhibitor; a 5-lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6 mercaptopurine; steroids such as corticosteroids or glucocorticosteroids or glucocorticoid analogs, e.g., prednisone (including prednisone equivalents, for example, including but not limited to, methylprednisolone, including SOLU-MEDROL.RTM. methylprednisolone sodium succinate, and cortef [hydrocortisone]), budesonide, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or antagonists including anti-interferon-alpha, -beta, or -gamma antibodies, anti-tumor necrosis factor(TNF)-alpha antibodies (infliximab (REMICADE.RTM.) or adalimumab), anti-TNF-alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CD11a and anti-CD 18 antibodies; anti-L3T4 antibodies; heterologous anti-lymphocyte globulin; pan-T antibodies, anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor (Cohen et al., U.S. Patent No. 5,114,721); T-cell receptor fragments (Offner et al., Science, 251: 430-432 (1991); WO 90/11294; Ianeway, Nature, 341: 482 (1989); and WO 91/01133); BAFF antagonists such as BAFF or BR3 antibodies or immunoadhesins and zTNF4 antagonists (for review, see Mackay and Mackay, Trends Immunol., 23:113-5 (2002) and see also definition below); biologic agents that interfere with T cell helper signals, such as anti-CD40 receptor or anti-CD40 ligand (CD154), including blocking antibodies to CD40-CD40 ligand.(e.g., Durie etal., Science, 261: 1328-30 (1993); Mohan et al., J. Immunol., 154: 1470-80 (1995)) and CTLA4-Ig (Finck et al., Science, 265: 1225-7 (1994)); and T-cell receptor antibodies (EP 340,109) such as T10B9.

As used herein, "corticosteroid-free" means that a patient, e.g. a patient with Crohn's disease, did not use corticosteroids to treat the disease or symptoms of the disease during the time which the patient is corticosteroid-free. For example, a patient with Crohn's disease who is corticosteroid-free for 12 months did not use corticosteroids for 12 months to treat symptoms of Crohn's disease.

The term "ameliorates" or "amelioration" as used herein refers to a decrease, reduction or elimination of a condition, disease, disorder, or phenotype, including an abnormality or symptom.

A "symptom" of a disease or disorder (e.g., inflammatory bowel disease, e.g., ulcerative colitis or Crohn's disease) is any morbid phenomenon or departure from the normal in structure, function, or sensation, experienced by a subject and indicative of disease.

The expression "therapeutically effective amount" refers to an amount that is effective for preventing, ameliorating, or treating a disease or disorder (e.g., inflammatory bowel disease, e.g., ulcerative colitis or Crohn's disease). For example, a "therapeutically effective amount" of an antibody refers to an amount of the antibody that is effective for preventing, ameliorating, or treating the specified disease or disorder. Similarly, a "therapeutically effective amount" of a combination of an antibody and a second compound refers to an amount of the antibody and an amount of the second compound that, in combination, is effective for preventing, ameliorating, or treating the specified disease or disorder.

It is to be understood that the terminology "a combination of' two compounds does not mean that the compounds have to be administered in admixture with each other. Thus, treatment with or use of such a combination encompasses a mixture of the compounds or separate administration of the compounds, and includes administration on the same day or different days. Thus the terminology "combination" means two or more compounds are used for the treatment, either individually or in admixture with each other. When an antibody and a second compound, for example, are administered in combination to a subject, the antibody is present in the subject at a time when the second compound is also present in the subject, whether the antibody and second compound are administered individually or in admixture to the subject. In certain embodiments, a compound other than the antibody is administered prior to the antibody. In certain embodiments, a compound other than the antibody is administered after the antibody.

For the purposes herein, "tumor necrosis factor-alpha (TNF-alpha)" refers to a human TNF-alpha molecule comprising the amino acid sequence as described in Pennica et al., Nature, 312:721 (1984) or Aggarwal et al., JBC, 260:2345 (1985).

A "TNF-alpha inhibitor" herein is an agent that inhibits, to some extent, a biological function of TNF-alpha, generally through binding to TNF-alpha and neutralizing its activity. Examples of TNF inhibitors specifically contemplated herein are etanercept (ENBREL^{®}), infliximab (REMICADE^{®}), adalimumab (HUMIRA^{®}), golimumab (SIMPONITM), and certolizumab pegol (CIMZIA^{®}).

"Corticosteroid" refers to any one of several synthetic or naturally occurring substances with the general chemical structure of steroids that mimic or augment the effects of the naturally occurring corticosteroids. Examples of synthetic corticosteroids include prednisone, prednisolone (including methylprednisolone), dexamethasone triamcinolone, budesonide, and betamethasone.

An "antagonist" refers to a molecule capable of neutralizing, blocking, inhibiting, abrogating, reducing or interfering with the activities of a particular or specified protein, including its binding to one or more receptors in the case of a ligand or binding to one or more ligands in case of a receptor. Antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of the protein, and fusion proteins, receptor molecules and derivatives which bind specifically to the protein thereby sequestering its binding to its target, antagonist variants of the protein, antisense molecules directed to the protein, RNA aptamers, and ribozymes against the protein.

A "self-inject device" refers to a medical device for self-administration, e.g., by a patient or in-home caregiver, of a therapeutic agent. Self-inject devices include autoinjector devices and other devices designed for self-administration.

A variety of additional terms are defined or otherwise characterized herein.

### COMPOSITIONS AND METHODS

### A. Beta7 integrin Antagonists

Methods of treating a gastrointestinal inflammatory disorder in a subject, e.g., a human, by administering beta7 integrin antagonists are provided. Examples of potential antagonists include an oligonucleotide that binds to the fusions of immunoglobulin with beta7 integrin, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the beta7 integrin that recognizes the ligand but imparts no effect, thereby competitively inhibiting the action of the beta7 integrin.

Another potential beta7 integrin antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the beta7 integrin herein, is used to design an antisense RNA oligonucleotide of from about 10 to 40 base pairs in length. A DNA oligonucleotide is designed to be complementary to a region of the gene involved in transcription (triple helix--see Lee et al., Nucl. Acids Res., 6:3073 (1979); Cooney et al., Science, 241: 456 (1988); Dervan et al., Science, 251:1360 (1991)), thereby preventing transcription and the production of the beta7 integrin. The antisense RNA oligonucleotide hybridizes to the mRNA *in vivo* and blocks translation of the mRNA molecule into beta7 integrin protein (antisense--Okano, Neurochem., 56:560 (1991); Oligodeoxynucleotides as Antisense Inhibitors of Gene Expression (CRC Press: Boca Raton, Fla., 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, e.g., between about -10 and +10 positions of the target gene nucleotide sequence, are typical.

Other potential antagonists include small molecules that bind to the active site, the ligand or binding molecule binding site, thereby blocking the normal biological activity of the beta7 integrin. Examples of small molecules include, but are not limited to, small peptides or peptide-like molecules, typically soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can-be identified by known techniques. For further details see, *e.g.,* Rossi, Current Biology, 4:469-471 (1994), and PCT Publication No. WO 97/33551 (published Sep. 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.,* PCT Publication No. WO 97/33551. These small molecules can be identified by any one or more of the screening assays discussed hereinabove and/or by any other screening techniques well known for those skilled in the art.

Screening assays for antagonists are designed to identify compounds that bind or complex with the beta7 integrin encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

### B. Anti-Beta7 integrin Antibodies

In one embodiment, the beta7 integrin antagonists are anti-beta7 antibodies. Exemplary antibodies include polyclonal, monoclonal, humanized, human, bispecific, and heteroconjugate antibodies, etc., as described below.

### 1. Polyclonal Antibodies

Polyclonal antibodies can be raised in animals by multiple subcutaneous (SC) or intraperitoneal (IP) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. In certain embodiments, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (see, e.g., U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium which medium may contain one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

In certain embodiments, fusion partner myeloma cells are those that fuse efficiently, support stable high-level production of antibody by the selected antibody-producing cells, and are sensitive to a selective medium that selects against the unfused parental cells. In certain embodiments, myeloma cell lines are murine myeloma lines, such as those derived from MOPC-21 and MPC-11 mouse tumors available from the Salk Institute Cell Distribution Center, San Diego, Calif. USA, and SP-2 and derivatives e.g., X63-Ag8-653 cells available from the American Type Culture Collection, Manassas, Va., USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. In certain embodiments, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980). Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal e.g., by i.p. injection of the cells into mice. The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs. 130:151-188 (1992).

In a further embodiment, monoclonal antibodies or antibody fragments can be isolated from antibody phage libraries generated using e.g., the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (CH and CL) sequences for the homologous murine sequences (U.S. Patent No. 4,816,567; and Morrison, et al., Proc. Natl. Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Exemplary anti-beta7 antibodies are Fib504, Fib 21, 22, 27, 30 (Tidswell, M. J Immunol. 1997 Aug 1;159(3):1497-505) or humanized derivatives thereof. Humanized antibodies of Fib504 was disclosed in detail in U.S. Patent Publication No. 20060093601 (issued as U.S. Patent No. 7,528,236), the content of which is incorporated by reference in its entirety (also see discussion below).

### 3. Human and Humanized Antibodies

The anti-beta7 integrin antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)). It is further important that antibodies be humanized with retention of high binding affinity for the antigen and other favorable biological properties. To achieve this goal, according to certain embodiments, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized Anti-beta7 integrin antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

Exemplary humanized anti-beta7 antibodies include, but are not limited to rhuMAb Beta7, which is a humanized monoclonal antibody against the integrin subunit β7 and was derived from the rat anti-mouse/human monoclonal antibody FIB504 (Andrew et al., 1994 J Immunol 1994;153:3847-61). It has been engineered to include human immunoglobulin IgG1 heavy chain and κ1 light chain frameworks and is produced by Chinese hamster ovary cells. This antibody binds to two integrins, α4β7 (Holzmann et al. 1989 Cell, 1989;56:37-46; Hu et al., 1992, Proc Natl Acad Sci USA 1992;89:8254-8) and αEβ7 (Cepek et al., 1993 J Immunol 1993;150:3459-70), which regulate trafficking and retention of lymphocyte subsets in the gastrointestinal tract and are involved in inflammatory bowel diseases (IBD) such as ulcerative colitis (UC) and Crohn's disease (CD). rhuMAb Beta7 is a potent *in vitro* blocker of the cellular interaction between α4β7 and its ligands (mucosal addressin cell adhesion molecule-1 [MAdCAM]-1, vascular cell adhesion molecule [VCAM]-1, and fibronectin) as well as the interaction between αEβ7 and its ligand (E-cadherin). rhuMAb Beta7 binds reversibly, with similar high affinity, to β7 on lymphocytes from rabbits, cynomolgus monkeys, and humans. It also binds to mouse β7 with high affinity. The amino acid sequence as well as the making and using of rhuMAb Beta7 and its variants are disclosed in detail in e.g., U.S. Patent Application Publication No. 20060093601 (issued as U.S. Patent No. 7,528,236), the content of which is incorporated in its entirety.

FIGS. 1A and 1B depict alignment of sequences of the variable light and heavy chains for the following: light chain human subgroup kappa I consensus sequence (FIG. 1A, SEQ ID NO:12), heavy chain human subgroup III consensus sequence (FIG. 1B, SEQ ID NO:13), rat anti-mouse beta7 antibody (Fib504) variable light chain (FIG. 1A, SEQ ID NO:10), rat anti-mouse beta7 antibody (Fib504) variable heavy chain (FIG. 1B, SEQ ID NO:11), and humanized antibody variants: Humanized hu504Kgraft variable light chain (FIG. 1A, SEQ ID NO: 14), humanized hu504K graft variable heavy chain (FIG. 1B, SEQ ID NO:15), variants hu504-5, hu504-16, and hu504-32 (amino acid variations from humanized hu504K graft are indicated in FIG. 1A (light chain) (SEQ ID NOS:22-24, respectively, in order of appearance) and FIG. 1B (heavy chain) for variants hu504-5, hu504-16, and 504-32 (SEQ ID NO:25).

### 4. Human Antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all of GenPharm); U.S. Patent No. 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628(1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patent Nos. 5,567,610 and 5,229,275).

### 5. Antibody Fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," e.g., as described in US Patent No. 5,641,870 for example. Such linear antibody fragments may be monospecific or bispecific.

### 6. Bispecific Antibodies

Bispecific antibodies are antibodies that have binding specificities for at least two different epitopes. Exemplary bispecific antibodies may bind to two different epitopes of beta7 integrin as described herein. Other such antibodies may combine a TAT binding site with a binding site for another protein. Alternatively, an anti-Beta7 integrin arm may be combined with an arm which binds to a triggering molecule on a leukocyte such as a T-cell receptor molecule *(e.g.,* CD3), or Fc receptors for IgG (Fc.y.R), such as Fc.yRI (CD64), Fc.yRII (CD32) and Fc. γ.RIII (CD16), so as to focus and localize cellular defense mechanisms to the TAT-expressing cell. Bispecific antibodies may also be used to localize cytotoxic agents to cells which express TAT. These antibodies possess a TAT-binding arm and an arm which binds the cytotoxic agent (e.g., saporin, anti-interferon-.alpha., vinca alkaloid, ricin A chain, methotrexate or radioactive isotope hapten). Bispecific antibodies can be prepared as full length antibodies or antibody fragments (e.g., F(ab')₂ bispecific antibodies).

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et a/., EMBO J. 10:3655-3659 (1991).

According to a different approach, antibody variable domains with the desired binding specificities (antibody-antigen combining sites) are fused to immunoglobulin constant domain sequences. In certain embodiments, the fusion is with an Ig heavy chain constant domain, comprising at least part of the hinge, C_{H2}, and C_{H3} regions. In certain embodiments, the first heavy-chain constant region (C_{H1}) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In certain embodiments, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. In certain embodiments, the interface comprises at least a part of the C_{H3} domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab').sub.2 fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives: One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. *coli,* which can be chemically coupled to form bispecific antibodies. Shalaby et al., J. Exp. Med. 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V.sub.H connected to a V.sub.L by a linker which is too short to allow pairing between the two domains on the same chain. Accordingly, the V.sub.H and V.sub.L domains of one fragment are forced to pair with the complementary V.sub.L and V.sub.H domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 7. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 8. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g., tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, the multivalent antibody herein comprises (or consists of) three to about eight, but typically four, antigen binding sites. The multivalent antibody comprises at least one polypeptide chain (and typically two polypeptide chains), wherein the polypeptide chain(s) comprise two or more variable domains. For instance, the polypeptide chain(s) may comprise VD1-(X1).sub.n-VD2-(X2).sub.n-Fc, wherein VD1 is a first variable domain, VD2 is a second variable domain, Fc is one polypeptide chain of an Fc region, XI and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (and typically four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 9. Effector Function Engineering

It may be desirable to modify the antibody of the invention with respect to effector function, *e.g.,* so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., AntiCancer Drug Design 3:219-230 (1989). To increase the serum half life of the antibody, one may incorporate a salvage receptor binding epitope into the antibody (especially an antibody fragment) as described in U.S. Patent No. 5,739,277, for example. As used herein, the term "salvage receptor binding epitope" refers to an epitope of the Fc region of an IgG molecule *(e.g.,* IgG₁, IgG₂, IgG₃, or IgG₄) that is responsible for increasing the *in vivo* serum half-life of the IgG molecule.

### 10. Immunoconjugates

The antagonist or antibody used in the methods herein is optionally conjugated to another agent, such as a cytotoxic agent, or cytokine.

Conjugation will ordinarily be achieved through a covalent linkage, the precise nature of which will be determined by the targeting molecule and the linking site on the integrin beta7 antagonist or antibody polypeptide. Typically, a non-peptidic agent is modified by the addition of a linker that allows conjugation to anti-beta7 integrin antibody through its amino acid side chains, carbohydrate chains, or reactive groups introduced on antibody by chemical modification. For example, a drug may be attached through the .epsilon.-amino group of a lysine residue, through a free .alpha.-amino group, by disulfide exchange to a cysteine residue, or by oxidation of the 1,2- diols in a carbohydrate chain with periodic acid to allow attachment of drugs containing various nucleophiles through a Schiff-base linkage. See, for example, U.S. Patent No. 4,256,833. Protein modifying agents include amine-reactive reagents (e.g., reactive esters, isothiocyanates, aldehydes, and sulfonyl halides), thiol-reactive reagents (e.g., haloacetyl derivatives and maleimides), and carboxylic acid- and aldehyde-reactive reagents. Integrin beta7 antagonist or antibody polypeptides can be covalently joined to peptidic agents through the use of bifunctional cross-linking reagents. Heterobifunctional reagents are more commonly used and permit the controlled coupling of two different proteins through the use of two different reactive moieties (e.g., amine-reactive plus thiol, iodoacetamide, or maleimide). The use of such linking agents is well known in the art. See, for example, Brinkley, *supra,* and U.S. Patent No. 4,671,958. Peptidic linkers can also be employed. In the alternative, an anti-beta7 integrin antibody polypeptide can be linked to a peptidic moiety through preparation of a fusion polypeptide.

Examples of further bifunctional protein coupling agents include N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### 11. Immunoliposomes

The anti-beta7 integrin antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Patent Nos. 4,485,045 and 4,544,545; and WO97/38731 published Oct. 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 12. Vectors, Host Cells and Recombinant Methods for Antibody Production

Also provided are isolated nucleic acids encoding the anti-beta7 antibodies or polypeptide agents described herein, vectors and host cells comprising the nucleic acids and recombinant techniques for the production of the antibodies.

For recombinant production of the antibody, the nucleic acid encoding it may be isolated and inserted into a replicable vector for further cloning (amplification of the DNA) or for expression. In another embodiment, the antibody may be produced by homologous recombination, e.g., as described in U.S. Patent No. 5,204,244, specifically incorporated herein by reference. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence, e.g., as described in U.S. Patent No. 5,534,615 issued Jul. 9, 1996 and specifically incorporated herein by reference.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, *e.g., E. coli,* Enterobacter, Erwinia, Klebsiella, Proteus, Salmonella, *e.g.,* Salmonella typhimurium, Serratia, *e.g.,* Serratia marcescans, and Shigella, as well as Bacilli such as B. subtilis and B. licheniformis (e.g., B. licheniformis 41P disclosed in DD 266,710 published 12 Apr. 1989), Pseudomonas such as P. aeruginosa, and Streptomyces. One *E. coli* cloning host is *E. coli* 294 (ATCC 31,446), although other strains such as E. *coli B, E. coli* X1776 (ATCC 31,537), and E. *coli* W3110 (ATCC 27,325) are suitable. These examples are illustrative rather than limiting.

In addition to prokaryotes, eukaryotic microbes such as filamentous fungi or yeast are suitable cloning or expression hosts for anti-beta7 integrin antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.,* Neurospora, Penicillium, Tolypocladium, and Aspergillus hosts such as A. nidulans and A. niger.

Suitable host cells for the expression of glycosylated anti-Beta7 antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-l variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR(CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for anti-beta7 integrin antibody production and cultured in conventional nutrient media modified as appropriate for inducing promoters, selecting transformants, or amplifying the genes encoding the desired sequences.

The host cells used to produce the anti-beta7 integrin antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 1 02:255 (1980), U.S. Patent Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN.TM.drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the typical purification technique. The suitability of protein A as an affinity ligand depends on the species and isotype of any immunoglobulin Fc domain that is present in the antibody. Protein A can be used to purify antibodies that are based on human .gamma.1, .gamma.2, or .gamma.4 heavy chains (Lindmark et al., J. Immunol. Meth. 62:1-13 (1983)). Protein G is recommended for all mouse isotypes and for human .gamma.3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C.sub.H3 domain, the Bakerbond ABX.TM.resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE.TM. chromatography on an anion or cation exchange resin (such as a polyaspartic acid column), chromatofocusing, SDS-PAGE, and ammonium sulfate precipitation are also available depending on the antibody to be recovered. Following any preliminary purification step(s), the mixture comprising the antibody of interest and contaminants may be subjected to low pH hydrophobic interaction chromatography using an elution buffer at a pH between about 2.5-4.5, typically performed at low salt concentrations (*e.g.,* from about 0-0.25 M salt).

### C. Pharmaceutical Formulations

Therapeutic formulations comprising the therapeutic agents, antagonists or antibodies of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN.TM., PLURONICS.TM. or polyethylene glycol (PEG).

The formulation herein may also contain more than one active compound as necessary for the particular indication being treated, typically those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and .gamma. ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### D. Administration

The physician administering treatment will be able to determine the appropriate dose for the individual subject by following the instructions on the label. Preparation and dosing schedules for commercially available second therapeutic and other compounds administered in combination with or concomitantly with the integrin beta7 antagonists may be used according to manufacturers' instructions or determined empirically by the skilled practitioner.

In certain embodiments, the integrin beta7 antagonist is administered once every four weeks or at 0, 2, and four weeks followed by once every four weeks for a period of one month (4 weeks), or two months, three months, or six months, or 12 months, or 18 months, or 24 months, or chronically for the lifetime of the patient. In certain embodiments, the treatment is self-administered by the patient. In certain embodiments, the patient self-administers using an autoinject device containing a prefilled syringe.

In certain embodiments, a flat dose of anti-beta7 antibody is administered to the patient. A flat dose is a particular amount of anti-beta7 antibody that is administered to every patient regardless of weight. Depending on the type and severity of the disease, a flat dose of between about 50 mg and 450 mg of anti-beta7 antibody is administered to the patient, which may be one or more separate injections or infusions or administrations. Such flat dose can be administered subcutaneously. In certain embodiments, the flat dose is about 100 mg or about 200 mg or about 300 mg or about 400 mg or about 450 mg. In certain embodiments, the flat dose is about 105 mg or about 210 mg. In certain embodiments, the flat dose is 105 mg. In certain embodiments the flat dose is 210 mg. In certain embodiments, a flat dose is administered once every four weeks subcutaneously.

In certain embodiments, an initial flat loading dose of anti-beta7 antibody is followed by one or more flat maintenance doses of anti-beta7 antibody. The loading dose is a larger quantity of anti-beta7 antibody than the maintenance dose. In certain embodiments, the loading dose is about 200 mg or about 210 mg. In certain embodiments, the loading dose is 210 mg. In certain embodiments, 210 mg of the anti-beta7 antibody is administered at week 0, week 2, week 4, week 8, and week 12, and the maintenance dose is administered once every 4 weeks thereafter. In certain embodiments, the maintenance dose is about 100 mg or about 105 mg. In certain embodiments, the maintenance dose is 105 mg.

In certain embodiments, the anti-beta7 antibody is administered for a period of 14 weeks. In certain embodiments, the anti-beta7 antibody is administered for a period of three months. In certain embodiments, the anti-beta7 antibody is administered for a period of six months. In certain embodiments, the anti-beta7 antibody is administered for a period of at least 12 months (52 weeks). In certain embodiments, the anti-beta7 antibody is administered for a period of at least 66 weeks. In certain embodiments, the anti-beta7 antibody is administered for a period of at least 70 weeks. In certain embodiments, the anti-beta7 antibody is administered for a period of at least 74 weeks. In certain embodiments, the anti-beta7 antibody is administered for the lifetime of the subject.

Typically, the clinician will administer an antibody (alone or in combination with a second compound) of the invention until a dosage(s) is reached that provides the required biological effect. The progress of the therapy of the invention can be monitored by methods described herein, for example, including but not limited to Crohn's Disease Activity Index (CDAI) score, Patient Reported Outcome 2 (PRO2) score, and Simplified Endoscopic Index for Crohn's Disease (SES-CD) score.

In certain embodiments, an anti-beta7 antibody is administered using, for example, a self-inject device, autoinjector device, or other device designed for self-administration. Various self-inject devices, including autoinjector devices, are known in the art and are commercially available. Exemplary devices include, but are not limited to, prefilled syringes (such as BD HYPAK SCF^{®}, READYFILLTM, and STERIFILL SCFTM from Becton Dickinson; CLEARSHOTTM copolymer prefilled syringes from Baxter; and Daikyo Seiko CRYSTAL ZENITH^{®} prefilled syringes available from West Pharmaceutical Services); disposable pen injection devices such as BD Pen from Becton Dickinson; ultra-sharp and microneedle devices (such as INJECT-EASETM and microinfuser devices from Becton Dickinson; and H-PATCHTM available from Valeritas) as well as needle-free injection devices (such as BIOJECTOR^{®} and IJECT^{®} available from Bioject; and SOF-SERTER^{®} and patch devices available from Medtronic). In certain embodiments, an article of manufacture is provided comprising a prefilled syringe comprising a single dose of etrolizumab. In certain embodiments, an article of manufacture is provided comprising a prefilled syring and autoinjector combination comprising a single dose of etrolizumab.

As noted, the integrin beta7 antagonist can be administered alone or in combination with at least a second therapeutic compound. These second therapeutic compounds are generally used in the same dosages and with administration routes as used heretofore, or about from 1 to 99% of the heretofore-employed dosages. If such second compounds are used, they are used in certain embodiments in lower amounts than if the integrin beta7 antagonist were not present, so as to eliminate or reduce side effects caused thereby.

Also as noted (e.g., see below), a variety of suitable second therapeutic compounds for the treatment of IBD, e.g., ulcerative colitis and Crohn's disease are known in the art, and dosages and administration methods for such second therapeutic compounds have likewise been described.

Administration of the integrin beta7 antagonist and any second therapeutic compound can be done simultaneously, e.g., as a single composition or as two or more distinct compositions using the same or different administration routes. Alternatively, or additionally, the administration can be done sequentially, in any order. In certain embodiments, intervals ranging from minutes to days, to weeks to months, can be present between the administrations of the two or more compositions. For example, the integrin beta7 antagonist may be administered first, followed by the second therapeutic compound. However, simultaneous administration or administration of the second therapeutic compound prior to the integrin beta7 antagonist is also contemplated.

The standard of care for subjects with moderately to severely active CD involves therapy with standard doses of: systemic corticosteroids, e.g., prednisone (or prednisone equivalent) or budesonide, immunosuppressants such as azathioprine, 6-mercaptopurine, or methotrexate, or tumor necrosis factor inhibitors (anti-TNFs), such as infliximab, adalimumab, or certolizumab pegol. Other anti-integrin therapies have been approved for treatment of CD and these are natalizumab and vedolizumab. Therapy with an integrin beta7 antagonist, such as an anti-beta7 integrin antibody as disclosed herein will result in an improvement in induction and/or maintenance of disease remission (rapid control of disease and/or prolonged remission), and/or clinical response, superior to that achieved with the standard of care, including anti-TNFs, for such subjects.

In one embodiment, the treatment of the present invention for Crohn's disease (CD) in a human subject with CD comprises administering to the subject an effective amount of a therapeutic agent, such as an anti-beta7 integrin antibody, and further comprising administering to the subject an effective amount of a second medicament, that is an immunosuppressant, a corticosteroid, an anti-TNF, a pain-control agent, an antidiarrheal agent, an antibiotic, or a combination thereof.

In an exemplary embodiment, said secondary medicine is selected from the group consisting of 6-mercaptopurine, azathioprine, methotrexate, prednisone (or prednisone equivalent), budesonide, infliximab, adalimumab, and certlizumab pegol.

All these second medicaments may be used in combination with each other or by themselves with the first medicament, so that the expression "second medicament" as used herein does not mean it is the only medicament besides the first medicament, respectively. Thus, the second medicament need not be one medicament, but may constitute or comprise more than one such drug.

Combined administration herein includes co-administration, using separate formulations or a single pharmaceutical formulation, and consecutive administration in either order, wherein generally there is a time period while both (or all) active agents simultaneously exert their biological activities.

The combined administration of a second medicament includes co-administration (concurrent administration), using separate formulations or a single pharmaceutical formulation, and consecutive administration in either order, wherein generally there is a time period while both (or all) active agents (medicaments) simultaneously exert their biological activities.

### E. Design Treatment Regimens

Drug development is a complex and expensive process. The cost of bringing a new drug to market is estimated to be between $800 million and $1 billion. Less than 10% of drugs in phase I clinical trials make it to the approval phase. Two key reasons why drugs fail at late stages are a lack of understanding of the relationship between dose-concentration response and unanticipated safety events. Given this scenario, it is important to have enabling tools that help predict how a drug will perform *in vivo* and assist in the success of a clinical therapeutic candidate (Lakshmi Kamath, Drug Discovery and Development; Modeling Success in PK/PD Testing Drug Discovery & Development (2006)).

Pharmacokinetics (PK) characterizes the absorption, distribution, metabolism, and elimination properties of a drug. Pharmacodynamics (PD) defines the physiological and biological response to the administered drug. PK/PD modeling establishes a mathematical and theoretical link between these two processes and helps better predict drug action. Integrated PK/PD modeling and computer-assisted trial design via simulation are being incorporated into many drug development programs and are having a growing impact (Lakshmi Kamath, Drug Discovery and Development; Modeling Success in PK/PD Testing Drug Discovery & Development (2006)).

PK/PD testing is typically performed at every stage of the drug development process. Because development is becoming increasingly complex, time consuming, and cost intensive, companies are looking to make better use of PK/PD data to eliminate flawed candidates at the beginning and identify those with the best chance of clinical success. (Lakshmi Kamath, *supra*).

PK/PD modeling approaches are proving useful in determining relationships between biomarker responses, drug levels, and dosing regimens. The PK/PD profile of a drug candidate and the ability to predict a patient's response to it are critical to the success of clinical trials. Recent advances in molecular biology techniques and a better understanding of targets for various diseases have validated biomarkers as a good clinical indicator of a drug's therapeutic efficacy. Biomarker assays help identify a biological response to a drug candidate. Once a biomarker is clinically validated, trial simulations can be effectively modeled. Biomarkers have the potential to achieve surrogate status that may someday substitute for clinical outcomes in drug development. (Lakshmi Kamath, *supra*)*.*

The amount of biomarkers in the peripheral blood can be used in identifying the biological response to a treatment with integrin beta7 antagonists and can therefore function as a good clinical indicator for the therapeutic efficacy of a candidate treatment.

Traditional PK/PD modeling in drug development defines parameters such as drug dose concentration, drug exposure effects, drug half-life, drug concentrations against time, and drug effects against time. When used more broadly, quantitative techniques such as drug modeling, disease modeling, trial modeling, and market modeling can support the entire development process, which results in better decisions through explicit consideration of risk and better utilization of knowledge. A variety of PK/PD modeling tools are available to drug development researchers, for example, WinNonlin and the Knowledgebase Server (PKS) developed by Pharsight, Inc. Mountain View, California.

The foregoing written specification and following examples are considered to be sufficient to enable one skilled in the art to practice the invention. Various modifications of the invention in addition to those shown and described herein will become apparent to those skilled in the art from the foregoing description and following examples and fall within the scope of the appended claims.

It is understood that the application of the teachings of the present invention to a specific problem or situation will be within the capabilities of one having ordinary skill in the art in light of the teachings contained herein.

Further details of the invention are illustrated by the following non-limiting Examples. The disclosures of all citations in the specification are expressly incorporated herein by reference.

### EXAMPLES

### Example 1

### A PHASE 111, RANDOMIZED, DOUBLE-BLIND, PLACEBO-CONTROLLED, MULTICENTER STUDY TO EVALUATE THE EFFICACY AND SAFETY OF ETROLIZUMAB AS AN INDUCTION AND MAINTENANCE TREATMENT FOR PATIENTS WITH MODERATELY TO SEVERELY ACTIVE CROHN'S DISEASE

### Description of the Study

### Study Rationale

The purpose of this study is to assess the efficacy and safety of etrolizumab, an anti-integrin with a unique mechanism of action (MOA) that has been shown to inhibit the trafficking and retention of inflammatory T-cells in the intestinal mucosa, via a disruption of α4β7/MAdCAM-1, and αEβ7/E-cadherin binding.

Although etrolizumab has not been studied in humans with CD, preliminary expression studies of the pharmacological target for etrolizumab, the integrin β7 receptor, on gut CD4+ and CD8+ T cells isolated from resections of patients with UC and patients with CD, suggests that expression levels are similar between both diseases. The reported efficacy of vedolizumab, an anti-α4β7 mAb, in CD demonstrates a role for α4β7 in the pathobiology of this disease (Sandborn WJ, et al., Aliment Pharmacol Ther 37:204-13, 2013) and it follows from such studies that etrolizumab will be efficacious in CD. In addition, because αEβ7+expression is reportedly elevated in patients with CD (Elewaut D, et al., Acta Gastroenterol Belg 61:288-94, 1998; Oshitani N, et al., Int J Mol Med 12:715-9, 2003) with an observed increase in expression from distal to proximal bowel, the dual MOA of etrolizumab may bring enhanced efficacy in CD without generalized immunosuppression, compared with available anti-integrin and anti-TNF therapies.

In addition, in a global Phase II study in UC patients, etrolizumab was efficacious in treating moderate to severe UC and achieved a placebo-corrected clinical remission rate of 20.5% (p = 0.058) and an endoscopic remission rate of 10.3% (p = 0.004) at 10 weeks after treatment initiation (105 mg (100 mg nominal dose) every 4 weeks [Q4W]) in an all comers population (Vermeire S, et al., Gastroenterology 144,S1:S-36, 2013; Vermeire S, et al., Lancet 384: 309-18, 2014; Lin et al., Gastroenterology 146:307-315, 2014). In addition, etrolizumab had an acceptable safety profile with no clinically significant safety signals observed.

### Study Design

This will be a multicenter, Phase III, double-blind, placebo-controlled study evaluating the efficacy, safety, and tolerability of etrolizumab compared with placebo during induction and maintenance treatment of moderate to severely active CD.

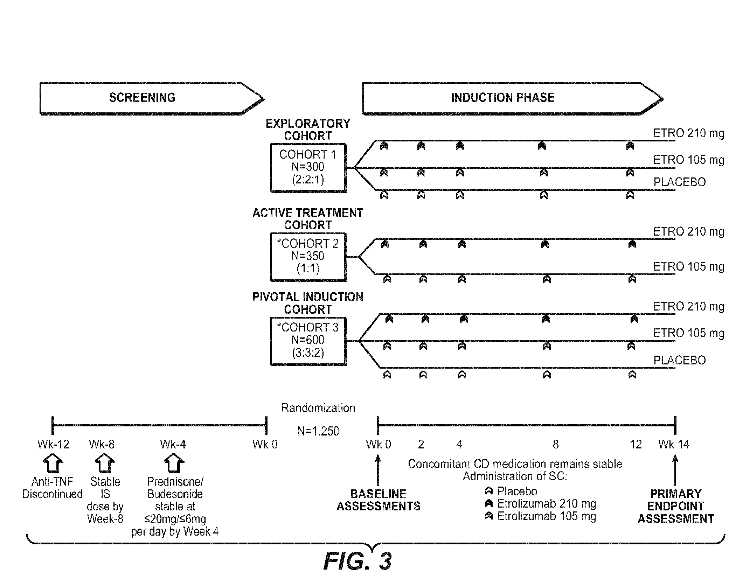

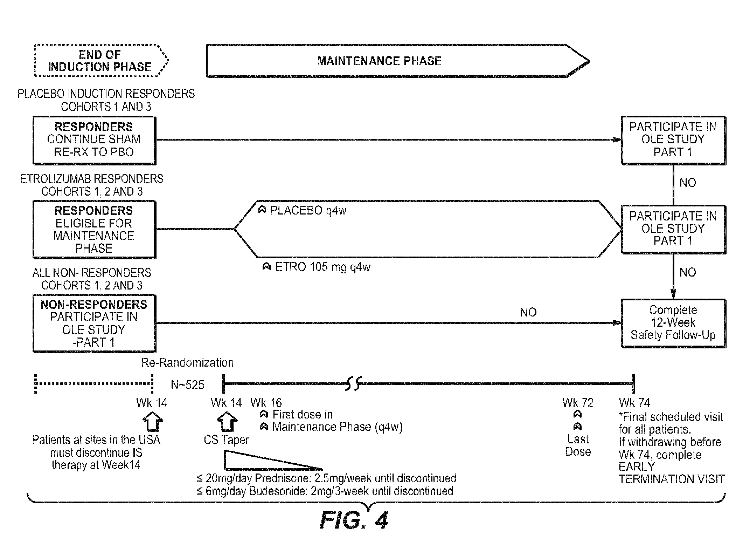

The study design will comprise 1) a Screening Phase (up to 28 days) to determine patients' eligibility for the study, 2) an Induction Phase (14 weeks), followed by 3) a Maintenance Phase (60 weeks) in patients demonstrating a CDAI-70 response (defined as a decrease from CDAI baseline score of at least 70 points) at the end of the Induction Phase, and 4) a Safety Follow-Up Phase (12 weeks) after administration of the last dose of study drug in the Maintenance Phase for those patients who are not participating in Part 1 of open-label extension of the Study to receive etrolizumab treatment (see Fig. 3 and Fig. 4). At the completion of the Safety Follow-Up Phase, patients will be asked to enter an extended PML-monitoring phase (open-label extension study) for 92 weeks. An independent Data Monitoring Committee (iDMC) will monitor safety and study conduct on an ongoing basis.

Moderate to severely active CD will be defined in the Screening Phase by clinical signs and symptoms that result in a CDAI score between ≥220 and ≤480, as well as by a PRO2 score ≥ 14, calculated in the 7 days prior to randomization. In conjunction, the presence of active inflammation, defined as a SES-CD score of ≥ 7, or ≥ 4 in cases of isolated ileitis or post-ileocecal resection, is required and will be determined by a screening ileocolonoscopy scored by central reading.

The study population will consist of patients who have not received prior anti-TNF therapy (TNF-naive) but are refractory or intolerant to corticosteroids (CS) and/or immunosuppressants (IS) therapy, as well as patients who are refractory or intolerant to anti-TNF therapy (as further described below). Eligible patients must be on stable IS for at least 8 weeks prior to randomization and on a stable dose of CS as described below. Patients who are non-responsive or refractory to anti-TNF therapy (TNF-IR) or intolerant to anti-TNF therapy must have discontinued this treatment for a period of at least 12 weeks prior to their randomization.

Approximately 1250 patients will be randomized into the study from approximately 380 global investigational sites via enrollment into one of three cohorts. The enrollment will be sequential, first into Cohort 1, then Cohort 2, and lastly Cohort 3.

### Screening Phase

Patients will be evaluated for eligibility in the 28-day Screening Phase (see Fig. 3 and Fig. 4). Major eligibility criteria are noted below.

During the Screening Phase, patients taking CS therapy must have been on a stable dose of ≤20 mg/day prednisone (or equivalent) or ≤6 mg/day oral budesonide for at least 2 weeks immediately prior to their randomization. Similarly, eligible patients requiring background IS therapy (e.g., 6-MP, or MTX) must be receiving a stable IS dose regimen for at least 8 weeks immediately prior to their randomization. Patients who are TNF-IR or intolerant to anti-TNF therapy must have discontinued this treatment for a period of at least 12 weeks prior to their randomization.

The ileocolonoscopy should be performed between Week -3 and Week -2 of the Screening Phase to allow sufficient time for a central reader scoring and determination of eligibility, and to avoid the ileocolonoscopy bowel preparation influencing the patient reported outcomes used in the determination of baseline PRO2 and CDAI scores (i.e., abdominal pain, general well-being, and stool frequency).

### Induction Phase

Eligible patients will be enrolled sequentially into one of three cohorts for the 14-week Induction Phase (see Fig. 3).

Patients enrolled in Cohort 1 (double-blind, placebo-controlled, exploratory cohort; n=300) will be randomized in a 1:2:2 ratio to receive placebo, etrolizumab 105 mg SC Q4W (low dose), or etrolizumab 210 mg SC (high dose) at Weeks 0, 2, 4, 8, and 12 within a 14-week Induction Phase (note that patients randomized to low-dose etrolizumab will receive a placebo injection at Week 2- see below). Patients enrolled in Cohort 2 (etrolizumab dose-blind, active-treatment cohort; n=350) will be randomized in a 1:1 ratio to receive low-dose or high-dose regimens of etrolizumab. Patients enrolled in Cohort 3 (double-blind, placebo-controlled, pivotal cohort; n=600) will be randomized in a 2:3:3 ratio to receive placebo or etrolizumab low-dose or high-dose. Because the low dose and high dose of etrolizumab are in syringes of different volumes, in order to preserve the blind, patients in all three cohorts will receive two injections at Weeks 0, 4, 8, and 12. Patients randomized to low-dose etrolizumab will receive one placebo (matching high-dose pre-filled syringe) and one low-dose etrolizumab injection at each administration except at Week 2, when they will receive one placebo injection. Patients randomized to high-dose etrolizumab will receive one placebo and one high-dose etrolizumab injection at each administration, except at Week 2 where they will receive one high-dose injection. Finally, patients randomized to placebo will receive two placebo injections at every administration, except at Week 2 where they will receive one placebo injection.

The randomization in all cohorts will be stratified by concomitant oral CS treatment (yes vs. no), concomitant IS treatment (yes vs. no), baseline CDAI <330 (yes vs. no), and TNF-IR patient (yes vs. no). The enrollment will be managed to ensure that the proportion of the TNF-IR patients in Cohort 3 does not exceed approximately 75% and that the proportion of patients with a CDAI score between >450 and ≤480 does not exceed approximately 10% in each cohort.

During the Induction Phase, patients in all cohorts must keep their dose(s) of CS and IS therapy stable (if requiring CS/IS at baseline). Adjustments to these medications will be considered rescue therapy. Rescue therapy means medication prescribed for new or worsening CD symptoms and includes any new medication for CD or any increase in dose or regimen of baseline CD medications. Also, every attempt should be made to keep anti-diarrheal medication at a fixed dose, if required. The impact of titrating anti-diarrheal medication on the placebo response rate for PRO2 has not been studied in a moderate to severely active CD population.

At Week 10 there is an optional escape to an Open Label Extension (OLE) study (Part 1) where patients can receive open label etrolizumab. This can only be exercised if a patient experiences disease worsening, defined as both CDAI and PRO2 Week 10 scores being greater than the patient's baseline (Week 0) score. At Week 10, patients who experience disease progression and who have not entered the OLE may use rescue therapy.

At week 14, patients achieving CDAI-70 response without the use of rescue therapy will continue to the Maintenance Phase. Patients participating at U.S. investigational sites must discontinue IS treatment at Week 14. Patients who do not achieve CDAI-70 response at Week 14 and/or who use rescue therapy during the Induction Phase will be considered non-responders and will be ineligible for the Maintenance Phase. Non-responders at Week 14 will have the option of entering an Open Label Extension (OLE) study (Part 1) unless they used rescue medication in the absence of disease worsening (as defined above). Non-responders who do not enter the OLE, will enter a 12-week Safety Follow-Up Phase and then be asked to enroll in an extended PML-monitoring phase (OLE Study, Part 2) for 92 weeks.

### Maintenance Phase

At the end of the Induction Phase (Week 14), patients will be assessed for a CDAI score and will undergo a full endoscopy (ileocolonscopy) with central reading to determine a SES-CD score. Every attempt must be made to schedule the ileocolonoscopy to take place at the Week 14 visit or, no later than 5 calendar days after this visit; the procedure must not be scheduled before Week 14. The patient reported outcomes (i.e., abdominal pain, general well-being, and stool frequency) that are captured in the 7 days prior to the bowel preparation will be used to calculate the Week 14 PRO2 and CDAI scores, thus removing any influence of the bowel preparation on these outcomes.

Patients who received placebo during the Induction Phase and achieved a CDAI-70 response at Week 14 without the use of rescue therapy will undergo a sham randomization to blinded placebo treatment during the Maintenance Phase. Patients who received etrolizumab and achieved a CDAI-70 response at Week 14 without the use of rescue therapy, will be randomized into the Maintenance Phase in a 1:1 ratio to treatment with placebo or etrolizumab 105 mg SC Q4W (see Fig. 4).

The randomization for the Maintenance Phase occurs at the Week 16 clinic visit, when the first maintenance dose will be administered. The randomization call may take place between Weeks 14 (last visit in the Induction Phase) and Week 16, provided the patient has been assessed as eligible for the Maintenance Phase. The randomization will be stratified by CDAI remission at both Weeks 10 and 14 (yes vs. no), induction dose regimen (low dose vs. high dose), concomitant oral CS treatment (yes vs. no), and prior TNF-IR patient (yes vs. no).

During the Maintenance Phase, patients in the United States must not take any concomitant IS therapy during the Maintenance Phase, and patients outside the United States must remain on a stable dose of IS therapy throughout the study, unless dose reduction or discontinuation is required because of a toxicity related to the medication. CS dose will be tapered starting at Week 14. The CS dose will be tapered according to the following schedule: ≤ 20 mg/day prednisone (or equivalent); titrated via dose reduction of 2.5 mg/week until discontinuation; and ≤ 6 mg/day oral budesonide: titrated via dose reduction of 3 mg every 2 weeks until discontinuation. Patients who cannot tolerate the CS taper without recurrence of CD symptoms or symptoms of steroid withdrawal, can receive an increase in CS dose but this must not exceed the dose administered at randomization. The dose-tapering regimen must be re-initiated within 2 weeks.

Patients who experience a clinical relapse, defined as ≥ 100-point increase in CDAI score from the Week 14 CDAI score on 2 consecutive visits (which may include unscheduled visits) and a CDAI score ≥220 points, will have the option of escaping to the OLE study during the Maintenance Phase.

Patients who complete their final Maintenance Phase visit at Week 74 can enroll in the OLE study (Part 2) if eligible, or enter a 12-week Safety Follow-Up Phase, after which they will be asked to enroll in an extended PML-monitoring phase (OLE Study, Part 2) for 92 weeks. Patients who require surgical intervention for CD will stop study treatment, enter the Safety Follow-Up Phase, and will be asked to enter the OLE Study Part 2 for PML monitoring. Patients who self-withdraw from the trial or who do not meet the eligibility criteria for OLE treatment will also enter the Safety Follow-Up Phase and be asked to enter the OLE Study Part 2 for PML monitoring.

### Outcome Measures

Separate outcome measures will be evaluated for the United States Food and Drug Administration (FDA) and for the European Medicines Agency (EMA), and potentially other health authorities outside the United States (ex-U.S.) as described further below.

### Primary Efficacy Outcome Measure (U.S.)

The primary efficacy outcome measure for the induction phase is PRO2 remission at Week 14. PRO2 remission is defined as PRO2 ≤ 11.

The primary efficacy outcome measure for the maintenance phase is PRO2 remission at Week 66 among patients who achieved PRO2 remission at Week 14. PRO2 remission is defined as PRO2 ≤ 11.

### Primary Efficacy Outcome Measure (ex-U.S.)

The primary efficacy outcome measure for the induction phase is CDAI remission at Week 14. CDAI remission is defined as CDAI < 150.

The primary efficacy outcome measure for the maintenance phase is maintenance of CDAI remission after at least 52-weeks, and corticosteroid-free throughout, among patients who achieved CDAI remission at weeks 10 and 14.

### Secondary Efficacy Outcome Measures

The U.S. primary efficacy outcome measures for induction and maintenance phases described above are secondary efficacy outcome measures for induction and maintenance, respectively, ex-U.S. The ex-U.S. primary efficacy outcome measures for induction and maintenance phases described above are secondary efficacy outcome measures for induction and maintenance, respectively, U.S.

Additional global secondary efficacy outcome measures for the induction phase of this study are (1) Endoscopic improvement at Week 14 where endoscopic improvement is defined as a 50% reduction in the baseline SES-CD score; (2) CDAI-100 response at Week 14 where CDAI-100 response is defined as a decrease from CDAI baseline score of at least 100 points; (3) CDAI remission at Week 10 and Week 14 where CDAI remission is defined as CDAI score < 150; (4) change from baseline to Week 14 in patient-reported HRQOL as assessed by the IBDQ (HRQOL = health-related quality of life; IBDQ = inflammatory bowel disease questionnaire); and (5) change from baseline to week 14 in CD signs and symptoms as assessed by the CD-PRO/SS measure (CD-PRO/SS = Crohn's Disease-Patient-Reported Outcome Signs and Symptoms).

Additional global secondary efficacy outcome measures for the maintenance phase of this study are (1) endoscopic improvement at Week 66 (compared to Week 0) where endoscopic improvement is defined as a 50% reduction in the baseline SES-CD score; (2) CDAI remission at Week 66, among patients who achieved CDA remission at Week 14 where CDAI remission is defined as CDAI score < 150; (3) CDAI remission at Week 66, among patients who achieved a clinical response at Week 14 where CDAI remission is defined as CDAI score < 150; (4) CDAI-100 response at Week 66 (compared to Week 0) where CDAI-100 response is defined as a decrease from CDAI baseline score of at least 100 points; (5) durable CDAI remission (achieved at ≥ 6 of the following Weeks: 24, 28, 32, 44 56, 66, 70 and 74) where durable CDAI remission is defined as a CDAI score <150 on at least 6 out of the 8 CDAI assessment visits during the maintenance phase; (6) CS-free CDAI remission at Week 66 among patients who achieved clinical response at Week 14 where CS-free CDAI remission means CDAI remission (CDAI score < 150) with no corticosteroid use; (7) CDAI remission (CDAI score < 150) at Week 66 and CS-free for 24 weeks prior to Week 66, in patients who achieved CDAI remission at Weeks 10 and 14; (8) maintenance of endoscopic improvement at Week 66 among patients who achieved endoscopic improvement and CDAI-70 response at Week 14 where the outcome measures are defined as above; (9) resolution of mucosal inflammation (SES-CD score 0) at Week 66; (10) change in patient-reported HRQOL from Week 14 to Week 66 as assessed by the IBDQ; and (11) change in CD signs and symptoms from Week 14 to Week 66 as assessed by the CD-PRO/SS measure

### Exploratory Outcome Measures

The global exploratory outcome measures for this study are (1) change in fecal calprotectin levels from Week 0 to 14; (2) change in fecal calprotectin levels from Week 14 to 66, among patients who achieved CDAI-70 response at Week 14; (3) change in CRP levels from Week 0 to 14; (4) change in CRP levels from Week 14 to 66, among patients who achieved CDAI-70 response at Week 14; and (5) ime from Week 14 to major CD-related events (including hospitalizations, bowel surgeries, and non-study procedures), among patients who achieved CDAI-70 response at Week 14.

### Safety Outcome Measures

The safety outcome measures for this study are: incidence and severity of adverse events, incidence of serious adverse events, incidence and severity of infection-related adverse events, incidence of infection-related serious adverse events, incidence and severity of injection-site reactions, incidence and severity of hypersensitivity reactions, incidence of adverse events leading to study drug discontinuation, incidence of specific laboratory abnormalities, incidence of malignancies, and incidence of anti-therapeutic antibodies (ATAs) to etrolizumab.

### Pharmacokinetic Outcome Measures

The pharmacokinetic outcome measures for this study are: etrolizumab serum concentrations at Weeks 14 and 66 and observed predose trough serum concentration Cₘᵢₙ at specified timepoints during the dosing period from Week 16 to Week 66.

### Study Population; Patients

The target population is patients who have moderate to severely active CD (as defined below) and have had an inadequate response, refractory response or intolerance to corticosteroids and/or immunosuppressant therapy and/or anti-TNFs. Moderate to severely active CD means moderate to severely active disease determined in the Screening Phase by meeting each of the following three signs and symptoms measures:
1. Clinical signs and symptoms resulting in a CDAI score of ≥220 to ≤ 480 calculated in the 7 days prior to randomization; and
2. A PRO2 score ≥ 14 calculated in the 7 days prior to randomization; and
3. The presence of active inflammation defined as a SES-CD score of ≥ 7 or ≥ 4 in cases of isolated ileitis or post-ileocecal resection as determined by a screening ileocolonoscopy scored by the central reader.

### Inclusion Criteria

Patients must meet the following criteria for study entry: (1) Able and willing to provide written informed consent; (2) 18-80 years of age; (3) Males and females who are not post-menopausal: use of effective contraception during the treatment period and for at least 24 weeks after the last dose of study drug; (4) Diagnosis of CD based on clinical and endoscopic evidence established ≥ 3 months prior to screening visit; (5) Moderate to severely active disease, as defined above, determined in the Screening Phase; (6) involvement of ileum and/or colon with at least four colonic segments traversable by a pediatric endoscope or three segments (colon and/or ileum) for patients who have undergone a bowel resection for CD; (7) completed a surveillance colonoscopy ≤ 12 months prior to screening if colonic disease of > 10 years' duration or ≤ 5 years prior to screening if patient has any risk factors for bowel cancer (surveillance may be performed during screening); (8) have experienced intolerance, refractory disease, or no response (as defined below) to at least one of the following therapies within 5 years from screening:
CS therapy: CS refractory means the patient has signs/symptoms of persistently active disease despite a history of at least one 4-week induction regimen including a dose equivalent to ≥30 mg/day prednisone (or equivalent) for 2 weeks if oral or 1 week if IV. Intolerance to CS therapy means the patient has a history including but not limited to Cushing's syndrome, osteopenia/osteoporosis, hyperglycemia, insomnia, and infection.
IS therapy: IS refractory means the patient has signs/symptoms of persistently active disease despite a history of at least one 12-week regimen of oral AZA (≥ 1.5 mg/kg) or 6-MP (≥ 0.75 mg/kg) or MTX (≥ 15 mg/week). Intolerance to IS therapy (6-MP, AZA, or MTX) means the patient has a history of intolerance to ≥ 1 of the above (including but not limited to infection, nausea/vomiting, abdominal pain, pancreatitis, liver function test abnormalities, lymphopenia, and thiopurine methyltransferase genetic polymorphism).
Anti-TNF therapy: Inadequate primary non-response means the patient did not respond (as evidenced by persistent signs/symptoms related to CD after receiving ≥ 2 induction doses of either infliximab [≥ 5 mg/kg] or adalimumab [160 mg/80 mg or 80 mg/40 mg] or certolizumab pegol [≥ 400 mg]). Inadequate secondary non-response means the patient initially responded to induction therapy with infliximab (≥ 5 mg/kg) or adalimumab (≥ 40 mg) or certolizumab pegol (≥ 400 mg) but experienced signs/symptoms related to recurrence of CD during maintenance. Intolerance means the patient experienced a significant injection-site reaction, demyelination, congestive heart failure, infection, or other condition that precluded continuing use of anti-TNF therapy at any time.

### Exclusion Criteria

Patients meeting any of the following criteria will be excluded from study entry. The exclusion criteria related to gastrointestinal health include: (1) underwent subtotal colectomy with ileorectal anastomosis or underwent total colectomy; (2) short-bowel syndrome; (3) has an ileostomy or colostomy; (4) has fistulizing disease and/or evidence of fixed stenosis or small-bowel stenosis with prestenotic dilation that precludes adequate endoscopic assessment of the bowel; (5) diagnosis of UC or indeterminate colitis; (6) suspicion of ischaemic colitis, radiation colitis, or microscopic colitis; (7) evidence of abdominal or perianal abscess; (8) expected to require surgery to manage CD-related complications during the study; (9) past or present adenomatous colonic polyps; (10) past or present disease-related colonic mucosal dysplasia (prior age-related polyps are acceptable).

The exclusion criteria related to prior or concomitant therapy include: (1) received any of adalimumab, certolizumab pegol, or infliximab for CD within ≤ 12 weeks prior to randomization; (2) any prior treatment with etrolizumab or other anti-integrin agents (including vedolizumab, natalizumab, and efalizumab); (3) prior treatment with T cell- or B cell-depleting agents (e.g., rituximab, alemtuzumab, or visilizumab) within ≤ 12 months prior to randomization; (4) received any investigational treatment that included investigational vaccines within 12 weeks prior to randomization in the study or five half-lives of the investigational product, whichever is greater; (5) history of moderate or severe allergic or anaphylactic/anaphylactoid reactions to chimeric, human, or humanized antibodies, fusion proteins, or murine proteins or hypersensitivity to etrolizumab (active drug substance) or any of the excipients; (6) treatment with corticosteroid enemas/suppositories and/or topical (rectal) 5-aminiosalicylate (5-ASA) preparations ≤2 weeks prior to randomization; (7) patients who have not discontinued tube feeding, defined formula diets, and/or parenteral alimentation/nutrition as treatment for CD ≥ 3 weeks prior to randomization; (8) patients who are expected to require tube feeding, defined formula diets, and/or parenteral alimentation/nutrition as treatment for CD during the study (9) received any live or attenuated vaccines ≤4 weeks prior to randomization; (10) use of IV steroids during screening, with the exception of a single IV steroid dose administered in the Emergency Department; (11) cyclosporine, tacrolimus, sirolimus, or mycophenolate mofetil ≤4 weeks prior to randomization; (12) chronic use of nonsteroidal anti-inflammatory drugs (NSAIDs). Prophylactic aspirin use up to 325 mg/day is permitted, as is occasional use of NSAIDs for conditions such as headache, arthritis, mylagia, and menstrual cramps; (13) if receiving oral CSs, patients will be excluded unless the dose is stable at </= 20mg/day prednisone (or equivalent) for >/= 2 weeks immediately prior to randomization; (14) if receiving ongoing treatment with oral or rectal 5 aminosalicylate (5-ASA), patients will be excluded if the dose is not stable for >/= 4 weeks immediately prior to randomization; (15) if receiving ongoing treatment with ISs, patients will be excluded if the dose is not stable for >/= 8 weeks immediately prior to randomization; (16) if receiving ongoing treatment with antibiotics for the treatment of CD, patients will be excluded if the dose is not stable for >/= 2 weeks immediately prior to randomization.

The exclusion criteria related to infection risk include: (1) congenital or acquired immune deficiency; (2) positive ELISA test result for HIV confirmed by Western blot; (3) positive hepatitis C virus (HCV) antibody test result, unless the patient has documented that HCV RNA is undetectable for > 6 months after completing a successful course of HCV antiviral treatment; (4) in the screening hepatitis B assessment, patients who test positive for HBsAg are excluded from the study; patients who test positive for hepatitis B core antibody (HBcAb) but negative for hepatitis B surface antigen (HBsAg) must have a confirmed negative hepatitis B virus (HBV) DNA test result to be eligible for the study and will be required to undergo periodic monitoring for HBV DNA during the study; (5) positive stool test result for ova or parasites or positive stool culture for pathogens at time of screening; (6) evidence of infection with and/or treatment for Clostridium difficile or other intestinal pathogen treatment for C. difficile infection within 8 weeks prior to the baseline visit; (7) a history of active or latent tuberculosis or suspicion of active tuberculosis on chest radiograph taken within 3 months of randomization; (8) history of recurrent opportunistic infections and/or history of severe or disseminated viral infections; (9) any serious opportunistic infections that occurred </= 6 months prior to screening; (10) any current or recent signs or symptoms (</= 8 weeks before screening) of infection; (11) any major episode of infection requiring hospitalization or treatment with intravenous antibiotics </= 8 weeks prior to screening or oral antibiotics </= 4 weeks prior to screening.

The exclusion criteria related to general safety include: (1) pregnant or lactating; (2) lack of peripheral venous access; (3) hospitalized within 8 weeks prior to randomization; (4) inability to comply with study protocol, in the opinion of the investigator; (5) significant uncontrolled comorbidity, such as neurological, cardiac, pulmonary, renal, hepatic, endocrine, or GI disorders (other than CD); (6) neurological conditions or diseases that may interfere with monitoring for PML (7) clinically significant abnormalities on screening neurologic examination; (8) history of demyelinating disease; (9) history of major neurological disorders, including stroke, MS, brain tumor, neurodegenerative disease, or poorly controlled epilepsy; (10) history of alcohol, drug, or chemical abuse ≤6 months prior to screening ; (11) conditions other than CD that could require treatment with >20 mg/day of prednisone (or equivalent) during the course of the study; (12) history of cancer, including hematologic malignancy, solid tumors, and carcinoma in situ within 5 years before screening; (13) female patients who have not have had a cervical smear within the previous year or at screening; (14) history of organ transplant, or cell transplantation; (15) patients for whom a magnetic resonance imaging (MRI) scan is considered unsafe, due to the presence of metal in the body that could a pose hazard during any potential scanning.

### Materials and Methods

### Study Treatment

Etrolizumab will be supplied as a single-use PFS containing 150 mg/mL etrolizumab for subcutaneous (SC) administration. Depending on the dose assignment in the Induction Phase, patients receive either study drug in a 1-mL PFS containing 0.7 mL of etrolizumab (105-mg dose) or a 2.25-mL PFS containing 1.4 mL of etrolizumab (210-mg dose) according to the treatment schedule. To preserve the blind to study drug assignment in the Induction Phase, at Weeks 0, 4, 8, and 12 all patients receive two injections: one 0.7-mL dose and a second 1.4-mL dose, and either one (if in one of the study drug arms) or both (if in placebo arm) will contain placebo. At Week 2, all patients will receive one 1.4-mL dose injection, which will contain placebo for patients in the low-dose etrolizumab and placebo arms and study drug for the high-dose etrolizumab arm. In the Maintenance Phase, patients receive a single 0.7-mL dose (105-mg dose) that will either contain etrolizumab or placebo, according to the treatment schedule.

For the placebo, the composition is exactly the same as that of active drug product without the presence of etrolizumab.

### Crohn's Disease Actvity Assessments

For each patient, a detailed history of CD, including date of diagnosis, disease severity, hospitalizations, and extraintestinal manifestations at screening will be collected. Disease severity will be evaluated using the CDAI, PRO2, and SES-CD as described above. Additional details for the CDAI are provided in Table 1 below and additional details for the SES-CD are provided below in Table 2. Additional details for the PRO2 are as follows.

As described above and in Khanna et al., Aliment Pharmacol Ther 41:77-86, 2015, the PRO2 evaluates two patient-reported factors, the frequency of liquid or soft stools and abdominal pain. The PRO2 score is calculated by taking the average frequency and pain scores measured in a 7-day period, multiplying each average score by a specified weighting (the same weighting used for the CDAI score [see Table 1 below]) and adding the weighted averages together). Khanna et al., *Id.,* identified cut-points for each component of the score that resulted in the most sensitive and specific correlation with clinical remission, benchmarked as CDAI score < 150. The optimal cut-points defined by Khanna et al. are mean daily stool frequency ≤ 1.5, abdominal pain ≤ 1. Based on the results published in Gasink et al., [abstract] ACG Annual Meeting 2014, we have defined a cut-point of mean daily stool frequency that differs from Khanna et al., *Id.,* the cut-point being < 3 representing clinical remission of liquid/soft stoods in a moderate-severe CD population (compared to ≤ 1.5 defined in Khanna et al., *Id*.). The cut-point for abdominal pain is ≤ 1. Accordingly, remission defined by PRO2 score is ≤ 11 (stool frequency of 3 × CDAI multiplication factor of 2 = 6 + [abdominal pain of ≤ 1 × CDAI multiplication factor of 5] ≤ 5; combining the two scores results in ≤ 11). We believe this study is the first use in a prospective study of this particular PRO2 score based on these cut-points to define remission.

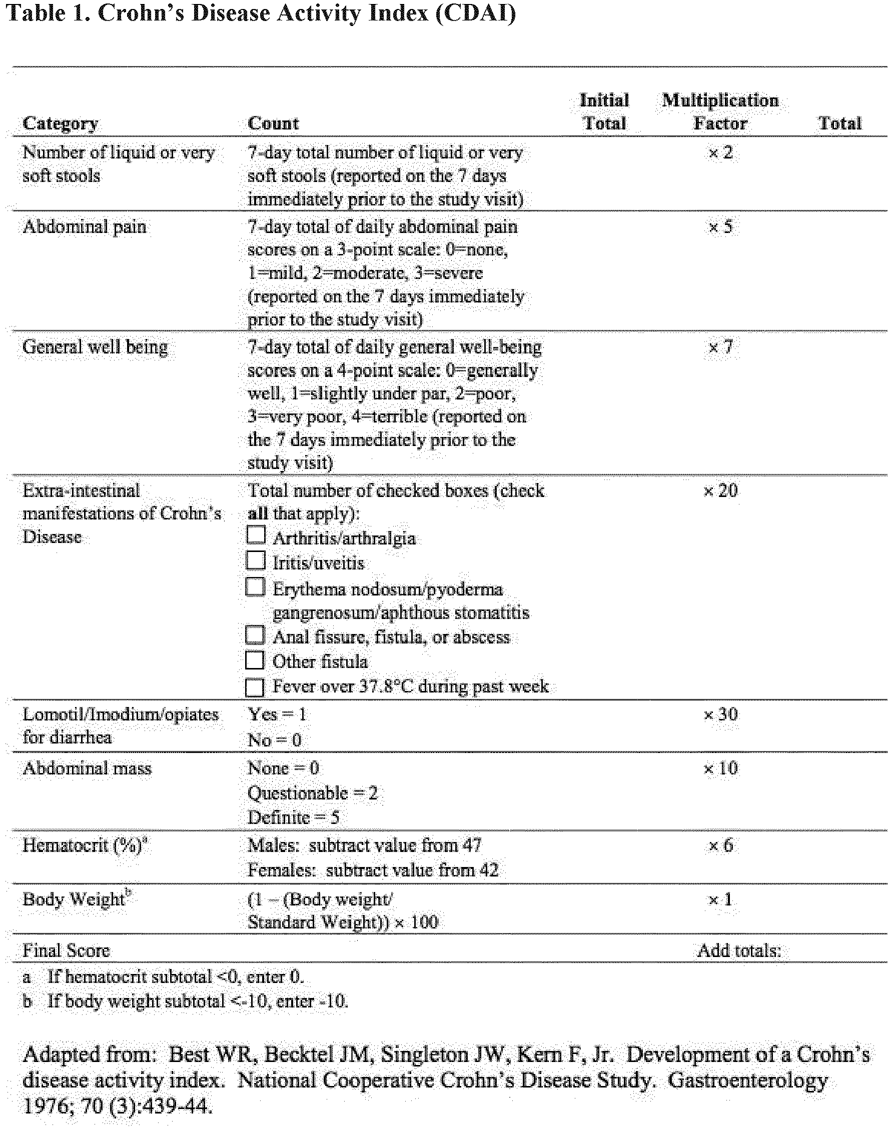

**Table 2. Definitions of SES-CD (Daperno et al., Gastrointest. Endosco. 60:505, 2004)**

| | | | | |
|---|---|---|---|---|
| **Simple Endoscopic Score for Crohn's Disease values** | | | | |

| **Variable** | **0** | **1** | **2** | **3** |
|---|---|---|---|---|
| Size of ulcers | None | Aphthous | Large ulcers | Very large |
| | | ulcers (Ø 0.1 to | (Ø 0.5 to 2 cm) | ulcers |
| | | 0.5 cm) | | (Ø > 2 cm) |
| Ulcerated | None | <10% | 10-30% | >30% |
| surface | | | | |
| Affected | Unaffected | <50% | 50-75% | >75% |
| surface | segment | | | |
| Presence of | None | Single, can be | Multiple, can | Cannot be |
| narrowings | | passed | be passed | passed |

| | | | | |
|---|---|---|---|---|
| Ø, Diameter. | | | | |

### Rationale for the Primary and Key Secondaru Study Endpoints

The study is designed to generate data for separate EMA and FDA primary endpoints in response to the agencies' separate recommendations for appropriate therapeutic primary outcome measures in CD (GREAT2 Workshop 2013 October 21^{st}-22^{nd} 2013. great2.org. Available from: http://www.fda.gov/drugs/newsevents/ucm362766.htm). This approach will allow etrolizumab to be developed for global use in the treatment of CD. Clinical remission defined as a CDAI score ≤ 150 will be used as the primary endpoint analysis for the EMA and rest of world (ex-U.S.). Signs and symptoms remission defined as a PRO2 score ≤ 11 will be used as the primary endpoint for the FDA (U.S.).

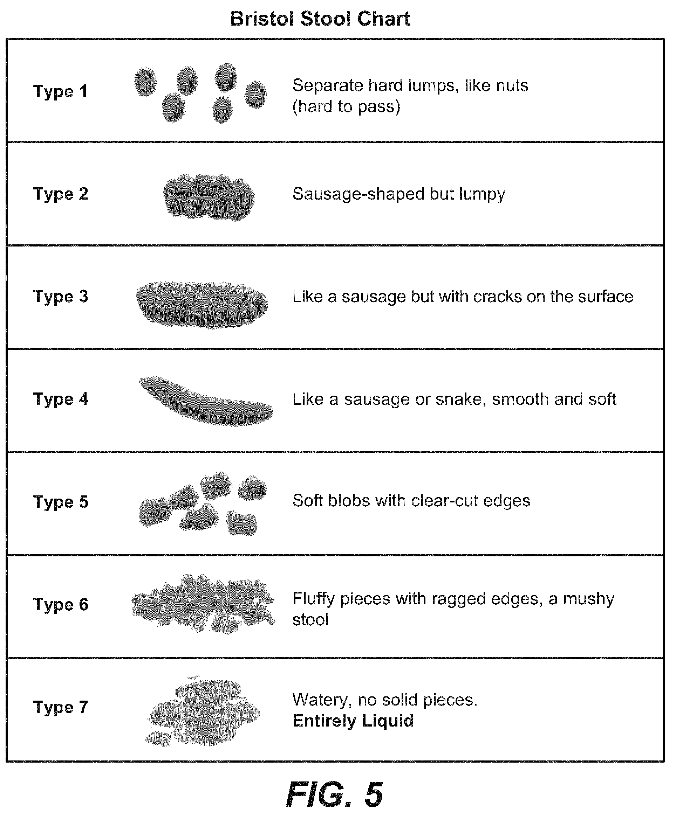

The CDAI was developed using a multivariable regression analysis to generate an equation that best predicted a physician's overall CD severity rating for a patient (Best WR, et al., Gastroenterology 77:843-6, 1979). Validation of the instrument (in terms of construct and content validity) was conducted prospectively during multiple clinical trials over a 25-year period, where the CDAI performed reproducibly and was responsive to change. The therapeutic efficacy of all licensed biologic therapies indicated for the treatment of moderate to severe CD has been consistently evaluated on the basis of a CDAI score of < 150 to define remission. However, concerns have been raised about poor criterion validity for the CDAI, a reported lack of correlation between the CDAI and endoscopic measures of inflammation (which may render the CDAI as a poor discriminator of active CD and irritable bowel syndrome) and high reported placebo rates (Korzenik et al., N Engl J Med. 352:2193-201, 2005; Sandborn WJ, et al., N Engl J Med 353:1912-25, 2005; Sandborn WJ, et al., Ann Intern 19;146:829-38, 2007, Epub 2007 Apr 30; Kim et al., Gastroenterology 146: (5 supplement 1) S-368, 2014). To address these concerns, the current study design enriches for patients with active inflammation by endoscopic assessment. In line with an FDA recommendation, the CDAI will be used in conjunction with the Bristol Stool Scale (Lewis et al., Scandinavian Journal of Gastroenterology Vol 32 (9): 920-924, 1997) as a medical aid for patients to consistently capture stool form (Fig. 5). This was reported to be a specific area lacking agreement in a survey conducted to assess methodological variation in administration of the CDAI (Sands et al., Inflammatory Bowel Disease 11:133-8, 2005).

In line with the EMA guideline on the development of new medicinal products for the treatment of CD, the primary study objective for the ex-U. S analysis is to evaluate the efficacy of etrolizumab compared with placebo in maintaining CS-free CDAI remission for 1 year (52 weeks) among patients who were in remission at the start of the Maintenance Phase. Patients included in this analysis will have been confirmed as in remission based on a CDAI score < 150 at both Weeks 10 and 14 in the Induction Phase. An outcome of maintenance of CDAI remission after at least 52 weeks, and CS-free throughout, has not been studied previously but is a clinically meaningful measure of treatment success given that long-term CS use is associated with serious side effects, such as weight gain, cataracts, hyperglycemia, osteoporosis, and increased risk of infection. A subgroup analysis will assess this outcome in the group of patients who required CS at baseline. Maintenance of CDAI remission for 52 weeks and CS-free for 24 weeks prior will be assessed as secondary outcome.

The PRO2 is a PRO measure that evaluates the frequency of loose/liquid stools and abdominal pain (Khanna et al., Aliment Pharmacol. Ther. 41:77-86, 2015). These items are derived and weighted accordingly from the CDAI and are the CDAI diary card items, along with general well-being, that contribute most to the observed clinical benefit measured by CDAI (Sandler et al., J. Clin. Epidemiol 41:451-8, 1988; Thia et al., Inflamm Bowel Dis 17:105-11, 2011; Kim et al., Gastroenterology 146: (5 supplement 1) S-368, 2014). The remission score of ≤ 11 is the CDAI-weighted sum of the average stool frequency and pain scores in a 7-day period, which yielded optimum sensitivity and specificity for identification of CDAI remission (score of < 150) in a retrospective data analysis of ustekinumab induction treatment for moderate to severe CD in the Phase II CERTIFI study (Gasink et al., [abstract] ACG Annual Meeting 2014). The PRO2 was shown to be sensitive and responsive when used as a continuous outcome measure in a retrospective data analysis of MTX treatment in active CD (Khanna et al., Aliment Pharmacol. Ther. 41:77-86, 2015) measured by CDAI; estimates of treatment effect were not improved when general well-being was incorporated in the score.

The use of PRO2 uses 7-day scores of loose/liquid stool frequency and abdominal pain that correlate with CDAI remission. In addition, as a composite score, PRO2 is able to measure clinical remission across the spectrum of proximal to distal disease presentation, where loose/liquid stool and abdominal pain may differ in their symptom contribution. The use of a 4-point ordinal scale for abdominal pain allows this score to be directly derived from the CDAI and avoids the inherent complexity of heterogeneous pain scores in one protocol.

Endoscopic improvement, defined as a change in SES-CD score ≥ 50% from the baseline score (Ferrante M, et al., Gastroenterology 145:978-86, 2013), is a key secondary endpoint. The SES-CD consists of four endoscopic variables (ulcers, ulcerated surface, inflamed surface, and presence of narrowings) that are scored in five ileocolonic segments. The SES-CD was prospectively developed and validated in patients with mild to severe CD (according to CDAI) by Daperno et al., Gastrointest. Endosc. 60(4):505-12, 2004. This scoring system was recommended by the FDA and is generally preferred by physicians to other measures because of quantification of ulcer size (rather than a qualitative assessment of ulcer characteristics), determination of the percent ulceration in a segment (rather than determination by a visual analogue scale), and for better inter rater reliability (inter class correlation coefficients were 0.83 and 0.71 for SES-CD and Crohn's Disease Endoscopic Index of Severity, respectively; Khanna et al., Aliment Pharmacol. Ther. 41:77-86, 2015). Because the score does not adjust for the number of visible segments, only segments visualized at baseline will be included in the endpoint assessment. This means any new narrowing due to inflammation that renders a segment not evaluable for scoring after study treatment will qualify the patient as not achieving endoscopic improvement for the secondary endpoint analysis. Similarly, any improvement in inflammation that renders a segment evaluable for scoring after study treatment will not be reflected in the endpoint assessment. The statistical analysis plan will describe any sensitivity analysis which are planned to assess the impact of these missing data on the endpoint. Given the limited experience with endoscopic scoring systems in general gastroenterology practice, all ileocolonoscopies in the study will be recorded at the investigational site, but central readers will determine the SES-CD scores.

Published studies have used multiple endoscopic endpoints to assess efficacy, which appear to be arbitrarily defined. Mucosal healing, defined qualitatively as an absence of mucosal ulcers, has been studied as a primary or secondary endpoint in a number of Phase IV studies (Rutgeerts P, et al., Gastro 126:1593-610, 2004 [natalizumab]; Rutgeerts P, et al., Gastrointest Endosc 63:433-42, 2006 [infliximab]; Colombel JF, Sandborn WJ, Reinisch W, et al. Infliximab, azathioprine, or combination therapy for Crohn's disease.et al., N Engl J Med 362:1383-95, 2010 [infliximab and azathioprine]; Hebuterne et al., Gut 62:201-8, 2013 [certolizumab]; Rutgeerts P, et al., Gastroenterology 142(5):1047-9, 2012 [adalimumab]). Some studies have defined endoscopic thresholds that represent endoscopic remission without clarity on the clinical significance of these scores (Hebuterne et al., *Id.* [certolizumab]; Rutgeerts et al., *Id.* 2012 [adalimumab]). In this study, endoscopic improvement is defined as the proportion of patients demonstrating a ≥ 50% reduction in SES-CD score versus their pretreatment baseline score. For induction, this endpoint will be measured at Week 14 and, for maintenance, at Week 66 among patients who achieved a clinical response at Week 14. The endpoint is based on recent post hoc analyses of the SONIC trial which determined a ≥ 50% reduction in SES-CD score to be predictive of CS-free CDAI remission following 50 weeks of treatment with biologic therapy (Ferrante, *Id.,* 2013). This definition is appropriate also, when considering the large variability in SES-CD score change at the end of a 6-week Induction Phase measured in a sample of 24 placebo patients (with moderate to severely active CD) who were participating in one of two trials of novel biologic agents (Ferrante et al. Gastroenterology 138, Issue 5, Supplement 1, S-358, 2010). The dataset showed that 6 patients achieved both 50% reduction in either SES-CD or CDEIS score, and at least a 5-point reduction in either CDEIS or SES-CD score.

### Rationale for Patient Population

Patients with uncontrolled, moderate to severely active CD are at risk for developing stricturing or penetrating complications of inflammation, as well as symptoms that are debilitating to quality of life. The treatment goals for CD are to induce and maintain symptom improvement, induce mucosal healing, and improve quality of life. However, for a significant proportion of patients, these goals are not met by current therapies (see Section **Error! Reference source not found.).** As such, the study population will include patients who have not achieved or maintained clinical remission with conventional therapy (IS and/or CS) or anti-TNF inhibitors. Data from previous studies of etrolizumab in ulcerative colitis (Rutgeerts, PJ et al., Gut 62:1122-1130, 2013; Vermeire et al., Lancet 384:309-18, 2014) and the vedolizumab GEMINI 2 and 3 studies (Sandborn WJ, et al., Aliment Pharmacol Ther 37:204-13, 2013; Sands BE, et al., Gastroenterology 147:618-27, 2014) have demonstrated the favorable efficacy of the anti-α4β7 mechanism of action in these patient subgroups. Patients belonging to each subgroup will be identified on the basis of refractory and inadequate-response criteria described herein.

Patients with moderate to severely active CD between the ages of 18 and 80 years of age will be studied. This age range is typical of patients enrolled in clinical trials of new investigational agents for CD and reflects the observation that adult CD can become or persist as moderate to severely active disease at any age. Given that the primary clearance mechanism for etrolizumab is neither renal elimination nor first-pass metabolism, the risk of accumulation in patients >65 years of age is considered low and is also mitigated by laboratory exclusions related to poor renal and hepatic function.

In line with EMA guidelines, eligible patients must have an established diagnosis of CD for at least 3 months, with moderately to severely active disease corroborated by endoscopic evidence of inflammation. This will allow improved specificity of the CDAI and PRO2 assessments and also allow the evaluation of endoscopic improvement endpoint. On the basis of the construction of the SES-CD score, patients with isolated ileitis or post-ileocecal resection can be expected to have a lower baseline score compared with patients with ileocolonic disease, regardless of whether the extent of inflammation and ulceration is the same in affected segments. As such, different SES-CD entry scores are proposed for these subgroups of patients.

### Rationale for the Design of Induction and Maintenance Phases

Aligned with the EMA guideline, randomization into the Induction Phase will be stratified by disease activity on the basis of CDAI score ≤330 or >330 (predictive of CDAI response and remission rates with biologic therapy; Sandborn WJ, et al., Aliment Pharmacol Ther 37:204-13, 2013; Sands BE, et al., Gastroenterology 147:618-27, 2014), CS use, IS use, and prior anti-TNF failure (all indicators of disease activity). These factors are considered sufficient to mitigate the risk of imbalance in disease severity across treatment arms.

Assessment of the induction of clinical remission and endoscopic improvement at Week 14 is justified on the basis of the observation that anti-integrin therapies have a slower onset of action compared with anti-TNF therapies (Sandborn WJ, et al., N Engl J Med 353:1912-25, 2005; Sandborn WJ, et al., Aliment Pharmacol Ther 37:204-13, 2013), as well as the clinical consensus that the onset of endoscopic improvement is typically observed 16-24 weeks after induction therapy, particularly in treatment refractory patients (a consensus supported by observed data showing that Mayo Clinic Score remission with etrolizumab took up to 14 weeks in TNF-IR patients with UC (see Vermeire et al., Lancet 384:309-18, 2014). A challenge of the 14-week Induction Phase is the requirement to keep concomitant CD therapies stable for the duration, so as not to confound the endpoint analysis. This is problematic in the context of patients who require rescue therapy to treat a flare and patients who are responding to treatment but unable to taper their CS dose. The study design addresses this by allowing use of rescue therapy in case of disease worsening, in which case patients would be classified as non-responders for the primary analysis, and restricting the maximum baseline CS to ≤20mg/day prednisone-equivalent dose. Although not prohibited therapeutic adjustment, increasing the dose of anti-diarrheal drugs should be avoided. Patients should keep their dose of anti-diarrheal medication stable as much as possible. Use of anti-diarrheal medication did not appear to generate a disproportionately high placebo-response rate for PRO2, compared with the placebo-response rate for CDAI. This result was observed in a sensitivity analysis of PRO2 with use of retrospective data from a randomized controlled trial of MTX in mild to moderately active CD, which detected similar remission rates for MTX and placebo when measured by PRO2 or CDAI (Khanna et al., Aliment Pharmacol. Ther. 41:77-86, 2015). The impact of titrating anti-diarrheal medication on the placebo-response rate for PRO2, in a moderate to severely active population however, is unknown.

Although PRO2 remission and endoscopic improvement incorporate the FDA's approach to defining new therapeutic outcome measures for CD, the CDAI is the only prospectively validated endpoint for CD. For this reason, as well as a pivotal induction cohort (Cohort 3) the Induction Phase includes an exploratory cohort (Cohort 1) that is adequately sized to assess effect size and accuracy of statistical planning assumptions for the new endpoints, the clinical validity of the dichotomous endpoint definitions, and the testing hierarchy of the endpoints. An active-treatment induction cohort (Cohort 2) is also included in this study to generate approximately 160 clinical remitters per arm for a statistically powered assessment of the maintenance of remission. The final cohort (Cohort 3) is a pivotal induction cohort, which will generate data for the endpoint analysis of the induction study.

In accordance with EMA guideline, maintenance of remission in the Induction Phase will be confirmed by a secondary endpoint assessing the proportion of patients in CDAI remission at both Weeks 10 and 14. Patients achieving a CDAI-70 response at Week 14 without rescue therapy use will be re-randomized into the Maintenance Phase. Patients achieving PRO2 remission and endoscopic improvement are hypothesized to be a subpopulation within this group. Randomization into the Maintenance Phase will be stratified by the use of disease activity, (described for the Induction Phase, except CDAI-remission (score <150) at both Weeks 10 and 14 will be used instead of CDAI ≤330 or >330). In addition, the randomization will be stratified by assignment to low- or high-dose etrolizumab (allowing an assessment of the impact of low- or high-dose induction therapy on the maintenance endpoints). Given the large number of strata, any potential imbalance in the proportion of endoscopic improvers at Week 14 will be handled using a covariate-adjusted analysis for the Week 66 endoscopic-improvement endpoint.

The duration of the Maintenance Phase (60 weeks) is considered an appropriate period by the FDA and EMA to establish maintenance of clinical remission. During the first 8 weeks of the Maintenance Phase, patients receiving CS during the Induction Phase will undergo weekly dose reductions aligned with recommendations for CS tapering in the current American College of Gastroenterology and European Crohn's and Colitis Organization guidelines and following the EMA recommendation to avoid a rapid taper. The tapering schedule will allow patients to be assessed for the ex U.S. primary outcome of maintenance of CDAI remission after at least 52-weeks, and CS-free throughout, among patients who achieved CDAI remission at Weeks 10 and 14.

### Rationale for Etrolizumab Dose, Dose Ranging and Schedule

Because etrolizumab has been evaluated in patients with moderate to severely active UC in a Phase II study (Vermeire et al., Lancet 384:309-18, 2014) in which clinically meaningful induction of disease remission was achieved at 105 mg (nominal dose of 0.7 mL 150mg/mL etrolizumab formulation) administered every four weeks (Q4W) (three doses at Weeks 0, 4, and 8) and 315 mg Q4W+loading dose (LD [LD of 420 mg at Week 0, 315 mg at Weeks 2, 4, and 8]) without significant safety concerns, the dose regimen (105 mg SC Q4W) of etrolizumab is also proposed as one of the doses to be tested in this study. In addition, in the Induction Phase, patients will be randomized to receive placebo, low-dose etrolizumab or high-dose etrolizumab.

Low-dose etrolizumab (105 mg) will be administered Q4W SC (Weeks 0, 4, 8, and 12). A low dose regimen of 105 mg SC at Q4W is specified for dose ranging in the Induction Phase on the basis of the following considerations: (1) In the Phase II UC trial, a nominal dose of 100 mg (0.7 mL of 150 mg/mL solution via vial and syringe), actual dose of 105 mg, administered Q4W SC, showed a clinically meaningful induction of remission in patients with UC and had a favorable safety profile in the Phase II trial (Vermeire et al., Lancet 384:309-18, 2014); (2) The exposure of 105 mg, administered Q4W SC, was shown to be sufficient for maximal β7-receptor occupancy in both blood and colonic tissue from all patients who provided evaluable samples in the Phase II trial, *Id.*, (3) Population PK/PD modeling predicts that a dose lower than the 105-mg SC Q4W regimen (e.g., 50 mg Q4W SC) will result in the loss of maximal β7-receptor occupancy during the Q4W dosing interval in approximately 44% of patients, and exposure is likely to be in the nonlinear PK range.

In addition to the 105-mg Q4W dose, a higher dose regimen of 210 mg SC at Weeks 0, 2, 4, 8, and 12 is specified for dose ranging in the Induction Phase on the basis of the following considerations: (1) Despite the similarities in pathogenesis, CD exhibits a more complex anatomical disease presentation throughout the GI tract (i.e., transmural inflammation, patchy distribution, and strictures) when compared with UC. A positive exposure-response relationship was reported recently for vedolizumab, an in-class, anti-integrin antibody, following the Induction Phase of a Phase III clinical trial in patients with CD. Results showed the induction dose is sufficient to reach a complete receptor occupancy in all patients (Rosario M, et al., (abstract) Crohn's and Colitis Foundation of America, Advances in Inflammatory Bowel Diseases, Abstract P-140, 2013), yet an increase in clinical response/remission was observed in patients with higher drug concentrations (Sandborn WJ, et al., Aliment Pharmacol Ther 37:204-13, 2013; Rosario M, et al., (abstract), European Crohn's and Colitis Organisation Congress, abstract P489, 2014). These observations from vedolizumab studies suggest that higher etrolizumab exposure may have the potential to offer more clinical benefit in this patient population; (2) in addition to Q4W dose of 210 mg, the additional 210 mg dose at Week 2 is intended to load the etrolizumab exposure upfront to allow exposure achieving steady state faster. Earlier loading dose of anti-TNF agents or anti-integrins antibodies (Rutgeerts P, et al., Gastro 126:1593-610, 2004) were found to be effective in inducing clinical remission, and such a loading dose strategy was also implemented in this study; (3) the proposed higher dose of 210 mg × 5 doses SC (at Weeks 0, 2, 4, 8, and 12) in patients with CD will result in a 2.5-fold total dose separation from the 105 mg Q4W × 4 dose regimen and is predicted to achieve an exposure level 30% lower than that from the 315 mg+420 mg LD cohort studied in the UC Phase II trial which had an acceptable safety/tolerability profile (see *supra*). In summary, given the favorable safety profile and positive clinical outcomes observed in the nominal 100-mg Q4W cohort (actual dose 105 mg) in etrolizumab UC Phase II study, it is appropriate to evaluate the 105-mg Q4W dose for CD induction therapy. In addition, given the complex pathophysiology of CD, an observed positive exposure-efficacy relationship for vedolizumab treatment despite a full receptor saturation in the blood, the available safety coverage and an acceptable safety profile of etrolizumab, it is scientifically sound to evaluate a higher dose regimen of 210 mg (Weeks 0, 2, 4, 8, and 12) to further understand the dose-response relationship in patients with CD during the Induction Phase.

### Rationale for the Maintenance Dose Regimen

Patients who respond to etrolizumab treatment in the Induction Phase (achieve a decrease from CDAI baseline score of at least 70 points, "CDAI-70 response") will be re-randomized to receive 105 mg etrolizumab or placebo Q4W in the Maintenance Phase; patients who respond to placebo in the Induction Phase will continue to receive placebo in the Maintenance Phase. A low-dose regimen of 105 mg SC at Q4W is specified in the Maintenance Phase on the basis the following considerations: (1) The 105-mg Q4W SC dose planned for the Phase III study in CD is anticipated (by population modeling) to maintain full β7-receptor occupancy at all times in >85% of patients. The nominal dose of 100 mg Q4W SC (105 mg actual dose) administered in the UC Phase II study demonstrated an acceptable safety profile (see *supra*); (2) the in-class anti-integrin vedolizumab was successful in maintaining remission with an every-8-week regimen that provided an average steady-state trough serum concentration sufficient to maintain maximal receptor occupancy (Rosario M, et al., (abstract) Crohn's and Colitis Foundation of America, Advances in Inflammatory Bowel Diseases, Abstract P-140, 2013; Sandborn WJ, et al., Aliment Pharmacol Ther 37:204-13, 2013).

### Patient-Reported Outcomes Assessments

PROs (IBDQ, CD-PRO/SS, EQ-5D, PRO2 and the loose stool frequency, abdominal pain, and general well-being components of the CDAI) will be collected to help characterize the patient-reported clinical profile of etrolizumab. The instruments will be translated as required into the local language. PRO data are collected electronically with the use of electronic PRO (ePRO) devices (i.e., e-diary and tablet). The investigator staff will provide the patient with an e-diary and instructions for completing the PRO questionnaires electronically for those PROs that need to be completed outside of the clinic. Patients will also be instructed to contact the site promptly if they have any questions about the use of the e-diary during screening or at any time during the study. For instances when PROs are to be completed at the clinic, the patient will fill them out on a tablet. Review electronic data captured by the patient since the previous study visit with the patient at each clinic visit. ePRO data is collected and assessed at visits. During screening, patients will be instructed on how to appropriately use and complete questions on the e-diary. The signs and symptoms of CD, specifically, number of liquid or soft stools, abdominal pain, and general well-being, must be recorded daily throughout the study, including the screening period. To ensure instrument validity and that data standards meet Health Authority requirements, the PROs completed at the sites (IBDQ and EQ-5D) must be administered at the investigational site prior to the completion of other non-PRO assessments and before the patient receives any disease-status information or study drug during that visit.

As discussed above, the CDAI quantifies the signs and symptoms of patients with CD (Table 1). The CDAI consists of eight factors; each factor is summed after adjustment with a weighting factor (Table 1). The components of the CDAI include number of liquid or soft stools, abdominal pain, general well-being, presence of complications, use of Lomitil or other opiates for diarrhea, presence of an abdominal mass, hematocrit, and percentage deviation from standard weight. Patients are to report their abdominal pain severity, loose-stool frequency, and general well-being on the e-diary on a daily basis. The Bristol Stool Scale is provided to patients as a reference for determining loose stools (Fig. 5). Because the ileocolonoscopy preparations can interfere with the assessment of other clinical parameters, e-diary entries used to calculate the complete CDAI should not correspond to day(s) of bowel preparation, endoscopy, or the day after endoscopy.

As discussed above, the PRO2 evaluates two patient reported factors: the frequency of liquid or soft stools and abdominal pain. The score is calculated using the CDAI-weighted sum of the average frequency and pain scores in a 7-day period. Patients are to report their loose-stool frequency (the Bristol Stool Scale [Fig. 5] will be provided) and abdominal pain severity on the e-diary on a daily basis. As with the CDAI, the PRO2 score should not use e-diary entries that correspond to day(s) of bowel preparation, endoscopy, or the day after endoscopy, to avoid interference related with the ileocolonoscopy.

The CD-PRO/SS measure will be used to assess patient-reported CD signs and symptoms. The 14-item questionnaire (some questions contain supplementary questions regarding severity/frequency) contains two domains: CD signs and symptoms and systemic symptoms. The CD-PRO/SS assesses the presence of CD signs and symptoms, and in some cases, the severity or frequency of the symptoms. The CD-PRO/SS measure has a recall specification of 24 hours. Patients complete the CD-PRO/SS on the e-diary at starting at Week 0 and thereafter for the 9 consecutive days preceding each clinic or telephone visit throughout the entire study.

The IBDQ assesses patients' health-related quality of life (HRQOL; Guyatt G, et al., J Clin Epidemiol 42(5):403-408, 1989; Irvine EJ, J Pediatr Gastroenterol Nutr 28:S23-7, 1999). The 32-item questionnaire contains four domains: bowel symptoms (10 items), systemic symptoms (five items), emotional function (12 items), and social function (five items). The items are scored on a 7-point Likert scale with a higher score indicating better HRQOL. The IBDQ has a recall specification of 2 weeks. Patients complete the IBDQ at the investigational site on a tablet at baseline, at Weeks 0, 14, 44, and 74 prior to the completion of other non-PRO assessments and before the patient receives any disease-status information or study drug during that visit.

The EuroQol Five Dimension Questionnaire (EQ-5D) is a generic preference-based HRQOL that provides a single index value for health status (Rabin R, et al., Enn Med 33:337-43, 2001). This tool includes questions about mobility, self-care, usual activities, pain/discomfort, and anxiety/depression that are used to build a composite of the patient's health status. Patients complete the EQ-5D at the investigational site on a tablet at Weeks 0, 14, 44, and 74 (or Early Withdrawal visit) prior to the completion of other non-PRO assessments and before the patient receives any disease-status information or study drug during that visit.

### Statistical Considerations and Analysis Plan

For the Induction Phase, a total of approximately 1250 patients will be randomized into one of three induction cohorts. The sample size of each cohort is summarized in Table 3 and described below.

**Table 3. Induction Phase Sample Size for Each Cohort.**

| Cohort | No. of Patients | | | |
|---|---|---|---|---|
| | Total | Placebo | Low-Dose (105mg) | High-Dose (210mg) |
| Exploratory induction cohort (Cohort 1) | ~300 | ~60 | ~120 | ~120 |
| Active-treatment induction cohort (Cohort 2) | ~350 | NA | ~175 | ∼175 |
| Pivotal induction cohort (Cohort 3) | ~600 | ~150 | ~225 | ~225 |

| | | | | |
|---|---|---|---|---|
| NA = not applicable. | | | | |

The sample size for Cohort 1 (see Table 3) provides approximately 90% power to detect a ≥20% difference in PRO2 or CDAI remission rates between each etrolizumab arm and placebo (under the assumption of placebo remission rate of ≤ 15%, similar to results reported in the GEMINI 2 trial of vedolizumab in patients with CD; Sandborn WJ, et al., Aliment Pharmacol Ther 37:204-13, 2013) and approximately 80% power to detect a 15% difference versus placebo in endoscopic response (under the assumption of placebo response rate of ≤ 10%) and a two-sided chi-square test at the 0.1 significance level.

The sample size for Cohort 3 is expected to provide ≥85% power to detect a ≥ 15% difference in PRO2 or CDAI remission rates between each etrolizumab arm and placebo under the assumption of a placebo remission rate of ≤ 15% and a two-sided χ² test at the 0.025 significance level (see Table 4).

In addition, these sample sizes will also provide ≥90% power to detect a 15% difference versus placebo for the key secondary endpoint of endoscopic improvement, under the assumption of placebo response rate of ≤ 10% and a two-sided chi-square test at the 0.025 significance level. Under the assumption that the placebo response rate at Week 14 is 10%, the power to detect a ≥ 15% difference versus placebo (10% vs. 25%) is estimated to be 93% at the 0.025 significance level (see Table 4).

Cohort 2 was sized to provide sufficient patients for the Maintenance Phase analyses. Additional patients may be randomized into Cohort 2, if needed, to achieve a target number of approximately 326 patients in PRO2 remission at Week 14 across all induction cohorts (1, 2, and 3).

**Table 4. Power Estimates for Primary and Key Secondary Efficacy Analysis in the Pivotal Induction Phase and Maintenace Phase.**

| Study | Endpoint | Power | Assumed Response Rates | Sample Size per Group |
|---|---|---|---|---|
| Induction | PRO2 or CDAI remission | 87% ^{a} | Placebo = 15% Etrolizumab = 30% | Placebo = 150 Etrolizumab=225 |
| | Endoscopic improvement | 80% ^{a} | Placebo = 5% Etrolizumab = 15% | Placebo = 150 Etrolizumab=225 |
| Maintenance | PRO2 remission | 80% ^{b} | Placebo = up to 30% Etrolizumab = 45% | 163 |
| | CS-free CDAI remission after 52 weeks | 80% ^{b} | Placebo = 10% Etrolizumab = 25% | 100 |
| | Endoscopic improvement | 94% ^{b} | Placebo = up to 30% Etrolizumab=45% | 260 |

| | | | | |
|---|---|---|---|---|
| CDAI = Crohn's Disease Activity Index; PRO2= Patient-Reported Outcomes-2 score. ^{a} Type I error, α = 2.5%. ^{b} Type I error, α = 5.0%. | | | | |

For the Maintenance Phase, the primary endpoints for the Maintenance Phase are PRO2 remission at Week 66, among etrolizumab patients in PRO2 remission at Week 14 (FDA only), and CS-free CDAI remission after at least 52 weeks of maintenance treatment, while off steroids for at least 52 weeks, among patients who achieved CDAI remission at both Week 10 and 14. Assuming a placebo PRO2 Week 66 remission rate, among Week 14 remitters, of up to 30%, a total of approximately 326 etrolizumab patients in PRO2 remission at Week 14 would provide ≥80% power to detect a 15% treatment difference using a two-sided chi-square test at the 0.05 significance level. This sample size would be achieved with the planned number of etrolizumab induction patients of 1040, if the PRO2 remission rate at Week 14 is at least 31%. It is projected that the PRO2 remission rate at Week 14 will be at least 30%, assuming similar or higher remission rates compared to CDAI remission.

Assuming a placebo remission rate for the CS-free CDAI remission maintenance primary endpoint of ≤ 10%, a total of approximately 200 patients in CDAI remission at Weeks 10 and 14 would provide ≥ 80% power to detect a 15% treatment difference using a two-sided chi-square test at the 0.05 significance level. This sample size is expected to be achieved with the planned number of etrolizumab induction patients (n = 1040) assuming the rate of CDAI remission at Weeks 10 and 14 with etrolizumab is at least 20%.

Under the assumption that at least 50% of patients treated with etrolizumab in the Induction Phase (cohorts 1, 2, and 3) achieve a CDAI-70 response at Week 14, a total of approximately 520 patients (approximately 260 per arm) will be re-randomized into the pivotal maintenance cohort. This sample size would also provide approximately 95% power to detect a ≥ 15% difference versus placebo for the secondary endoscopic improvement test at the 0.05 significance level (see Table 4).

### Efficacy Analyses

For the purpose of statistical analyses, the Induction and Maintenance Phases will be treated as independent studies. Patients who are non-evaluable for efficacy at a specific timepoint, due to missing data, will be considered non-responders for all categorical endpoints. In addition, patients requiring permitted rescue medication, and/or did not reinitiate the steroid-tapering regimen within 2 weeks of any CS increase for worsening of CD and/or surgical intervention for CD, and/or took prohibited medications (e.g., anti-integrins, T- or B- cell-depleting agents, TNF antagonists, anti-metabolites, cyclosporine, tacrolimus and after week 14, immunosuppressant medications such as AZA, 6-MP, and MTX) will be considered non-responders for the analysis. The following analyses will be performed for the primary efficacy endpoint and key secondary efficacy endpoints: (1) subgroup analyses to evaluate the consistency of results across prespecified subgroups (including baseline anti-TNF-status [naive vs. IR], baseline CS status [on CS vs. not on CS], baseline IS status [on IS vs. not on IS], age, sex, race/ethnicity, etc.) and (2) sensitivity analyses to evaluate the robustness of results to the primary analysis methods (e.g., handling of dropouts).

### Induction Phase

Efficacy analyses for the Induction Phase will be performed separately for each cohort, and will include all patients who were randomized and received at least one dose of study (modified intent-to-treat population [mITT]). Patients will be grouped according to the treatment assigned at randomization.

*Cohort 1:* An exploratory analysis of the endpoints will take place when all patients in Cohort 1 (n=300) have completed the Week 14 visit or, if not complete, have discontinued the study. At this point, the Sponsor will be unblind to individual treatment assignments in Cohort 1. Patients, site monitors, and investigators will remain blind to patient-specific treatment assignments.

This exploratory analysis is to inform the analysis plan for the pivotal Cohort 3, to assess the statistical assumptions of the PRO2 and SES-CD endpoint definitions, and to refine statistical methodology, as needed. The exploratory analysis of Cohort 1 is not intended to inform the study design/conduct of cohorts 2 or 3, or the maintenance study; it will therefore be conducted while Cohort 2 is enrolling.

The primary efficacy assessment will test for differences in the proportion of patients who achieve CDAI remission, and PRO2 remission, respectively at Week 14 in each etrolizumab dose arm versus placebo using the Cochran-Mantel-Haenszel (CMH) test, with stratification according to the stratification factors used at randomization at the 0.10 significance level. The absolute treatment difference will be provided along with the 90% two-sided CI estimate.

All categorical secondary endpoints will be analyzed using the same methodology as the primary endpoint.

Continuous endpoints will be analyzed using an analysis of covariance (ANCOVA) model with the stratification variables used at randomization and the baseline value of the studied measure as a covariate.

Summary statistics of the continuous endpoints will be calculated for each treatment arm for absolute changes and raw values. Means, SDs, medians, and lower and upper quartiles and minimum and maximum will be reported. For all summaries involving change from baseline, patients without baseline values will be excluded from the analyses. In addition to the 95% CIs, two-sided p-values will be reported for all secondary efficacy endpoints. No adjustments for multiplicity will be made.

*Cohort 2*: Cohort 2 is considered a "feeder" cohort to help achieve the necessary sample size for the maintenance study. All primary and secondary efficacy parameters will be summarized descriptively for each treatment arm. Demographic and baseline characteristics such as age, sex, race, region, use of corticosteroids and immunosuppressants, duration of disease and CD activity scores will be summarized for each treatment group by use of descriptive statistics.

*Cohort 3:* The primary endpoint analysis will compare for each etrolizumab dose arm versus the placebo arm the proportion of patients who achieve PRO2 remission (analysis for the FDA) or CDAI remission (analysis for ex-U.S.) at Week 14The primary endpoint analysis will compare for each etrolizumab dose arm versus the placebo arm the proportion of patients who achieve PRO2 remission (analysis for the FDA) or CDAI remission (analysis for ex-U.S.) at Week 14.

The difference between each etrolizumab arm and placebo arm will be evaluated using the CMH test statistic stratified by the factors used at randomization. The absolute treatment difference will be provided along with the 95% two-sided CI estimate.

All categorical secondary endpoints will be analyzed using the same methodology as the primary endpoint. For all efficacy endpoints, descriptive summary statistics will be provided for each treatment arm.

Continuous endpoints will be analyzed using an ANCOVA model with the stratification variables used at randomization and the baseline value of the studied measure as a covariate.

For each of the two comparisons of the primary endpoint (low dose vs. placebo and high dose vs. placebo), a Bonferroni-adjusted 0.025 two-sided significance level will be used. If neither dose is declared significant for the primary endpoint, all other hypothesis tests will be considered exploratory. For any dose regimen that is significant at the two-sided 0.025 level, key secondary endpoints will be tested sequentially. The remaining secondary endpoints and all exploratory endpoints will be considered to provide supportive information and no adjustments for multiple comparisons will be performed.

### Maintenance Phase

Efficacy analyses for the Maintenance Phase will include all etrolizumab induction patients who were randomized into the Maintenance Phase and received at least one dose of study drug (mITT population). Patients will be grouped according to the treatment assigned at randomization into the Maintenance Phase.

For all categorical endpoints, the difference in proportions between the two treatment arms will be evaluated using the CMH test statistic stratified by the factors used at randomization into the Maintenance Phase. The test will be performed at the two-sided 0.05 significance level. The absolute treatment difference will be provided along with the 95% two-sided CI estimate. All categorical secondary endpoints will be analyzed using the same methodology as for the primary endpoint.

Continuous endpoints will be analyzed using an ANCOVA model with the stratification variables used at randomization and the baseline value of the studied measure as a covariate.

If the primary endpoint is statistically significant, key secondary endpoints will be tested sequentially.

Patients who are non-evaluable for efficacy at a specific timepoint (e.g., because of missing data or early enrollment into the OLE study) will be considered non-responders for all categorical endpoints.
Embodiment 1: A method of inducing remission in a patient with Crohn's disease, the method comprising administering subcutaneously to the patient a therapeutically effective amount of an integrin beta7 antagonist, wherein the therapeutically effective amount induces remission 14 weeks after administration of a first dose.
Embodiment 2. The method of embodiment 1, wherein the integrin beta7 antagonist is a monoclonal anti-integrin beta7 antibody.
Embodiment 3. The method of embodiment 2, wherein the anti-integrin beta7 antibody is selected from a chimeric antibody, a human antibody, and a humanized antibody.
Embodiment 4. The method of embodiment 3, wherein the anti-integrin beta7 antibody is an antibody fragment.
Embodiment 5. The method of embodiment 3, wherein the anti-beta7 antibody comprises six hypervariable regions (HVRs), wherein:
   (i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs: 1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
   (ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
   (iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
   (iv) HVR-H1 comprises amino acid sequence D1-D10 wherein D1-D10 is GFFITNNYWG (SEQ ID NO:4);
   (v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
   (vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.
Embodiment 6. The method of Embodiment 5, wherein the anti-integrin beta7 antibody comprises three heavy chain hypervariable region (HVR-H1-H3) sequences and three light chain hypervariable region (HVR-L1-L3) sequences, wherein:
   (i) HVR-L1 comprises SEQ ID NO:7, SEQ ID NO:8 or SEQ ID NO:9;
   (ii) HVR-L2 comprises SEQ ID NO:2;
   (iii) HVR-L3 comprises SEQ ID NO:3;
   (iv) HVR-H1 comprises SEQ ID NO:4;
   (v) HVR-H2 comprises SEQ ID NO:5; and
   (vi) HVR-H3 comprises SEQ ID NO:6 or SEQ ID NO:16 or SEQ ID NO:17 or SEQ ID NO:19.
Embodiment 7. The method of embodiment 6, wherein the anti-integrin beta7 antibody comprises a variable light chain comprising the amino acid sequence of SEQ ID NO:31 and a variable heavy chain comprising the amino acid sequence of SEQ ID NO:32.
Embodiment 8. The method of embodiment 7, wherein the anti-integrin beta7 antibody is etrolizumab.
Embodiment 9. The method of any one of embodiments 1-8, wherein the patient has moderately to severely active Crohn's disease prior to administration of the first dose of the integrin beta7 antagonist.
Embodiment 10. The method of embodiment 9, wherein the patient is determined to have a Crohn's Disease Activity Index (CDAI) score of greater than or equal to 220 and less than or equal to 480 at any time in the seven days prior to administration of the first dose.
Embodiment 11. The method of embodiment 9 or 10, wherein the patient is determined to have a Patient Reported Outcomes 2 (PRO2) score of greater than or equal to 14 at any time in the seven days prior to administration of the first dose.
Embodiment 12. The method of any one of embodiments 9-11, wherein the patient is determined to have active inflammation, wherein the active inflammation is determined as a Simplified Endoscopic Index for Crohn's Disease (SES-CD) score of greater than or equal to 7 as determined by ileocolonoscopy.
Embodiment 13. The method of any one of embodiments 9-11, wherein the patient has isolated ileitis or post-ileocecal resection and wherein the patient is determined to have active inflammation, wherein the active inflammation is determined as a SES-CD score of greater than or equal to 4 as determined by ileocolonoscopy.
Embodiment 14. The method of any one of embodiments 9-13, wherein the patient had an inadequate response, a loss of response, or intolerance to conventional therapy.
Embodiment 15. The method of embodiment 14, wherein the conventional therapy is selected from one or more of immunosuppressant therapy, corticosteroid therapy, and anti-TNF therapy.
Embodiment 16. The method of embodiment 15, wherein the immunosuppressant therapy is selected from 6-mercaptopurine, azathioprine, and methotrexate.
Embodiment 17. The method of embodiment 15, wherein the corticosteroid therapy is selected from prednisone, prednisone equivalent, and budesonide.
Embodiment 18. The method of embodiment 15, wherein the anti-TNF therapy is selected from infliximab, adalimumab, and certolizumab pegol.
Embodiment 19. The method of any one of embodiments 1-18, wherein the anti-integrin beta7 antibody is administered at a flat dose of 105 mg every 4 weeks or at a flat dose of 210 mg every 4 weeks.
Embodiment 20. The method of any one of embodiments 1-18, wherein the anti-integtrin beta7 antibody is administered as a flat dose of 210 mg at the first dose, 210 mg two weeks after the first dose, 210 mg four weeks after the first dose, 210 mg eight weeks after the first dose, and 210 mg 12 weeks after the first dose.
Embodiment 21. The method of any one of embodiments 1-20, wherein remission is determined by Crohn's Disease Activity Index (CDAI) score, wherein the CDAI score is less than 150.
Embodiment 22. The method of embodiment 21, wherein the therapeutically effective amount induces remission 10 weeks after administration of the first dose.
Embodiment 23. The method of any one of embodiments 1-22, wherein remission is determined by Patient Reported Outcomes 2 (PRO2) score, wherein the PRO2 score is less than or equal to 11.
Embodiment 24. The method of any of embodiments 1-23, wherein the therapeutically effective amount induces endoscopic improvement as determined by Simplified Endoscopic Index for Crohn's Disease (SES-CD) score.
Embodiment 25. The method of embodiment 24, wherein the SES-CD score determined 14 weeks after administration of the first dose is reduced by 50% compared to the SES-CD score determined at baseline.
Embodiment 26. The method of any one of embodiments 1-25, wherein the therapeutically effective amount induces a response, wherein the response is determined as a decrease of CDAI score of at least 70 points compared to CDAI score determined at baseline.
Embodiment 27. The method of 26, wherein the response is determined as a decrease of CDAI score of at least 100 points compared to CDAI score determined at baseline.
Embodiment 28. A method of maintaining remission in a patient with Crohn's disease, the method comprising administering subcutaneously to the patient a therapeutically effective amount of an integrin beta7 antagonist, wherein the therapeutically effective amount maintains remission for at least 52 weeks, or for at least 66 weeks, or for at least 70 weeks, or for at least 74 weeks, after administration of a first dose.
Embodiment 29. The method of embodiment 28, wherein the therapeutically effective amount maintains remission for at least 74 weeks after administration of the first dose, wherein the patient receives corticosteroid therapy for 14 weeks after administration of the first dose and the corticosteroid therapy is reduced over time beginning at 14 weeks after administration of the first dose until discontinuation.
Embodiment 30. The method of embodiment 29, wherein the corticosteroid therapy is (i) less than or equal to 20 mg of prednisone per day and wherein the corticosteroid therapy is reduced by 2.5 mg prednisone per week until discontinuation or (ii) less than or equal to 20 mg of prednisone equivalent per day and wherein the corticosteroid therapy is reduced by 2.5 mg prednisone equivalent per week until discontinuation.
Embodiment 31. The method of embodiment 29, wherein the corticosteroid therapy is less than or equal to 6 mg oral budesonide per day and wherein the he corticosteroid therapy is reduced by 3 mg oral budesonide every 2 weeks until discontinuation.
Embodiment 32. The method of any one embodiments 28-31, wherein the therapeutically effective amount maintains durable remission, wherein durable remission is determined by CDAI score less than 150 at each of six or more timepoints selected from, 24 weeks after administration of the first dose, 28 weeks after administration of the first dose, 32 weeks after administration of the first dose, 44 weeks after administration of the first dose, 56 weeks after administration of the first dose, 66 weeks after administration of the first dose, 70 weeks after administration of the first dose, and 74 weeks after administration of the first dose.
Embodiment 33. The method of any one of embodiments 28-32, wherein the integrin beta7 antagonist is a monoclonal anti-integrin beta7 antibody.
Embodiment 34. The method of embodiment 33, wherein the anti-integrin beta7 antibody is selected from a chimeric antibody, a human antibody, and a humanized antibody.
Embodiment 35. The method of embodiment 34, wherein the anti-integrin beta7 antibody is an antibody fragment.
Embodiment 36. The method of embodiment 34, wherein the anti-beta7 antibody comprises six hypervariable regions (HVRs), wherein:
   (i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs: 1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
   (ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
   (iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
   (iv) HVR-H1 comprises amino acid sequence D1-D10 wherein D1-D10 is GFFITNNYWG (SEQ ID NO:4);
   (v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
   (vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO: 17), or AQTGSSGYFDF (SEQ ID NO: 18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.
Embodiment 37. The method of Embodiment 36, wherein the anti-integrin beta7 antibody comprises three heavy chain hypervariable region (HVR-H1-H3) sequences and three light chain hypervariable region (HVR-L1-L3) sequences, wherein:
   (i) HVR-L1 comprises SEQ ID NO:7, SEQ ID NO:8 or SEQ ID NO:9;
   (ii) HVR-L2 comprises SEQ ID NO:2;
   (iii) HVR-L3 comprises SEQ ID NO:3;
   (iv) HVR-H1 comprises SEQ ID NO:4;
   (v) HVR-H2 comprises SEQ ID NO:5; and
   (vi) HVR-H3 comprises SEQ ID NO:6 or SEQ ID NO:16 or SEQ ID NO:17 or SEQ ID NO:19.
Embodiment 38. The method of embodiment 37, wherein the anti-integrin beta7 antibody comprises a variable light chain comprising the amino acid sequence of SEQ ID NO:31 and a variable heavy chain comprising the amino acid sequence of SEQ ID NO:32.
Embodiment 39. The method of embodiment 38, wherein the anti-integrin beta7 antibody is etrolizumab.
Embodiment 40. The method of any one of embodiments 28-39, wherein the patient has moderately to severely active Crohn's disease prior to administration of the first dose of the integrin beta7 antagonist.
Embodiment 41. The method of embodiment 40, wherein the patient is determined to have a CDAI score of greater than or equal to 220 and less than or equal to 480 at any time in the seven days prior to administration of the first dose.
Embodiment 42. The method of embodiment 40 or 41, wherein the patient is determined to have a PRO2 score of greater than or equal to 14 at any time in the seven days prior to administration of the first dose.
Embodiment 43. The method of any one of embodiments 40-42, wherein the patient is determined to have active inflammation, wherein the active inflammation is determined as a SES-CD score of greater than or equal to 7 as determined by ileocolonoscopy.
Embodiment 44. The method of any one of embodiments 40-42, wherein the patient has isolated ileitis or post-ileocecal resection and wherein the patient is determined to have active inflammation, wherein the active inflammation is determined as a SES-CD score of greater than or equal to 4 as determined by ileocolonoscopy.
Embodiment 45. The method of any one of embodiments 40-44, wherein the patient had an inadequate response, a loss of response, or intolerance to conventional therapy.
Embodiment 46. The method of embodiment 45, wherein the conventional therapy is selected from one or more of immunosuppressant therapy, corticosteroid therapy, and anti-TNF therapy.
Embodiment 47. The method of embodiment 46, wherein the immunosuppressant therapy is selected from 6-mercaptopurine, azathioprine, and methotrexate.
Embodiment 48. The method of embodiment 46, wherein the corticosteroid therapy is selected from prednisone, prednisone equivalent, and budesonide.
Embodiment 49. The method of embodiment 46, wherein the anti-TNF therapy is selected from infliximab, adalimumab, and certolizumab pegol.
Embodiment 50. The method of any one of embodiments 28-49, wherein the anti-integrin beta7 antibody is administered at a flat dose of 105 mg every 4 weeks or at a flat dose of 210 mg every 4 weeks.
Embodiment 51. The method of any one of embodiments 28-49, wherein the anti-integtrin beta7 antibody is administered as a flat dose of 210 mg at the first dose, 210 mg two weeks after the first dose, 210 mg four weeks after the first dose, 210 mg eight weeks after the first dose, 210 mg 12 weeks after the first dose and 105 mg every 4 weeks thereafter.
Embodiment 52. The method of any one of embodiments 28-51, wherein remission is determined by Crohn's Disease Activity Index (CDAI) score, wherein the CDAI score is less than 150.
Embodiment 53. The method of embodiment 52, wherein the patient is corticosteroid-free for at least 52 weeks.
Embodiment 54. The method of any one of embodiments 28-53, wherein remission is determined by Patient Reported Outcomes 2 (PRO2) score, wherein the PRO2 score is less than or equal to 11.
Embodiment 55. The method of any of embodiments 28-54, wherein the therapeutically effective amount induces endoscopic improvement as determined by Simplified Endoscopic Index for Crohn's Disease (SES-CD) score.
Embodiment 56. The method of embodiment 55, wherein the SES-CD score determined 66 weeks after administration of the first dose is reduced by 50% compared to the SES-CD score determined at baseline.
Embodiment 57. The method of embodiment 55, wherein the endoscopic improvement 66 weeks after administration of the first dose is resolution of mucosal inflammation, wherein resolution of mucosal inflammation is SES-CD score determined as zero.
Embodiment 58. The method of any one of embodiments 28-57, wherein the therapeutically effective amount induces a response 14 weeks after administration of the first dose, wherein the response is determined as a decrease of CDAI score of at least 70 points compared to CDAI score determined at baseline.
Embodiment 59. The method of any one of embodiments 28-57, wherein the therapeutically effective amount induces a response 66 weeks after administration of the first dose, wherein the response is determined as a decrease of CDAI score of at least 100 points compared to CDAI score determined at baseline.
Embodiment 60. The method according to any one of the preceding embodiments, wherein the integrin beta7 antagonist is administered using a prefilled syringe or a prefilled syringe and autoinjector combination.

## Claims

1. A method of inducing remission in a patient with Crohn's disease, the method comprising administering subcutaneously to the patient a therapeutically effective amount of an integrin beta7 antagonist, wherein the therapeutically effective amount induces remission 14 weeks after administration of a first dose.

2. The method of claim 1, wherein the integrin beta7 antagonist is a monoclonal anti-integrin beta7 antibody.

3. The method of claim 2, wherein the anti-integrin beta7 antibody is selected from a chimeric antibody, a human antibody, and a humanized antibody.

4. The method of claim 3, wherein the anti-integrin beta7 antibody is an antibody fragment.

5. The method of claim 3, wherein the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs: 1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii)HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv)HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi)HVR-H3 comprises amino acid sequence F2-F11 wherein F2 -F11 is MTGSSGYFDF (SEQ ID NO:6) or RTGSSGYFDF (SEQ ID NO: 19); or comprises amino acid sequence F1-F11, wherein F1-F11 is AMTGSSGYFDF (SEQ ID NO:16), ARTGSSGYFDF (SEQ ID NO: 17), or AQTGSSGYFDF (SEQ ID NO: 18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

6. The method of Claim 5, wherein the anti-integrin beta7 antibody comprises three heavy chain hypervariable region (HVR-H1-H3) sequences and three light chain hypervariable region (HVR-L1-L3) sequences, wherein:
HVR-L1 comprises SEQ ID NO:7, SEQ ID NO:8 or SEQ ID NO:9;
HVR-L2 comprises SEQ ID NO:2;
HVR-L3 comprises SEQ ID NO:3;
HVR-H1 comprises SEQ ID NO:4;
HVR-H2 comprises SEQ ID NO:5; and
HVR-H3 comprises SEQ ID NO:6 or SEQ ID NO:16 or SEQ ID NO:17 or SEQ ID NO:19.

7. The method of claim 6, wherein the anti-integrin beta7 antibody comprises a variable light chain comprising the amino acid sequence of SEQ ID NO:31 and a variable heavy chain comprising the amino acid sequence of SEQ ID NO:32.

8. The method of claim 7, wherein the anti-integrin beta7 antibody is etrolizumab.

9. The method of any one of claims 1-8, wherein the patient has moderately to severely active Crohn's disease prior to administration of the first dose of the integrin beta7 antagonist.

10. The method of claim 9, wherein the patient is determined to have a Crohn's Disease Activity Index (CDAI) score of greater than or equal to 220 and less than or equal to 480 at any time in the seven days prior to administration of the first dose.

11. The method of claim 9 or 10, wherein the patient is determined to have a Patient Reported Outcomes 2 (PRO2) score of greater than or equal to 14 at any time in the seven days prior to administration of the first dose.

12. The method of any one of claims 9-11, wherein the patient is determined to have active inflammation, wherein the active inflammation is determined as a Simplified Endoscopic Index for Crohn's Disease (SES-CD) score of greater than or equal to 7 as determined by ileocolonoscopy.

13. The method of any one of claims 9-11, wherein the patient has isolated ileitis or post-ileocecal resection and wherein the patient is determined to have active inflammation, wherein the active inflammation is determined as a SES-CD score of greater than or equal to 4 as determined by ileocolonoscopy.

14. The method of any one of claims 9-13, wherein the patient had an inadequate response, a loss of response, or intolerance to conventional therapy.

15. The method of claim 14, wherein the conventional therapy is selected from one or more of immunosuppressant therapy, corticosteroid therapy, and anti-TNF therapy.

16. The method of claim 15, wherein the immunosuppressant therapy is selected from 6-mercaptopurine, azathioprine, and methotrexate.

17. The method of claim 15, wherein the corticosteroid therapy is selected from prednisone, prednisone equivalent, and budesonide.

18. The method of claim 15, wherein the anti-TNF therapy is selected from infliximab, adalimumab, and certolizumab pegol.

19. The method of any one of claims 1-18, wherein the anti-integrin beta7 antibody is administered at a flat dose of 105 mg every 4 weeks or at a flat dose of 210 mg every 4 weeks.

20. The method of any one of claims 1-18, wherein the anti-integtrin beta7 antibody is administered as a flat dose of 210 mg at the first dose, 210 mg two weeks after the first dose, 210 mg four weeks after the first dose, 210 mg eight weeks after the first dose, and 210 mg 12 weeks after the first dose.

21. The method of any one of claims 1-20, wherein remission is determined by Crohn's Disease Activity Index (CDAI) score, wherein the CDAI score is less than 150.

22. The method of claim 21, wherein the therapeutically effective amount induces remission 10 weeks after administration of the first dose.

23. The method of any one of claims 1-22, wherein remission is determined by Patient Reported Outcomes 2 (PRO2) score, wherein the PRO2 score is less than or equal to 11.

24. The method of any of claims 1-23, wherein the therapeutically effective amount induces endoscopic improvement as determined by Simplified Endoscopic Index for Crohn's Disease (SES-CD) score.

25. The method of claim 24, wherein the SES-CD score determined 14 weeks after administration of the first dose is reduced by 50% compared to the SES-CD score determined at baseline.

26. The method of any one of claims 1-25, wherein the therapeutically effective amount induces a response, wherein the response is determined as a decrease of CDAI score of at least 70 points compared to CDAI score determined at baseline.

27. The method of 26, wherein the response is determined as a decrease of CDAI score of at least 100 points compared to CDAI score determined at baseline.

28. A method of maintaining remission in a patient with Crohn's disease, the method comprising administering subcutaneously to the patient a therapeutically effective amount of an integrin beta7 antagonist, wherein the therapeutically effective amount maintains remission for at least 52 weeks, or for at least 66 weeks, or for at least 70 weeks, or for at least 74 weeks, after administration of a first dose.

29. The method of claim 28, wherein the therapeutically effective amount maintains remission for at least 74 weeks after administration of the first dose, wherein the patient receives corticosteroid therapy for 14 weeks after administration of the first dose and the corticosteroid therapy is reduced over time beginning at 14 weeks after administration of the first dose until discontinuation.

30. The method of claim 29, wherein the corticosteroid therapy is (i) less than or equal to 20 mg of prednisone per day and wherein the corticosteroid therapy is reduced by 2.5 mg prednisone per week until discontinuation or (ii) less than or equal to 20 mg of prednisone equivalent per day and wherein the corticosteroid therapy is reduced by 2.5 mg prednisone equivalent per week until discontinuation.

31. The method of claim 29, wherein the corticosteroid therapy is less than or equal to 6 mg oral budesonide per day and wherein the he corticosteroid therapy is reduced by 3 mg oral budesonide every 2 weeks until discontinuation.

32. The method of any one claims 28-31, wherein the therapeutically effective amount maintains durable remission, wherein durable remission is determined by CDAI score less than 150 at each of six or more timepoints selected from, 24 weeks after administration of the first dose, 28 weeks after administration of the first dose, 32 weeks after administration of the first dose, 44 weeks after administration of the first dose, 56 weeks after administration of the first dose, 66 weeks after administration of the first dose, 70 weeks after administration of the first dose, and 74 weeks after administration of the first dose.

33. The method of any one of claims 28-32, wherein the integrin beta7 antagonist is a monoclonal anti-integrin beta7 antibody.

34. The method of claim 33, wherein the anti-integrin beta7 antibody is selected from a chimeric antibody, a human antibody, and a humanized antibody.

35. The method of claim 34, wherein the anti-integrin beta7 antibody is an antibody fragment.

36. The method of claim 34, wherein the anti-beta7 antibody comprises six hypervariable regions (HVRs), wherein:
HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs: 1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
HVR-H1 comprises amino acid sequence D1-D10 wherein D1-D10 is GFFITNNYWG (SEQ ID NO:4);
HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO: 17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

37. The method of Claim 36, wherein the anti-integrin beta7 antibody comprises three heavy chain hypervariable region (HVR-H1-H3) sequences and three light chain hypervariable region (HVR-L1-L3) sequences, wherein:
HVR-L1 comprises SEQ ID NO:7, SEQ ID NO:8 or SEQ ID NO:9;
HVR-L2 comprises SEQ ID NO:2;
HVR-L3 comprises SEQ ID NO:3;
HVR-H1 comprises SEQ ID NO:4;
HVR-H2 comprises SEQ ID NO:5; and
HVR-H3 comprises SEQ ID NO:6 or SEQ ID NO:16 or SEQ ID NO:17 or SEQ ID NO:19.

38. The method of claim 37, wherein the anti-integrin beta7 antibody comprises a variable light chain comprising the amino acid sequence of SEQ ID NO:31 and a variable heavy chain comprising the amino acid sequence of SEQ ID NO:32.

39. The method of claim 38, wherein the anti-integrin beta7 antibody is etrolizumab.

40. The method of any one of claims 28-39, wherein the patient has moderately to severely active Crohn's disease prior to administration of the first dose of the integrin beta7 antagonist.

41. The method of claim 40, wherein the patient is determined to have a CDAI score of greater than or equal to 220 and less than or equal to 480 at any time in the seven days prior to administration of the first dose.

42. The method of claim 40 or 41, wherein the patient is determined to have a PRO2 score of greater than or equal to 14 at any time in the seven days prior to administration of the first dose.

43. The method of any one of claims 40-42, wherein the patient is determined to have active inflammation, wherein the active inflammation is determined as a SES-CD score of greater than or equal to 7 as determined by ileocolonoscopy.

44. The method of any one of claims 40-42, wherein the patient has isolated ileitis or post-ileocecal resection and wherein the patient is determined to have active inflammation, wherein the active inflammation is determined as a SES-CD score of greater than or equal to 4 as determined by ileocolonoscopy.

45. The method of any one of claims 40-44, wherein the patient had an inadequate response, a loss of response, or intolerance to conventional therapy.

46. The method of claim 45, wherein the conventional therapy is selected from one or more of immunosuppressant therapy, corticosteroid therapy, and anti-TNF therapy.

47. The method of claim 46, wherein the immunosuppressant therapy is selected from 6-mercaptopurine, azathioprine, and methotrexate.

48. The method of claim 46, wherein the corticosteroid therapy is selected from prednisone, prednisone equivalent, and budesonide.

49. The method of claim 46, wherein the anti-TNF therapy is selected from infliximab, adalimumab, and certolizumab pegol.

50. The method of any one of claims 28-49, wherein the anti-integrin beta7 antibody is administered at a flat dose of 105 mg every 4 weeks or at a flat dose of 210 mg every 4 weeks.

51. The method of any one of claims 28-49, wherein the anti-integtrin beta7 antibody is administered as a flat dose of 210 mg at the first dose, 210 mg two weeks after the first dose, 210 mg four weeks after the first dose, 210 mg eight weeks after the first dose, 210 mg 12 weeks after the first dose and 105 mg every 4 weeks thereafter.

52. The method of any one of claims 28-51, wherein remission is determined by Crohn's Disease Activity Index (CDAI) score, wherein the CDAI score is less than 150.

53. The method of claim 52, wherein the patient is corticosteroid-free for at least 52 weeks.

54. The method of any one of claims 28-53, wherein remission is determined by Patient Reported Outcomes 2 (PRO2) score, wherein the PRO2 score is less than or equal to 11.

55. The method of any of claims 28-54, wherein the therapeutically effective amount induces endoscopic improvement as determined by Simplified Endoscopic Index for Crohn's Disease (SES-CD) score.

56. The method of claim 55, wherein the SES-CD score determined 66 weeks after administration of the first dose is reduced by 50% compared to the SES-CD score determined at baseline.

57. The method of claim 55, wherein the endoscopic improvement 66 weeks after administration of the first dose is resolution of mucosal inflammation, wherein resolution of mucosal inflammation is SES-CD score determined as zero.

58. The method of any one of claims 28-57, wherein the therapeutically effective amount induces a response 14 weeks after administration of the first dose, wherein the response is determined as a decrease of CDAI score of at least 70 points compared to CDAI score determined at baseline.

59. The method of any one of claims 28-57, wherein the therapeutically effective amount induces a response 66 weeks after administration of the first dose, wherein the response is determined as a decrease of CDAI score of at least 100 points compared to CDAI score determined at baseline.

60. The method according to any one of the preceding claims, wherein the integrin beta7 antagonist is administered using a prefilled syringe or a prefilled syringe and autoinjector combination.
